# EUROPEAN PATENT SPECIFICATION

(11) **EP 1 512 758 B1**
(45) Date of publication and mention of the grant of the patent: **17.10.2012**
(21) Application number: 04255208.3
(22) Date of filing: 27.08.2004
(51) Int. Cl.: C12Q 1/68

(54) **Colorectal cancer prognostics**
Prognose von kolorektalem Krebs
Pronostic de cancer colorectal

(30) Priority: 27.08.2003 US 651237; 18.02.2004 US 782413
(43) Date of publication of application: 09.03.2005
(73) Proprietor: Veridex, LLC, Raritan, NJ 08869 (US)
(72) Inventor: Wang, Yixin, San Diego, CA 92130 (US)
(74) Representative: Mercer, Christopher Paul

(56) References cited:
- EP-A- 1 355 150
- WO-A-00/19206
- WO-A-01/94629
- WO-A-01/96388
- WO-A-02/068579
- US-A1- 2004 191 782
- ZHANG L ET AL: "Gene expression profiles in normal and cancer cells" SCIENCE, AMERICAN ASSOCIATION FOR THE ADVANCEMENT OF SCIENCE,, US, vol. 276, 23 May 1997 (1997-05-23), pages 1268-1272, XP002083785 ISSN: 0036-8075
- HEGDE ET AL: "Identification of tumor markers in models of human colorectal cancer using a 19200-element complementary DNA microarray" CANCER RESEARCH, AMERICAN ASSOCIATION FOR CANCER RESEARCH, BALTIMORE, MD, US, vol. 61, no. 21, 1 November 2001 (2001-11-01), pages 7792-7797, XP002210615 ISSN: 0008-5472
- AFFYMETRIX: 'Genechip Human Genome U133 Arrays'
- MARSHALL A ET AL: "DNA CHIPS: AN ARRAY OF POSSIBILITIES", NATURE BIOTECHNOLOGY, NATURE PUBLISHING GROUP, NEW YORK, NY, US, vol. 16, 1 January 1998 (1998-01-01), pages 27-31, XP000198576, ISSN: 1087-0156, DOI: 10.1038/NBT0198-27

## Description

### BACKGROUND

This invention relates to prognostics for colorectal cancer based on the gene expression profiles of biological samples.

Colorectal cancer is a heterogenous disease with complex origins. Once a patient is treated for colorectal cancer, the likelihood of a recurrence is related to the degree of tumor penetration through the bowel wall and the presence or absence of nodal involvement. These characteristics are the basis for the current staging system defined by Duke's classification. Duke's A disease is confined to submucosa layers of colon or rectum. Duke's B tumor invades through muscularis propria and could penetrate the wall of colon or rectum. Duke's C disease includes any degree of bowel wall invasion with regional lymph node metastasis.

Surgical resection is highly effective for early stage colorectal cancers, providing cure rates of 95% in Duke's A and 75% in Duke's B patients. The presence of positive lymph node in Duke's C disease predicts a 60% likelihood of recurrence within five years. Treatment of Duke's C patients with a post surgical course of chemotherapy reduces the recurrence rate to 40%-50%, and is now the standard of care for Duke's C patients. Because of the relatively low rate of reoccurrence, the benefit of post surgical chemotherapy in Duke' B has been harder to detect and remains controversial. However, the Duke's B classification is imperfect as approximately 20 - 30% of these patients behave more like Duke's C and relapse within a 5-year timeframe.

There is clearly a need to identify better prognostic factors than nodal involvement for guiding selection of Duke's B into those that are likely to relapse and those that will survive. In commonly owned US Patent Application 10/403,499 to Wang, gene expression profiles prognostic for colon cancer were presented. This specification presents different gene expression profiles.

### SUMMARY OF THE INVENTION

The invention is a method of assessing the likelihood of a recurrence of colorectal cancer in a patient diagnosed with or treated for colorectal cancer. The method involves the analysis of a gene expression profile.

In one aspect of the invention, the gene expression profile includes at least seven particular genes.

In another aspect of the invention, the gene expression profile includes at least fifteen particular genes.

In yet another aspect of the invention, the gene expression profile includes the seven particular genes as well as the fifteen particular genes described above. In one embodiment, the gene profile comprises twenty-three genes.

Articles used in practising the methods are also an described. Such articles include gene expression profiles or representations of them that are fixed in machine-readable media such as computer readable media.

Articles used to identify gene expression profiles can also include substrates or surfaces, such as microarrays, to capture and/or indicate the presence, absence, or degree of gene expression.

In yet another aspect of the invention, kits include reagents for conducting the gene expression analysis prognostic of colorectal caner recurrence.

### BRIEF DESCRIPTION OF THE DRAWINGS

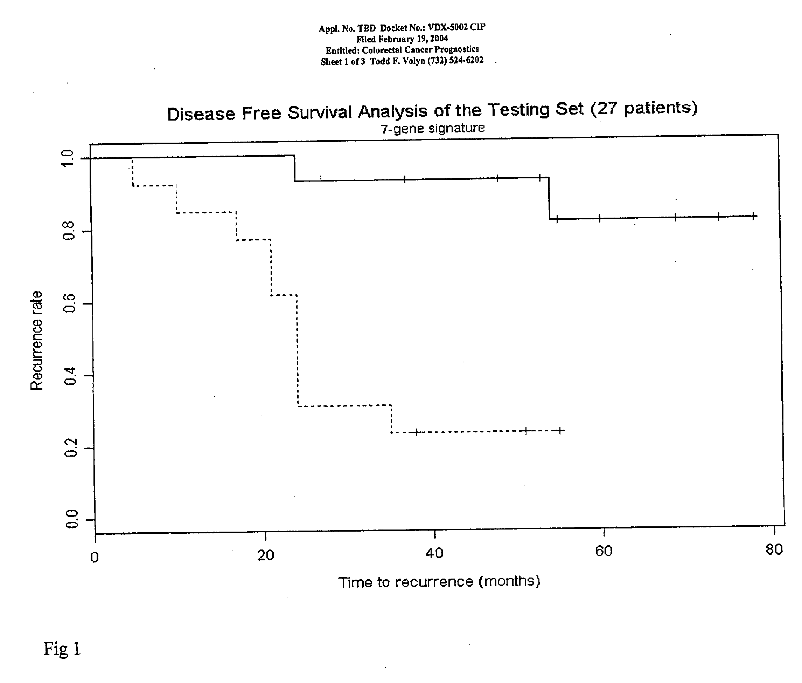

Fig. 1 is a standard Kaplan-Meier Plot constructed from the independent patient data set of 27 patients (14 survivors, 13 relapses) as described in the Examples for the analysis of the seven gene portfolio. Two classes of patients are indicated as predicted by chip data. The vertical axis shows the probability of disease-free survival among patients in each class.

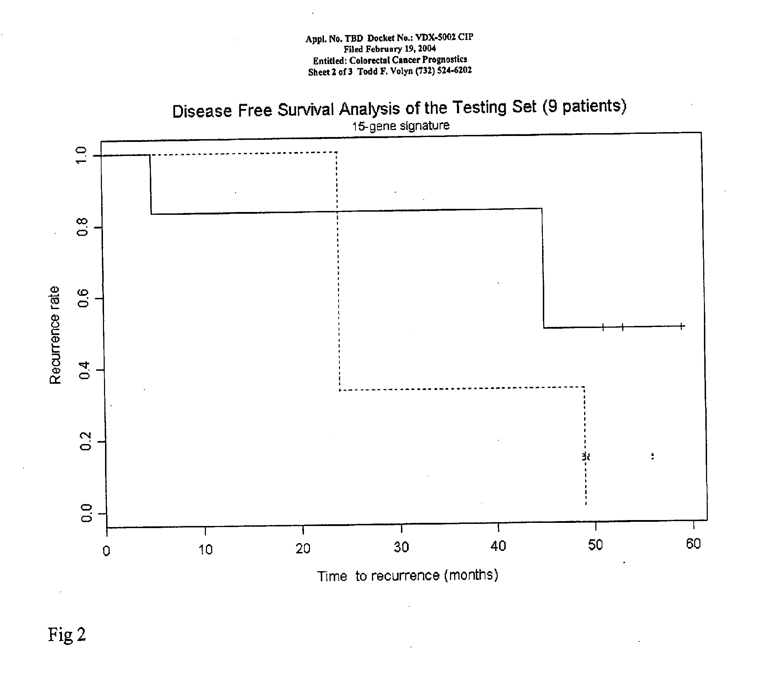

Fig. 2 is a standard Kaplan-Meier Plot constructed from the independent patient data set of 9 patients (6 survivors, 3 relapses) as described in the Examples for the analysis of the 15 gene portfolio. Two classes of patients are indicated as predicted by chip data. The vertical axis shows the probability of disease-free survival among patients in each class.

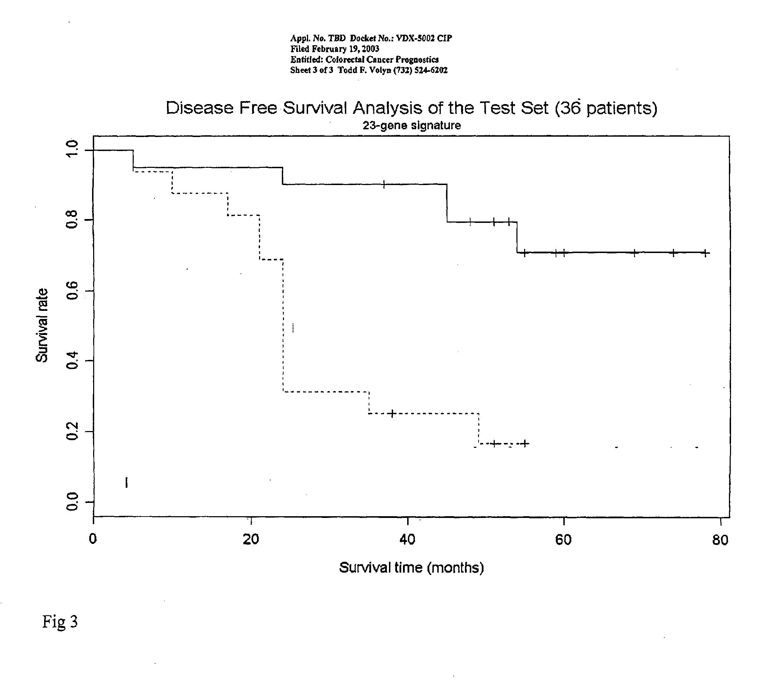

Fig. 3 is a standard Kaplan-Meier Plot constructed from patient data as described in the Examples and using the 22- gene profile with the inclusion of Cadherin 17 (Seq. ID 6) to the portfolio. Thirty-six samples were tested (20 survivor, 16 relapses) Two classes of patients are indicated as predicted by chip data of the 23-gene panel. The vertical axis shows the probability of disease-free survival among patients in each class.

### DETAILED DESCRIPTION

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA ( such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumerogenesis, metastasis, apoptosis, and other clinically relevant phenomena. Relative indications of the degree to which genes are active or inactive can be found in gene expression profiles. The gene expression profiles of this invention are used to provide a prognosis and treat patients for colorectal cancer.

Sample preparation requires the collection of patient samples. Patient samples used in the inventive method are those that are suspected of containing diseased cells such as epithelial cells taken from the primary tumor in a colon sample or from surgical margins. Laser Capture Microdisection (LCM) technology is one way to select the cells to be studied, minimizing variability caused by cell type heterogeneity. Consequently, moderate or small changes in gene expression between normal and cancerous cells can be readily detected. Samples can also comprise circulating epithelial cells extracted from peripheral blood. These can be obtained according to a number of methods but the most preferred method is the magnetic separation technique described in U.S. Patent 6,136,182. Once the sample containing the cells of interest has been obtained, RNA is extracted and amplified and a gene expression profile is obtained, preferably via micro-array, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complimentary DNA (cDNA) or complimentary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al.

Analysis of the expression levels is conducted by comparing such signal intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.

Gene expression profiles can also be displayed in a number of ways. The most common method is to arrange a raw fluorescence intensities or ratio matrix into a graphical dendogram where columns indicate test samples and rows indicate genes. The data is arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRING" from Silicon Genetics, Inc. and "DISCOVERY" and "INFER" software from Partek, Inc.

Modulated genes used in the methods of the invention are described in the Examples. The genes that are differentially expressed are either up regulated or down regulated in patients with a relapse of colon cancer relative to those without a relapse. Up regulation and down regulation are relative terms meaning that a detectable difference (beyond the contribution of noise in the system used to measure it) is found in the amount of expression of the genes relative to some baseline. In this case, the baseline is the measured gene expression of a non-relapsing patient. The genes of interest in the diseased cells (from the relapsing patients) are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis includes the determination of disease/status issues such as determining the likelihood of relapse and therapy monitoring. In therapy monitoring, clinical judgments are made regarding the effect of a given course of therapy by comparing the expression of genes over time to determine whether the gene expression profiles have changed or are changing to patterns more consistent with normal tissue.

Preferably, levels of up and down regulation are distinguished based on fold changes of the intensity measurements of hybridized microarray probes. A 2.0 fold difference is preferred for making such distinctions or a p-value less than .05. That is, before a gene is said to be differentially expressed in diseased/relapsing versus normal/non-relapsing cells, the diseased cell is found to yield at least 2 more, or 2 times less intensity than the normal cells. The greater the fold difference, the more preferred is use of the gene as a diagnostic or prognostic tool. Genes selected for the gene expression profiles of the instant invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, one is unlikely to see small p-values just by chance and adjustments for this using a Sidak correction as well as a randomization/permutation experiment can be made. A p-value less than .05 by the t-test is evidence that the gene is significantly different. More compelling evidence is a p-value less then .05 after the Sidak correction is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Genes can be grouped so that information obtained about the set of genes in the group provides a sound basis for making a clinically relevant judgment such as a diagnosis, prognosis, or treatment choice. These sets of genes make up the portfolios of the invention. In this case, the judgments supported by the portfolios involve colorectal cancer and its chance of recurrence, most preferably, among Dukes B patients. As with most diagnostic markers, it is often desirable to use the fewest number of markers sufficient to make a correct medical judgment. This prevents a delay in treatment pending further analysis as well inappropriate use of time and resources.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity.

One method of establishing gene expression portfolios is through the use of optimization algorithms such as the mean variance algorithm widely used in establishing stock portfolios. This method is described in detail in the patent application entitled "Portfolio Selection" by Tim Jatkoe, et. al., filed on March 21, 2003. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application", referred to as "Wagner Software" throughout this specification, is preferred. This software uses functions from the "Wagner Associates Mean-Variance Optimization Library" to determine an efficient frontier and optimal portfolios in the Markowitz sense.is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with colorectal cancer. Output from the method would be an optimized set of genes that could include some genes that are expressed in peripheral blood as well as in diseased tissue. If samples used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a certain percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

One method of the invention involves comparing gene expression profiles for various genes (or portfolios) to ascribe prognoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal or diseased. In a more sophisticated embodiment, patterns of the expression signals (e.g., flourescent intensity) are recorded digitally or graphically. The gene expression patterns from the gene portfolios used in conjunction with patient samples are then compared to the expression patterns. Pattern comparison software can then be used to determine whether the patient samples have a pattern indicative of recurrence of the disease. Of course, these comparisons can also be used to determine whether the patient is not likely to experience disease recurrence. The expression profiles of the samples are then compared to the portfolio of a control cell. If the sample expression patterns are consistent with the expression pattern for recurrence of a colorectal cancer then (in the absence of countervailing medical considerations) the patient is treated as one would treat a relapse patient. If the sample expression patterns are consistent with the expression pattern from the normal/control cell then the patient is diagnosed negative for colorectal cancer.

The preferred profiles of this invention are the seven-gene portfolio shown in Table 2 and the fifteen-gene portfolio shown in Table 3. It is more preferred to use a portfolio in which both seven and fifteen gene groups are combined. Gene expression portfolios made up another independently verified colorectal prognostic gene such as Cadherin 17 (Seq. ID No. 6) together with the combination of genes in both Table 2 and Table 3 are most preferred (Table 4). This most preferred portfolio best segregates Duke's B patients at high risk of relapse from those who are not. Once the high-risk patients are identified they can then be treated with adjuvant therapy. Other independently verified prognostic genes that can be used in place of Cadherin 17 include, without limitation, genes that correspond to Seq ID No. 29-94.

In this invention, the most preferred method for analyzing the gene expression pattern of a patient to determine prognosis of colon cancer is through the use of a Cox hazard analysis program. Most preferably, the analysis is conducted using S-Plus software (commercially available from Insightful Corporation). Using such methods, a gene expression profile is compared to that of a profile that confidently represents relapse (i.e., expression levels for the combination of genes in the profile is indicative of relapse). The Cox hazard model with the established threshold is used to compare the similarity of the two profiles (known relapse versus patient) and then determines whether the patient profile exceeds the threshold. If it does, then the patient is classified as one who will relapse and is accorded treatment such as adjuvant therapy. If the patient profile does not exceed the threshold then they are classified as a non-relapsing patient. Other analytical tools can also be used to answer the same question such as, linear discriminate analysis, logistic regression and neural network approaches.

Numerous other well-known methods of pattern recognition are available. The following references provide some examples:
Weighted Voting:
   Golub, TR., Slonim, DK., Tamaya, P., Huard, C., Gaasenbeek, M., Mesirov, JP., Coller, H., Loh, L., Downing, JR., Caligiuri, MA., Bloomfield, CD., Lander, ES. Molecular classification of cancer: class discovery and class prediction by gene expression monitoring. Science 286:531-537, 1999.
Support Vector Machines:
   Su, AL, Welsh, JB., Sapinoso, LM., Kern, SG., Dimitrov, P., Lapp, H., Schultz, PG., Powell, SM., Moskaluk, CA., Frierson, HF. Jr., Hampton, GM. Molecular classification of human carcinomas by use of gene expression signatures. Cancer Research 61:7388-93, 2001.
      Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. Multiclass cancer diagnosis using tumor gene expression signatures Proceedings of the National Academy of Sciences of the USA 98:15149-15154, 2001.
K-nearest Neighbors:
   Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. Multiclass cancer diagnosis using tumor gene expression signatures Proceedings of the National Academy of Sciences of the USA 98:15149-15154, 2001.
Correlation Coefficients:
   van 't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature, 2002 Jan 31;415(6871):530-6.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., carcinoembryonic antigen). A range of such markers exists including such analytes as CEA. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

Articles cited include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting colorectal cancer.

Kits made according to the invention include formatted assays for determining the gene expression profiles. These can include all or some of the materials needed to conduct the assays such as reagents and instructions.

The invention is further illustrated by the following non-limiting examples.

**Examples:** Genes analyzed according to this invention are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

### Example 1- Sample Handling and LCM.

Fresh frozen tissue samples were collected from patients who had surgery for colorectal tumors. The samples that were used were from 63 patients staged with Duke's B according to standard clinical diagnostics and pathology. Clinical outcome of the patients was known. Thirty-six of the patients have remained disease-free for more than 3 years while 27 patients had tumor relapse within 3 years.

The tissues were snap frozen in liquid nitrogen within 20-30 minutes of harvesting, and stored at -80C° thereafter. For laser capture, the samples were cut (6µm), and one section was mounted on a glass slide, and the second on film (P.A.L.M.), which had been fixed onto a glass slide (Micro Slides Colorfrost, VWR Scientific, Media, PA). The section mounted on a glass slide was after fixed in cold acetone, and stained with Mayer's Haematoxylin (Sigma, St. Louis, MO). A pathologist analyzed the samples for diagnosis and grade. The clinical stage was estimated from the accompanying surgical pathology and clinical reports to verify the Dukes classification. The section mounted on film was after fixed for five minutes in 100% ethanol, counter stained for 1 minute in eosin/100% ethanol (100µg of Eosin in 100ml of dehydrated ethanol), quickly soaked once in 100% ethanol to remove the free stain, and air dried for 10 minutes.

Before use in LCM, the membrane (LPC-MEMBRANE PEN FOIL 1.35 µm No 8100, P.A.L.M. GmbH Mikrolaser Technologie, Bernried, Germany) and slides were pretreated to abolish RNases, and to enhance the attachment of the tissue sample onto the film. Briefly, the slides were washed in DEP H₂O, and the film was washed in RNase AWAY (Molecular Bioproducts, Inc., San Diego, CA) and rinsed in DEP H₂O. After attaching the film onto the glass slides, the slides were baked at +120°C for 8 hours, treated with TI-SAD (Diagnostic Products Corporation, Los Angeles, CA, 1:50 in DEP H₂O, filtered through cotton wool), and incubated at +37°C for 30 minutes. Immediately before use, a 10µl aliquot of RNase inhibitor solution (Rnasin Inhibitor 2500U=33U/µl N211A, Promega GmbH, Mannheim, Germany, 0.5µl in 400µl of freezing solution, containing 0.15 mol NaCl, 10 mmol Tris pH 8.0, 0.25 mmol dithiothreitol) was spread onto the film, where the tissue sample was to be mounted.

The tissue sections mounted on film were used for LCM. Approximately 2000 epithelial cells/sample were captured using the PALM Robot-Microbeam technology (P.A.L.M. Mikrolaser Technologie, Carl Zeiss, Inc., Thornwood, NY), coupled into Zeiss Axiovert 135 microscope (Carl Zeiss Jena GmbH, Jena, Germany). The surrounding stroma in the normal mucosa, and the occasional intervening stromal components in cancer samples, were included. The captured cells were put in tubes in 100% ethanol and preserved at -80°C.

### Example 2- RNA Extraction and Amplification.

Zymo-Spin Column (Zymo Research, Orange, CA 92867) was used to extract total RNA from the LCM captured samples. About 2 ng of total RNA was resuspended in 10 ul of water and 2 rounds of the T7 RNA polymerase based amplification were performed to yield about 50 ug of amplified RNA.

### Example 3- DNA Microarray Hybridization and Quantitation.

A set of DNA microarrays consisting of approximately 23,000 human DNA clones was used to test the samples by use of the humanU133a chip obtained and commercially available from Affymetrix, Inc. Total RNA obtained and prepared as outlined above and applied to the chips and analyzed by Agilent BioAnalyzer according to the manufacturer's s protocol. All 63 samples passed the quality control standards and the data were used for marker selection.

Chip intensity data was analyzed using MAS Version 5.0 software commercially available from Affymetrix, Inc. ("MAS 5.0"). An unsupervised analysis was used to identify two genes that distinguish patients that would relapse from those who would not as follows.

The chip intensity data obtained as described was the input for the unsupervised clustering software commercially available as PARTEK version 5.1 software. This unsupervised clustering algorithm identified a group of 20 patients with a high frequency of relapse (13 relapsers and 7 survivors). From the original 23,000 genes, t-testing analysis selected 276 genes that significantly differentially expressed in these patients. From this group, two genes were selected that best distinguish relapsing patients from those that do not relapse: Human intestinal peptide-associated transporter (Seq. ID. No. 3) and Homo sapiens fatty acid binding protein 1 (Seq. ID No. 1). These two genes are down-regulated (in fact, they are turned off or not expressed) in the relapsing patients from this patient group.

Supervised analysis was then conducted to further discriminate relapsing patients from those who did not relapse in the remaining 43 patients. This group of patient data was then divided into the following groups: 27 patients were assigned as the training set and 16 patients were assigned as the testing set. This ensured that the same data was not used to both identify markers and then validate their utility.

An unequal variance t-test was performed on the training set. From a list of 28 genes that have significant corrected p values, MHC II-DR-B was chosen. These genes are down-regulated in relapsers. MHC II-DR-B (Seq. ID No. 2) also had the smallest p-value.

In an additional round of supervised analysis, a variable selection procedure for linear discriminant analysis was implemented using the Partek Version 5.0 software described above to separate relapsers from survivors in the training set. The search method was forward selection. The variable selected with the lowest posterior error was immunoglobulin-like transcript 5 protein (Seq. ID No. 4). A Cox proportional hazard model (using "S Plus" software from Insightful, Inc.) was then used for gene selection to confirm gene selection identified above for survival time. In each cycle of total 27 cycles, each of the 27 patients in the training set was held out, the remaining 26 patients were used in the univariate Cox model regression to assess the strength of association of gene expression with the patient survival time. The strength of such association was evaluated by the corresponding estimated standardized parameter estimate and P value returned from the Cox model regression. P value of 0.01 was used as the threshold to select top genes from each cycle of the leave-one-out gene selection. The top genes selected from each cycle were then compared in order to select those genes that showed up in at least 26 times in the total of 27 leave-one-out gene selection cycles. A total of 70 genes were selected and both MHC II-DR-B and immunoglobulin-like transcript 5 protein were among them (Again, showing down regulation).

***Construction of a multiple-gene predictor:*** Two genes, MHC II-DR-B and immunoglobulin-like transcript 5 protein were used to produce a predictor using linear discriminant analysis. The voting score was defined as the posterior probability of relapse. If the patient score was greater than 0.5, the patient was classified as a relapser. If the patient score was less than 0.5, the patient was classified as a survivor. The predictor was tested on the training set.

***Cross-validation and evaluation of predictor:*** Performance of the predictor should be determined on an independent data set because most classification methods work well on the examples that were used in their establishment. The 16 patients test set was used to assess prediction accuracy. The cutoff for the classification was determined by using a ROC curve. With the selected cutoff, the numbers of correct prediction for relapse and survival patients in the test set were determined.

***Overall prediction:*** Gene expression profiling of 63 Duke's B colon cancer patients led to identification of 4 genes that have differential expression (down regulation or turned off) in these patients. These genes are Seq. ID No. 1, Seq. ID No. 2, Seq. ID No. 3, and Seq. ID No. 4. Thirty-six of the patients have remained disease-free for more than 3 years while 27 patients had tumor relapse within 3 years. Using the 3 gene markers portfolio of Seq. ID No. 2 , Seq. ID No. 3, and Seq. ID No. 4, 22 of the 27 relapse patients and 27 of 36 disease-free patients are identified correctly. This result represents a sensitivity of 82% and a specificity of 75%. The positive predictive value is 71% and the negative predictive value is 84%.

### Example 4: Further Sampling

Frozen tumor specimens from 74 coded Dukes' B colon cancer patients were then studied. Primary tumor and adjacent non-neoplastic colon tissue were collected at the time of surgery. The histopathology of each specimen was reviewed to confirm diagnosis and uniform involvement with tumor. Regions chosen for analysis contained a tumor cellularity greater than 50% with no mixed histology. Uniform follow-up information was also available.

### Example 5: Gene Expression Analysis

Total RNA was extracted from the samples of Example 4 according to the method described in Examples 1-3. Arrays were scanned using standard Affymetrix protocols and scanners. For subsequent analysis, each probe set was considered as a separate gene. Expression values for each gene were calculated by using Affymetrix GeneChip analysis software MAS 5.0. All data used for subsequent analysis passed quality control criteria.

### Statistical Methods

Gene expression data were first subjected to a variation filter that excluded genes called "absent" in all the samples. Of the 22,000 genes considered, 17,616 passed this filter and were used for clustering. Prior to the hierarchical clustering, each gene was divided by its median expression level in the patients. Genes that showed greater than 4-fold changes over the mean expression level in at least 10% of the patients were included in the clustering. To identify patient subgroups with distinct genetic profiles, average linkage hierarchical clustering and k-mean clustering was performed by using GeneSpring 5.0 (San Jose, CA) and Partek 5.1 software (St. Louis, MO), respectively. T-tests with Bonferroni corrections were used to identify genes that have different expression levels between 2 patient subgroups implicated by the clustering result. A Bonferroni corrected P value of 0.01 was chosen as the threshold for gene selection. Patients in each cluster that had a distinct expression profile were further examined with the outcome information.

In order to identify gene markers that can discriminate the relapse and the disease-free patients, each subgroup of the patients was analyzed separately as described further below. All the statistical analyses were performed using S-Plus software (Insightful, VA).

### Patient and Tumor Characteristics.

Clinical and pathological features of the patients and their tumors are summarized in Table 1. The patients had information on age, gender, TNM stage, grade, tumor size and tumor location. Seventy-three of the 74 patients had data on the number of lymph nodes that were examined, and 72 of the 74 patients had estimated tumor size information. The patient and tumor characteristics did not differ significantly between the relapse and non-relapse patients. None of the patients received pre-operative treatment. A minimum of 3 years of follow-up data was available for all the patients in the study.

### Patient Subgroups Identified by Genetic Profiles

Unsupervised hierarchical clustering analysis resulted in a cluster of the 74 patients on the basis of the similarities of their expression profiles measured over 17,000 significant genes. Two subgroups of patients were identified that have over 600 differentially expressed genes between them (p < 0.00001). The larger subgroup and the smaller subgroup contained 54 and 20 patients, respectively. In the larger subgroup of the 54 patients only 18 patients (33%) developed tumor relapse within 3 years whereas in the smaller subgroup of the 20 patients 13 patients (65%) had progressive diseases. Chi square analysis gave a p value of 0.028.

Two dominant gene clusters that had drastic differential expression between the two types of tumors were selected and examined. The first gene cluster had a group of down-regulated genes in the smaller subgroup of the 20 patients, represented by liver-intestine specific cadherin 17, fatty acid binding protein 1, caudal type homeo box transcription factors CDX1 and CDX2, mucin and cadherin-like protein MUCDHL. The second gene cluster is represented by a group of up-regulated genes in the smaller subgroup including serum-inducible kinase SNK, annexin A1, B cell RAG associated protein, calbindin 2, and tumor antigen L6. The smaller subgroup of the 20 patients thus represent less differentiated tumors on the basis of their genetic profiles.

### Gene Signature and its Prognostic Value

In order to identify gene markers that can discriminate the relapse and the disease-free patients, each subgroup of the patients were analyzed separately. The patients in each subgroup were first divided into a training set and a testing set with approximately equal number of patients. The training set was used to select the gene markers and to build a prognostic signature. The testing set was used for independent validation. In the larger subgroup of the 54 tumors, 36 patients had remained disease-free for at least 3 years after their initial diagnosis and 18 patients had developed tumor relapse with 3 years. The 54 patients were divided into two groups. The training set contained 21 disease-free patients and 6 relapse patients. In the smaller subgroup of the 20 tumors, 7 patients had remained disease-free for at least 3 years and 13 patients had developed tumor relapse with 3 years. The 20 patients were divided into two groups. The training set contained 4 disease-free patients and 7 relapse patients. To identify a gene signature that discriminates the good prognosis group from the poor prognosis group, a supervised classification method was used on each of the training sets. Univariate Cox proportional hazards regression was used to identify genes whose expression levels are correlated to patient survival time. Genes were selected using p-values less than 0.02 as the selection criteria. Next, t-tests were performed on the selected genes to determine the significance of the differential expression between relapse and disease-free patients (P < 0.01). To avoid selection of genes that over-fit the training set, re-sampling of 100 times was performed with the t-test in order to search for genes that have significant p values in more than 80% of the re-sampling tests. Seven genes (Table 2) were selected from the 27 patient training set and 15 genes (Table 3) were selected from the 11 patient training set. Taking the 22 genes and cadherin 17 together, a Cox model to predict patient recurrence was built using the S-Plus software. The Kaplan-Meier survival analysis showed a clear difference in the probability that patients would remain disease free between the group predicted with good prognosis and the group predicted with poor prognosis (Fig. 3).

Several genes are related to cell proliferation or tumor progression. For example, tyrosine 3 monooxygenase tryptophan 5-monooxygenase activation protein (YWHAH) belongs to 14-3-3 family of proteins that is responsible for G2 cell cycle control in response to DNA damage in human cells. RCC1 is another cell cycle gene involved in the regulation of onset of chromosome condensation. BTEB2 is a zinc finger transcription factor that has been implicated as a beta-catenin independent Wnt-1 responsive genes. A few genes are likely involved in local immune responses. Immunoglobulin-like transcript 5 protein is a common inhibitory receptor for MHC I molecules. A unique member of the gelsolin/villin family capping protein, CAPG is primarily expressed in macrophages. LAT is a highly tyrosine phosphorylated protein that links T cell receptor to cellular activation. Thus both tumor cell- and immune cell-expressed genes can be used as prognostic factors for patient recurrence.

In order to validate the 23-gene prognostic signature, the patients in the two testing sets that included 27 patients from the larger subgroup and 9 patients from the smaller subgroup were combined and outcome was predicted for the 36 independent patients in the testing sets. This testing set consisted of 18 patients who developed tumor relapses within 3 years and 18 patients who had remained disease free for more than 3 years. The prediction resulted in 13 correct relapse classification and 15 correct disease-free classifications. The overall performance accuracy was 78% (28 of 36) with a sensitivity of 72% (13 of 18) and a specificity of 83% (15 of 18). This performance indicates that the Dukes' B patients that have a value below the threshold of the prognostic signature have a 13-fold odds ratio of (95% CI: 2.6, 65; p=0.003) developing a tumor relapse within 3 years compared with those that have a value above the threshold of the prognostic signature. Furthermore, the Kaplan-Meier survival analysis showed a significant difference in the probability that patients would remain disease free between the group predicted with good prognosis and the group predicted with poor prognosis (P < 0.0001). In a multivariate Cox proportional hazards regression, the estimated hazards ratio for tumor recurrence was 0.41 (95% confidence interval, 0.24 to 0.71; P = 0.001), indicating that the 23-gene set represents a prognosis signature and it is inversely associated with a higher risk of tumor recurrence. Using the seven gene portfolio (Table 2), an 83% sensitivity and 80% specificity were obtained (based on a 12 relapse and 15 survivor sample set). Using the 15 gene portfolio (Table 3), a 50% sensitivity and 100% specificity were obtained (based on 6 relapse and three survivor sample sets). Figures 1 and 2 are graphical portrayals of the Kaplan-Meier analyses for the seven and fifteen gene portfolios respectively.

Furthermore, as these results demonstrate, prognosis can be derived from gene expression profiles of the primary tumor.

**Table 1. Clinical and Pathological Characteristics of Patients and Their Tumors**

| **Characteristics** | **Disease-free** no. of patients (%) | | **Recurrence** | | **P Value*** |
|---|---|---|---|---|---|
| Age | 43 | | 31 | | 0.7649 |
| Mean | 58.93 | | 58.06 | | |
| | | | | | |
| Sex | 43 | | 31 | | 0.8778 |
| Female | 23 | (53) | 18 | (58) | |
| Male | 20 | (47) | 13 | (42) | |
| | | | | | |
| T Stage | 43 | | 31 | | 0.2035 |
| 2 | 12 | (28) | 5 | (16) | |
| 3 | 29 | (67) | 26 | (84) | |
| 4 | 2 | (5) | 0 | (0) | |
| | | | | | |
| Differentiation | 43 | | 31 | | 0.4082 |
| Poor | 5 | (12) | 6 | (19) | |
| Moderate | 37 | (86) | 23 | (74) | |
| Well | 1 | (2) | 2 | (6) | |
| | | | | | |
| Tumor size | 41 | | 31 | | 0.1575 |
| <5 | 29 | (71) | 16 | (52) | |
| >=5 | 12 | (29) | 15 | (48) | |
| | | | | | |
| Location | 43 | | 31 | | 0.7997 |
| LC | 1 | (2) | 1 | (3) | |
| RC | 17 | (40) | 10 | (32) | |
| TC | 6 | (14) | 3 | (10) | |
| SC | 19 | (44) | 17 | (55) | |
| Number of LN examined Mean | | | | | |
| | 43 | | 30 | | 0.0456 |
| | 12.81 | | 8.63 | | |

| | | | | | |
|---|---|---|---|---|---|
| ^{*} P values for Age, Lymph node number and Tumor content are obtained by t tests; P values for others are obtained by □² tests. | | | | | |

**Table 2: 7 Gene List**

| **Accession** | **Seq. I.D No.** |
|---|---|
| AF009643.1 | 7 |
| NM_003405.1 | 8 |
| X06130.1 | 9 |
| AB030824.1 | 10 |
| NM_001747.1 | 11 |
| AF036906.1 | 12 |
| BC005286.1 | 13 |

**Table 3: 15 Gene List**

| **Accession** | **Seq. I.D. No.** |
|---|---|
| NM_012345.1 | 14 |
| NM_030955.1 | 15 |
| NM_001474.1 | 16 |
| AF239764.1 | 17 |
| D13368.1 | 18 |
| NM_012387.1 | 19 |
| NM_ 016611.1 | 20 |
| NM_014792.1 | 21 |
| NM_017937.1 | 22 |
| NM_001645.2 | 23 |
| AL545035 | 24 |
| NM_022078.1 | 25 |
| AL133089.1 | 26 |
| NM_001271.1 | 27 |
| AL137428.1 | 28 |

**Table 4. Twenty-three genes form the prognostic signature.**

| **Direction of change** | **Seq. ID No.** | **P value (Cox)** | **Gene Description** |
|---|---|---|---|
| - | **7** | 0.0011 | immunoglobulin-like transcript 5 protein |
| - | **8** | 0.0016 | tyrosine 3-monooxygenasetryptophan 5-monooxygenase activation protein |
| - | **9** | 0.0024 | cell cycle gene RCC1 |
| + | **10** | 0.0027 | transcription factor BTEB2 |
| - | **11** | 0.0045 | capping protein (actin filament), gelsolin-like (CAPG) |
| - | **12** | 0.0012 | linker for activation of T cells (LAT) |
| - | **13** | 0.0046 | Lafora disease (laforin) |
| - | **14** | 0.0110 | nuclear fragile X mental retardation protein interacting protein 1 (NUFIP1) |
| + | **15** | 0.0126 | disintegrin-like and metalloprotease (reprolysin type) with thrombospondin type 1 motif, 12 (ADAMTS 12) |
| + | **16** | 0.0126 | G antigen 4 (GAGE4) |
| + | **17** | 0.0130 | EGF-like module-containing mucin-like receptor EMR3 |
| + | **18** | 0.0131 | alanine:glyoxylate aminotransferase |
| + | **19** | 0.0131 | peptidyl arginine deiminase, type V (PAD) |
| + | **20** | 0.0136 | potassium inwardly-rectifying channel, subfamily K, member 4 (KCNK4) |
| + | **21** | 0.0139 | KIAA0125 gene product (KIAA0125) |
| + | **22** | 0.0142 | hypothetical protein FLJ20712 (FLJ20712) |
| + | **23** | 0.0145 | apolipoprotein C-I (APOC1) |
| + | **24** | 0.0146 | Consensus includes gb:AL545035 |
| + | **25** | 0.0149 | hypothetical protein FLJ12455 (FLJ12455) |
| + | **26** | 0.0150 | Consensus includes gb:AL133089.1 |
| + | **27** | 0.0151 | chromodomain helicase DNA binding protein 2 (CHD2) |
| + | **28** | 0.0152 | Consensus includes gb:AL137428.1 |
| **N/A** | **6** | Not tested | Cadherin 17 |

## Claims

1. A method of assessing colorectal cancer status among Dukes B patients comprising identifying differential expression in a combination of seven genes, wherein said combination consists of all of the genes corresponding to SEQ. ID. NOS.: 7-13.

2. The method of claim 1 wherein the expression pattern of the genes is compared to an expression pattern indicative of a relapse patient.

3. The method of claim 2 wherein the comparison of expression patterns is conducted with pattern recognition methods.

4. The method of claim 3 wherein the pattern recognition methods include the use of a Cox proportional hazards analysis.

5. The method of any one of claims I to 4 conducted on primary tumor sample.

6. The method of any one of claims 1 to 5 wherein there is at least a 2 fold difference in the expression of the modulated genes.

7. The method of any one of claims 1 to 6, wherein the p-value indicating differential modulation is less than .05.

8. The method of any one of claims 1 to 7 further comprising a colorectal diagnostic that is not genetically based.

9. A microarray, wherein the nucleic acid sequences on said microarray consist of the sequences of SEQ. ID. NOS.: 7-13, or their complements.

10. The microarray of claim 9 wherein said microarray is a cDNA microarray.

11. The microarray of claim 10 wherein said microarray is an oligonucleotide microarray.

12. The use of a microarray comprising isolated nucleic acid sequences or their complements in a method according to any one of claims 1 to 8, wherein said microarray comprises the sequences of SEQ. ID. NOS.: 7-13.

## Patentansprüche

1. Verfahren zur Beurteilung des Kolorektalkarzinom-Status unter Dukes-B-Patienten, wobei man differentielle Expression bei einer Kombination von sieben Genen identifiziert, wobei die Kombination aus allen SEQ ID NO: 7-13 entsprechenden Genen besteht.

2. Verfahren nach Anspruch 1, wobei das Expressionsmuster der Gene mit einem einen Rückfall eines Patienten anzeigenden Expressionsmuster verglichen wird.

3. Verfahren nach Anspruch 2, wobei der Vergleich von Expressionsmustern mit Mustererkennungsmethoden ausgeführt wird.

4. Verfahren nach Anspruch 3, wobei die Mustererkennungsmethoden die Verwendung einer Cox-Regressionsanalyse (Proportional Hazards) beinhalten.

5. Verfahren nach einem der Ansprüche 1 bis 4, ausgeführt an einer Primärtumorprobe.

6. Verfahren nach einem der Ansprüche 1 bis 5, wobei wenigstens ein 2-facher Unterschied in der Expression der modulierten Gene besteht.

7. Verfahren nach einem der Ansprüche 1 bis 6, wobei der differentielle Modulation anzeigende p-Wert weniger als 0,05 beträgt.

8. Verfahren nach einem der Ansprüche 1 bis 7, ferner umfassend eine Kolorektaldiagnostik, die nicht auf Genetik beruht.

9. Mikroarray, wobei die Nukleinsäuresequenzen auf dem Mikroarray aus den Sequenzen SEQ ID NO: 7-13 oder deren Komplementen bestehen.

10. Mikroarray nach Anspruch 9, bei dem es sich um einen cDNA-Mikroarray handelt.

11. Mikroarray nach Anspruch 10, bei dem es sich um einen Oligonukleotid-Mikroarray handelt.

12. Verwendung eines isolierte Nukleinsäuresequenzen oder deren Komplemente umfassenden Mikroarray bei einem Verfahren gemäß einem der Ansprüche 1 bis 8, wobei der Mikroarray die Sequenzen SEQ ID NO: 7-13 umfasst.

## Revendications

1. Procédé d'évaluation du statut pour le cancer colorectal parmi des patients Dukes B comprenant l'identification d'une expression différentielle dans une combinaison de sept gènes, dans lequel ladite combinaison est constituée de tous les gènes correspondant à SEQ ID NO: 7 à 13.

2. Procédé de la revendication 1 dans lequel le profil d'expression des gènes est comparé à un profil d'expression indicatif d'un patient en rechute.

3. Procédé de la revendication 2 dans lequel la comparaison de profils d'expression est conduite avec des procédés de reconnaissance de profil.

4. Procédé de la revendication 3 dans lequel les procédés de reconnaissance de profil comprennent l'utilisation d'une analyse de risques proportionnels de Cox.

5. Procédé de l'une quelconque des revendications 1 à 4 conduit sur un échantillon de tumeur primaire.

6. Procédé de l'une quelconque des revendications 1 à 5 dans lequel il y a une différence d'au moins 2 fois de l'expression des gènes modulés.

7. Procédé de l'une quelconque des revendications 1 à 6, dans lequel la valeur p indiquant une modulation différentielle est inférieure à 0,05.

8. Procédé de l'une quelconque des revendications 1 à 7 comprenant en outre un diagnostic colorectal qui n'est pas basé sur la génétique.

9. Micropuce, dans laquelle les séquences d'acide nucléique sur ladite micropuce sont constituées des séquences de SEQ ID NO: 7 à 13, ou leurs compléments.

10. Micropuce de la revendication 9 dans laquelle ladite micropuce est une micropuce à ADNc.

11. Micropuce de la revendication 10 dans laquelle ladite micropuce est une micropuce à oligonucléotides.

12. Utilisation d'une micropuce comprenant des séquences d'acide nucléique isolées ou leurs compléments dans un procédé selon l'une quelconque des revendications 1 à 8, dans laquelle ladite micropuce comprend les séquences de SEQ ID NO: 7 à 13.


### SEQUENCE LISTING

<110> Ortho-Clinical Diagnostics, Inc. Wang, Yixin
<120> Colorectal Cancer Prognostics
<130> P038616EP
<160> 94
<170> PatentIn version 3.1
<210> 1
   <211> 489
   <212> DNA
   <213> human
<400> 1
   agagccgcag gtcagtcgtg aagagggagc tctattgcca ccatgagttt ctccggcaag      60
   taccaactgc agagccagga aaactttgaa gccttcatga aggcaatcgg tctgccggaa     120
   gagctcatcc agaaggggaa ggatatcaag ggggtgtcgg aaatcgtgca gaatgggaag      180
   cacttcaagt tcaccatcac cgctgggtcc aaagtgatcc aaaacgaatt cacggtgggg     240
   gaggaatgtg agctggagac aatgacaggg gagaaagtca agacagtggt tcagttggaa     300
   ggtgacaata aactggtgac aactttcaaa aacatcaagt ctgtgaccga actcaacggc     360
   gacataatca ccaataccat gacattgggt gacattgtct tcaagagaat cagcaagaga     420
   atttaaacaa gtctgcattt catattattt tagtgtgtaa aattaatgta ataaagtgaa     480
   ctttgtttt                                                             489
<210> 2
   <211> 853
   <212> DNA
   <213> human
<400> 2
   gcctgctgct ctggcccctg gtcctgtcct gttctccagc atggtgtgtc tgaggctccc      60
   tggaggctcc tgcatggcag ttctgacagt gacactgatg gtgctgagct ccccactggc     120
   tttggctggg gacaccagac cacgtttctt ggagtactct acgtctgagt gtcatttctt     180
   caatgggacg gagcgggtgc ggtacctgga cagatacttc cataaccagg aggagaacgt     240
   gcgcttcgac agcgacgtgg gggagttccg ggcggtgacg gagctggggc ggcctgctgc     300
   ggagcactgg aacagccaga aggacctcct ggagcagaag cggggccggg tggacaacta     360
   ctgcagacac aactacgggg ttgtggagag cttcacagtg cagcggcgag tccatcctaa     420
   ggtgactgtg tatccttcaa agacccagcc cctgcagcac cataacctcc tggtctgttc     480
   tgtgagtggt ttctatccag gcagcattga agtcaggtgg ttccggaatg gccaggaaga     540
   gaagactggg gtggtgtcca caggcctgat ccacaatgga gactggacct tccagaccct     600
   ggtgatgctg gaaacagttc ctcggagtgg agaggtttac acctgccaag tggagcaccc     660
   aagcgtgaca agccctctca cagtggaatg gagagcacgg tctgaatctg cacagagcaa     720
   gatgctgagt ggagtcgggg gctttgtgct gggcctgctc ttccttgggg ccgggctgtt     780
   catctacttc aggaatcaga aaggacactc tggacttcag ccaagaggat tcctgagctg     840
   aagtgcagat gac                                                        853
<210> 3
   <211> 3345
   <212> DNA
   <213> human
<400> 3
   gaattccgtc tcgaccactg aatggaagaa aaggactttt aaccaccatt ttgtgactta      60
   cagaaaggaa tttgaataaa gaaaactatg atacttcagg cccatcttca ctccctgtgt     120
   cttcttatgc tttatttggc aactggatat ggccaagagg ggaagtttag tggacccctg    180
   aaacccatga cattttctat ttatgaaggc caagaaccga gtcaaattat attccagttt     240
   aaggccaatc ctcctgctgt gacttttgaa ctaactgggg agacagacaa catatttgtg     300
   atagaacggg agggacttct gtattacaac agagccttgg acagggaaac aagatctact     360
   cacaatctcc aggttgcagc cctggacgct aatggaatta tagtggaggg tccagtccct     420
   atcaccatag aagtgaagga catcaacgac aatcgaccca cgtttctcca gtcaaagtac     480
   gaaggctcag taaggcagaa ctctcgccca ggaaagccct tcttgtatgt caatgccaca     540
   gacctggatg atccggccac tcccaatggc cagctttatt accagattgt catccagctt     600
   cccatgatca acaatgtcat gtactttcag atcaacaaca aaacgggagc catctctctt     660
   acccgagagg gatctcagga attgaatcct gctaagaatc cttcctataa tctggtgatc     720
   tcagtgaagg acatgggagg ccagagtgag aattccttca gtgataccac atctgtggat     780
   atcatagtga cagagaatat ttggaaagca ccaaaacctg tggagatggt ggaaaactca     840
   actgatcctc accccatcaa aatcactcag gtgcggtgga atgatcccgg tgcacaatat     900
   tccttagttg acaaagagaa gctgccaaga ttcccatttt caattgacca ggaaggagat     960
   atttacgtga ctcagccctt ggaccgagaa gaaaaggatg catatgtttt ttatgcagtt    1020
   gcaaaggatg agtacggaaa accactttca tatccgctgg aaattcatgt aaaagttaaa    1080
   gatattaatg ataatccacc tacatgtccg tcaccagtaa ccgtatttga ggtccaggag    1140
   aatgaacgac tgggtaacag tatcgggacc cttactgcac atgacaggga tgaagaaaat    1200
   actgccaaca gttttctaaa ctacaggatt gtggagcaaa ctcccaaact tcccatggat    1260
   ggactcttcc taatccaaac ctatgctgga atgttacagt tagctaaaca gtccttgaag 1320
   aagcaagata ctcctcagta caacttaacg atagaggtgt ctgacaaaga tttcaagacc 1380
   ctttgttttg tgcaaatcaa cgttattgat atcaatgatc agatccccat ctttgaaaaa 1440
   tcagattatg gaaacctgac tcttgctgaa gacacaaaca ttgggtccac catcttaacc 1500
   atccaggcca ctgatgctga tgagccattt actgggagtt ctaaaattct gtatcatatc 1560
   ataaagggag acagtgaggg acgcctgggg gttgacacag atccccatac caacaccgga 1620
   tatgtcataa ttaaaaagcc tcttgatttt gaaacagcag ctgtttccaa cattgtgttc 1680
   aaagcagaaa atcctgagcc tctagtgttt ggtgtgaagt acaatgcaag ttcttttgcc 1740
   aagttcacgc ttattgtgac agatgtgaat gaagcacctc aattttccca acacgtattc 1800
   caagcgaaag tcagtgagga tgtagctata ggcactaaag tgggcaatgt gactgccaag 1860
   gatccagaag gtctggacat aagctattca ctgaggggag acacaagagg ttggcttaaa 1920
   attgaccacg tgactggtga gatctttagt gtggctccat tggacagaga agccggaagt 1980
   ccatatcggg tacaagtggt ggccacagaa gtaggggggt cttccttaag ctctgtgtca 2040
   gagttccacc tgatccttat ggatgtgaat gacaaccctc ccaggctagc caaggactac 2100
   acgggcttgt tcttctgcca tcccctcagt gcacctggaa gtctcatttt cgaggctact 2160
   gatgatgatc agcacttatt tcggggtccc cattttacat tttccctcgg cagtggaagc 2220
   ttacaaaacg actgggaagt ttccaaaatc aatggtactc atgcccgact gtctaccagg 2280
   cacacagact ttgaggagag ggcgtatgtc gtcttgatcc gcatcaatga tgggggtcgg 2340
   ccacccttgg aaggcattgt ttctttacca gttacattct gcagttgtgt ggaaggaagt 2400
   tgtttccggc cagcaggtca ccagactggg atacccactg tgggcatggc agttggtata 2460
   ctgctgacca cccttctggt gattggtata attttagcag ttgtgtttat ccgcataaag 2520
   aaggataaag gcaaagataa tgttgaaagt gctcaagcat ctgaagtcaa acctctgaga 2580
   agctgaattt gaaaaggaat gtttgaattt atatagcaag tgctatttca gcaacaacca 2640
   tctcatccta ttacttttca tctaacgtgc attataattt tttaaacaga tattccctct 2700
   tgtcctttaa tatttgctaa atatttcttt tttgaggtgg agtcttgctc tgtcgcccag 2760
   gctggagtac agtggtgtga tcccagctca ctgcaacctc cgcctcctgg gttcacatga 2820
   ttctcctgcc tcagcttcct aagtagctgg gtttacaggc acccaccacc atgcccagct 2880
   aatttttgta tttttaatag agacggggtt tcgccatttg gccaggctgg tcttgaactc 2940
   ctgacgtcaa gtgatctgcc tgccttggtc tcccaataca ggcatgaacc actgcaccca 3000
   cctacttaga tatttcatgt gctatagaca ttagagagat ttttcatttt tccatgacat 3060
   ttttcctctc tgcaaatggc ttagctactt gtgtttttcc cttttggggc aagacagact 3120
   cattaaatat tctgtacatt ttttctttat caaggagata tatcagtgtt gtctcataga 3180
   actgcctgga ttccatttat gttttttctg attccatcct gtgtcccctt catccttgac 3240
   tcctttggta tttcactgaa tttcaaacat ttgtcagaga agaaaaaagt gaggactcag 3300
   gaaaaataaa taaataaaag aacagccttt tgcggccgcg aattc 3345
<210> 4
   <211> 1924
   <212> DNA
   <213> human
<400> 4
   ccatgacgcc cgccctcaca gccctgctct gccttgggct gagtctgggc cccaggaccc 60
   gcatgcaggc agggcccttc cccaaaccca ccctctgggc tgagccaggc tctgtgatca 120
   gctgggggag ccccgtgacc atctggtgtc aggggagcct ggaggcccag gagtaccaac 180
   tggataaaga gggaagccca gagccctggg acagaaataa cccactggaa cccaagaaca 240
   aggccagatt ctccatccca tccatgacac agcaccatgc agggagatac cgctgccact 300
   attacagctc tgcaggctgg tcagagccca gcgaccccct ggagctggtg atgacaggat 360
   tctacaacaa acccaccctc tcagccctgc ccagccctgt ggtggcctca ggggggaata 420
   tgaccctccg atgtggctca cagaagggat atcaccattt tgttctgatg aaggaaggag 480
   aacaccagct cccccggacc ctggactcac agcagctcca cagtgggggg ttccaggccc 540
   tgttccctgt gggccccgtg acccccagcc acaggcgtgt ctaggaagcc ctccctcctg 600
   accctgcagg gccctgtcct ggcccctggg cagagcctga ccctccagtg tggctctgat 660
   gtcggctacg acagatttgt tctgtataag gagggggaac gtgacttcct ccagcgccct 720
   ggccagcagc cccaggctgg gctctcccag gccaacttca ccctgggccc tgtgagccgc 780
   tcctacgggg gccagtacag gtgctatggt gcacacaacc tctcctccga gtggtcggcc 840
   cccagtgacc ccctggacat cctgatcaca ggacagatct atgacaccgt ctccctgtca 900
   gcacagccgg gccccacagt ggcctcagga gagaacatga ccctgctgtg tcagtcacgg 960
   gggtattttg acactttcct tctgaccaaa gaaggggcag cccatccccc actgcgtctg 1020
   agatcaatgt acggagctca taagtaccag gctgaattcc ccatgagtcc tgtgacctca 1080
   gcccacgcgg ggacctacag gtgctacggc tcacgcagct ccaaccccca cctgctgtct 1140
   ttccccagtg agcccctgga actcatggtc tcaggacact ctggaggctc cagcctccca 1200
   cccacagggc cgccctccac acctggtctg ggaagatacc tggaggtttt gattggggtc 1260
   tcggtggcct tcgtcctgct gctcttcctc ctcctcttcc tcctcctccg acgtcagcgt 1320
   cacagcaaac acaggacatc tgaccagaga aagactgatt tccagcgtcc tgcaggggct 1380
   gcggagacag agcccaagga caggggcctg ctgaggaggt ccagcccagc tgctgacgtc 1440
   caggaagaaa acctctagcc cacacgatga agacccccag gcagtgacgt atgccccggt 1500
   gaaacactcc agtcctagga gagaaatggc ctctcctccc tcctcactgt ctggggaatt 1560
   cctggacaca aaggacagac aggtggaaga ggacaggcag atggacactg aggctgctgc 1620
   atctgaagcc tcccaggatg tgacctacgc ccagctgcac agcttgaccc ttagacggaa 1680
   ggcaactgag cctcctccat cccaggaagg ggaacctcca gctgagccca gcatctacgc 1740
   cactctggcc atccactagc ccggggggta cgcagacccc acactcagca gaaggagact 1800
   caggactgct gaaggcacgg gagctgcccc cagtggacac cagtgaaccc cagtcagcct 1860
   ggacccctaa cacagaccat gaggagacgc tgggaacttg tgggactcac ctgactcaaa 1920
   gatg 1924
<210> 5
   <211> 1536
   <212> DNA
   <213> human
<400> 5
   gtgacgcgag gctctgcgga gaccaggagt cagactgtag gacgacctcg ggtcccacgt 60
   gtccccggta ctcgccggcc ggagcccccg gcttcccggg gccgggggac cttagcggca 120
   cccacacaca gcctactttc caagcggagc catgtctggt aacggcaatg cggctgcaac 180
   ggcggaagaa aacagcccaa agatgagagt gattcgcgtg ggtacccgca agagccagct 240
   tgctcgcata cagacggaca gtgtggtggc aacattgaaa gcctcgtacc ctggcctgca 300
   gtttgaaatc attgctatgt ccaccacagg ggacaagatt cttgatactg cactctctaa 360
   gattggagag aaaagcctgt ttaccaagga gcttgaacat gccctggaga agaatgaagt 420
   ggacctggtt gttcactcct tgaaggacct gcccactgtg cttcctcctg gcttcaccat 480
   cggagccatc tgcaagcggg aaaaccctca tgatgctgtt gtctttcacc caaaatttgt 540
   tgggaagacc ctagaaaccc tgccagagaa gagtgtggtg ggaaccagct ccctgcgaag 600
   agcagcccag ctgcagagaa agttcccgca tctggagttc aggagtattc ggggaaacct 660
   caacacccgg cttcggaagc tggacgagca gcaggagttc agtgccatca tcctggcaac 720
   agctggcctg cagcgcatgg gctggcacaa ccgggtgggg cagatcctgc accctgagga 780
   atgcatgtat gctgtgggcc aggggccctt gggcgtggaa gtgcgagcca aggaccagga 840
   catcttggat ctggtgggtg tgctgcacga tcccgagact ctgcttcgct gcatcgctga 900
   aagggccttc ctgaggcacc tggaaggagg ctgcagtgtg ccagtagccg tgcatacagc 960
   tatgaaggat gggcaactgt acctgactgg aggagtctgg agtctagacg gctcagatag 1020
   catacaagag accatgcagg ctaccatcca tgtccctgcc cagcatgaag atggccctga 1080
   ggatgaccca cagttggtag gcatcactgc tcgtaacatt ccacgagggc cccagttggc 1140
   tgcccagaac ttgggcatca gcctggccaa cttgttgctg agcaaaggag ccaaaaacat 1200
   cctggatgtt gcacggcagc ttaacgatgc ccattaactg gtttgtgggg cacagatgcc 1260
   tgggttgctg ctgtccagtg cctacatccc gggcctcagt gccccattct cactgctatc 1320
   tggggagtga ttaccccggg agactgaact gcagggttca agccttccag ggatttgcct 1380
   caccttgggg ccttgatgac tgccttgcct cctcagtatg tgggggcttc atctctttag 1440
   agaagtccaa gcaacagcct ttgaatgtaa ccaatcctac taataaacca gttctgaagg 1500
   taaaaaaaaa aaaaaaaaaa aaaaaaaaaa aaaaaa 1536
<210> 6
   <211> 3345
   <212> DNA
   <213> human
<400> 6
   gaattccgtc tcgaccactg aatggaagaa aaggactttt aaccaccatt ttgtgactta 60
   cagaaaggaa tttgaataaa gaaaactatg atacttcagg cccatcttca ctccctgtgt 120
   cttcttatgc tttatttggc aactggatat ggccaagagg ggaagtttag tggacccctg 180
   aaacccatga cattttctat ttatgaaggc caagaaccga gtcaaattat attccagttt 240
   aaggccaatc ctcctgctgt gacttttgaa ctaactgggg agacagacaa catatttgtg 300
   atagaacggg agggacttct gtattacaac agagccttgg acagggaaac aagatctact 360
   cacaatctcc aggttgcagc cctggacgct aatggaatta tagtggaggg tccagtccct 420
   atcaccatag aagtgaagga catcaacgac aatcgaccca cgtttctcca gtcaaagtac 480
   gaaggctcag taaggcagaa ctctcgccca ggaaagccct tcttgtatgt caatgccaca 540
   gacctggatg atccggccac tcccaatggc cagctttatt accagattgt catccagctt 600
   cccatgatca acaatgtcat gtactttcag atcaacaaca aaacgggagc catctctctt 660
   acccgagagg gatctcagga attgaatcct gctaagaatc cttcctataa tctggtgatc 720
   tcagtgaagg acatgggagg ccagagtgag aattccttca gtgataccac atctgtggat 780
   atcatagtga cagagaatat ttggaaagca ccaaaacctg tggagatggt ggaaaactca 840
   actgatcctc accccatcaa aatcactcag gtgcggtgga atgatcccgg tgcacaatat 900
   tccttagttg acaaagagaa gctgccaaga ttcccatttt caattgacca ggaaggagat 960
   atttacgtga ctcagccctt ggaccgagaa gaaaaggatg catatgtttt ttatgcagtt 1020
   gcaaaggatg agtacggaaa accactttca tatccgctgg aaattcatgt aaaagttaaa 1080
   gatattaatg ataatccacc tacatgtccg tcaccagtaa ccgtatttga ggtccaggag 1140
   aatgaacgac tgggtaacag tatcgggacc cttactgcac atgacaggga tgaagaaaat 1200
   actgccaaca gttttctaaa ctacaggatt gtggagcaaa ctcccaaact tcccatggat 1260
   ggactcttcc taatccaaac ctatgctgga atgttacagt tagctaaaca gtccttgaag 1320
   aagcaagata ctcctcagta caacttaacg atagaggtgt ctgacaaaga tttcaagacc 1380
   ctttgttttg tgcaaatcaa cgttattgat atcaatgatc agatccccat ctttgaaaaa 1440
   tcagattatg gaaacctgac tcttgctgaa gacacaaaca ttgggtccac catcttaacc 1500
   atccaggcca ctgatgctga tgagccattt actgggagtt ctaaaattct gtatcatatc 1560
   ataaagggag acagtgaggg acgcctgggg gttgacacag atccccatac caacaccgga 1620
   tatgtcataa ttaaaaagcc tcttgatttt gaaacagcag ctgtttccaa cattgtgttc 1680
   aaagcagaaa atcctgagcc tctagtgttt ggtgtgaagt acaatgcaag ttcttttgcc 1740
   aagttcacgc ttattgtgac agatgtgaat gaagcacctc aattttccca acacgtattc 1800
   caagcgaaag tcagtgagga tgtagctata ggcactaaag tgggcaatgt gactgccaag 1860
   gatccagaag gtctggacat aagctattca ctgaggggag acacaagagg ttggcttaaa 1920
   attgaccacg tgactggtga gatctttagt gtggctccat tggacagaga agccggaagt 1980
   ccatatcggg tacaagtggt ggccacagaa gtaggggggt cttccttaag ctctgtgtca 2040
   gagttccacc tgatccttat ggatgtgaat gacaaccctc ccaggctagc caaggactac 2100
   acgggcttgt tcttctgcca tcccctcagt gcacctggaa gtctcatttt cgaggctact 2160
   gatgatgatc agcacttatt tcggggtccc cattttacat tttccctcgg cagtggaagc 2220
   ttacaaaacg actgggaagt ttccaaaatc aatggtactc atgcccgact gtctaccagg 2280
   cacacagact ttgaggagag ggcgtatgtc gtcttgatcc gcatcaatga tgggggtcgg 2340
   ccacccttgg aaggcattgt ttctttacca gttacattct gcagttgtgt ggaaggaagt 2400
   tgtttccggc cagcaggtca ccagactggg atacccactg tgggcatggc agttggtata 2460
   ctgctgacca cccttctggt gattggtata attttagcag ttgtgtttat ccgcataaag 2520
   aaggataaag gcaaagataa tgttgaaagt gctcaagcat ctgaagtcaa acctctgaga 2580
   agctgaattt gaaaaggaat gtttgaattt atatagcaag tgctatttca gcaacaacca 2640
   tctcatccta ttacttttca tctaacgtgc attataattt tttaaacaga tattccctct 2700
   tgtcctttaa tatttgctaa atatttcttt tttgaggtgg agtcttgctc tgtcgcccag 2760
   gctggagtac agtggtgtga tcccagctca ctgcaacctc cgcctcctgg gttcacatga 2820
   ttctcctgcc tcagcttcct aagtagctgg gtttacaggc acccaccacc atgcccagct 2880
   aatttttgta tttttaatag agacggggtt tcgccatttg gccaggctgg tcttgaactc 2940
   ctgacgtcaa gtgatctgcc tgccttggtc tcccaataca ggcatgaacc actgcaccca 3000
   cctacttaga tatttcatgt gctatagaca ttagagagat ttttcatttt tccatgacat 3060
   ttttcctctc tgcaaatggc ttagctactt gtgtttttcc cttttggggc aagacagact 3120
   cattaaatat tctgtacatt ttttctttat caaggagata tatcagtgtt gtctcataga 3180
   actgcctgga ttccatttat gttttttctg attccatcct gtgtcccctt catccttgac 3240
   tcctttggta tttcactgaa tttcaaacat ttgtcagaga agaaaaaagt gaggactcag 3300
   gaaaaataaa taaataaaag aacagccttt tgcggccgcg aattc 3345
<210> 7
   <211> 1924
   <212> DNA
   <213> human
<400> 7
   ccatgacgcc cgccctcaca gccctgctct gccttgggct gagtctgggc cccaggaccc 60
   gcatgcaggc agggcccttc cccaaaccca ccctctgggc tgagccaggc tctgtgatca 120
   gctgggggag ccccgtgacc atctggtgtc aggggagcct ggaggcccag gagtaccaac 180
   tggataaaga gggaagccca gagccctggg acagaaataa cccactggaa cccaagaaca 240
   aggccagatt ctccatccca tccatgacac agcaccatgc agggagatac cgctgccact 300
   attacagctc tgcaggctgg tcagagccca gcgaccccct ggagctggtg atgacaggat 360
   tctacaacaa acccaccctc tcagccctgc ccagccctgt ggtggcctca ggggggaata 420
   tgaccctccg atgtggctca cagaagggat atcaccattt tgttctgatg aaggaaggag 480
   aacaccagct cccccggacc ctggactcac agcagctcca cagtgggggg ttccaggccc 540
   tgttccctgt gggccccgtg acccccagcc acaggcgtgt ctaggaagcc ctccctcctg 600
   accctgcagg gccctgtcct ggcccctggg cagagcctga ccctccagtg tggctctgat 660
   gtcggctacg acagatttgt tctgtataag gagggggaac gtgacttcct ccagcgccct 720
   ggccagcagc cccaggctgg gctctcccag gccaacttca ccctgggccc tgtgagccgc 780
   tcctacgggg gccagtacag gtgctatggt gcacacaacc tctcctccga gtggtcggcc 840
   cccagtgacc ccctggacat cctgatcaca ggacagatct atgacaccgt ctccctgtca 900
   gcacagccgg gccccacagt ggcctcagga gagaacatga ccctgctgtg tcagtcacgg 960
   gggtattttg acactttcct tctgaccaaa gaaggggcag cccatccccc actgcgtctg 1020
   agatcaatgt acggagctca taagtaccag gctgaattcc ccatgagtcc tgtgacctca 1080
   gcccacgcgg ggacctacag gtgctacggc tcacgcagct ccaaccccca cctgctgtct 1140
   ttccccagtg agcccctgga actcatggtc tcaggacact ctggaggctc cagcctccca 1200
   cccacagggc cgccctccac acctggtctg ggaagatacc tggaggtttt gattggggtc 1260
   tcggtggcct tcgtcctgct gctcttcctc ctcctcttcc tcctcctccg acgtcagcgt 1320
   cacagcaaac acaggacatc tgaccagaga aagactgatt tccagcgtcc tgcaggggct 1380
   gcggagacag agcccaagga caggggcctg ctgaggaggt ccagcccagc tgctgacgtc 1440
   caggaagaaa acctctagcc cacacgatga agacccccag gcagtgacgt atgccccggt 1500
   gaaacactcc agtcctagga gagaaatggc ctctcctccc tcctcactgt ctggggaatt 1560
   cctggacaca aaggacagac aggtggaaga ggacaggcag atggacactg aggctgctgc 1620
   atctgaagcc tcccaggatg tgacctacgc ccagctgcac agcttgaccc ttagacggaa 1680
   ggcaactgag cctcctccat cccaggaagg ggaacctcca gctgagccca gcatctacgc 1740
   cactctggcc atccactagc ccggggggta cgcagacccc acactcagca gaaggagact 1800
   caggactgct gaaggcacgg gagctgcccc cagtggacac cagtgaaccc cagtcagcct 1860
   ggacccctaa cacagaccat gaggagacgc tgggaacttg tgggactcac ctgactcaaa 1920
   gatg 1924
<210> 8
   <211> 1775
   <212> DNA
   <213> human
<400> 8
   agcggccggg gcgagccagc gagagggcgc gagcggcggc gctgcctgca gcctgcagcc 60
   tgcagcctcc ggccggccgg cgagccagtg cgcgtgcgcg gcggcggcct ccgcagcgac 120
   cggggagcgg actgaccggc gggagggcta gcgagccagc ggtgtgaggc gcgaggcgag 180
   gccgagccgc gagcgacatg ggggaccggg agcagctgct gcagcgggcg cggctggccg 240
   agcaggcgga gcgctacgac gacatggcct ccgctatgaa ggcggtgaca gagctgaatg 300
   aacctctctc caatgaagat cgaaatctcc tctctgtggc ctacaagaat gtggttggtg 360
   ccaggcgatc ttcctggagg gtcattagca gcattgagca gaaaaccatg gctgatggaa 420
   acgaaaagaa attggagaaa gttaaagctt accgggagaa gattgagaag gagctggaga 480
   cagtttgcaa tgatgtcctg tctctgcttg acaagttcct gatcaagaac tgcaatgatt 540
   tccagtatga gagcaaggtg ttttacctga aaatgaaggg tgattactac cgctacttag 600
   cagaggtcgc ttctggggag aagaaaaaca gtgtggtcga agcttctgaa gctgcctaca 660
   aggaagcctt tgaaatcagc aaagagcaga tgcaacccac gcatcccatc cggctgggcc 720
   tggccctcaa cttctccgtg ttctactatg agatccagaa tgcacctgag caagcctgcc 780
   tcttagccaa acaagccttc gatgatgcca tagctgagct ggacacacta aacgaggatt 840
   cctataagga ctccacgctg atcatgcagt tgctgcgaga caacctcacc ctctggacga 900
   gcgaccagca ggatgaagaa gcaggagaag gcaactgaag atccttcagg tcccctggcc 960
   cttccttcac ccaccacccc catcatcacc gattcttcct tgccacaatc actaaatatc 1020
   tagtgctaaa cctatctgta ttggcagcac agctactcag atctgcactc ctgtctcttg 1080
   ggaagcagtt tcagataaat catgggcatt gctggactga tggttgcttt gagcccacag 1140
   gagctccctt tttgaattgt gtggagaagt gtgttctgat gaggcatttt actatgcctg 1200
   ttgatctatg ggaaatctag gcgaaagtaa tggggaagat tagaaagaat tagccaacca 1260
   ggctacagtt gatatttaaa agatccattt aaaacaagct gatagtgttt cgttaagcag 1320
   tacatcttgt gcatgcaaaa atgaattcac ccctcccacc tctttcttca attaatggaa 1380
   aactgttaag ggaagctgat acagagagac aacttgctcc tttccatcag ctttataata 1440
   aactgtttaa cgtgaggttt cagtagctcc ttggttttgc ctctttaaat tatgacgtgc 1500
   acaaaccttc ttttcaatgc aatgcatctg aaagttttga tacttgtaac tttttttttt 1560
   ttttggttgc aattgtttaa gaatcatgga tttatttttt gtaactcttt ggctattgtc 1620
   cttgtgtatc ctgacagcgc catgtgtgtc agcccatgtc aatcaagatg ggtgattatg 1680
   aaatgccaga cttctaaaat aaatgttttg gaattcaatg ggtaaataaa tgctgctttg 1740
   gggatattaa aaaaaaaaaa aaaaaaaaaa aaaaa 1775
<210> 9
   <211> 1724
   <212> DNA
   <213> human
<400> 9
   ctttttggag acagattcgc agtggtcgct tcttctcctt ggatttgtta aggattccaa 60
   gtaactctta tttggagaga agacgatctg cacttcgcat tttggcattg acatttaatt 120
   ttagggtcct ttatatagaa gggagagtag ctacatgaat gtgtaagatc ttggaggaag 180
   acagcagaga gagagagaga gatcagagat cccagggtta aaagttggag aaatttcaca 240
   gtacatcatc caaaagagga gtccatgatg gaggcagagg taaacttgga gaggacagga 300
   agatgtcacc caagcgcata gctaaaagaa ggtccccccc agcagatgcc atccccaaaa 360
   gcaagaaggt gaaggtctca cacaggtccc acagcacaga acccggcttg gtgctgacac 420
   taggccaggg cgacgtgggc cagctggggc tgggtgagaa tgtgatggag aggaagaagc 480
   cggccctggt atccattccg gaggatgttg tgcaggctga ggctgggggc atgcacaccg 540
   tgtgtctaag caaaagtggc caggtctatt ccttcggctg caatgatgag ggtgccctgg 600
   gaagggacac atcagtggag ggctcggaga tggtccctgg gaaagtggag ctgcaagaga    660
   aggtggtaca ggtgtcagca ggagacagtc acacagcagc cctcaccgat gatggccgtg    720
   tcttcctctg gggctccttc cgggacaata acggtgtgat tggactgttg gagcccatga    780
   agaagagcat ggtgcctgtg caggtgcagc tggatgtgcc tgtggtaaag gtggcctcag    840
   gaaacgacca cttggtgatg ctgacagctg atggtgacct ctacaccttg ggctgcgggg    900
   aacagggcca gctaggccgt gtgcctgagt tatttgccaa ccgtggtggc cggcaaggcc    960
   tcgaacgact cctggtcccc aagtgtgtga tgctgaaatc caggggaagc cggggccacg   1020
   tgagattcca ggatgccttt tgtggtgcct atttcacctt tgccatctcc catgagggcc   1080
   acgtgtacgg cttcggcctc tccaactacc atcagcttgg aactccgggc acagaatctt   1140
   gcttcatacc ccagaaccta acatccttca agaattccac caagtcctgg gtgggcttct   1200
   ctggtggcca gcaccataca gtctgcatgg attcggaagg aaaagcatac agcctgggcc   1260
   gggctgagta tgggcggctg ggccttggag agggtgctga ggagaagagc atacccaccc   1320
   tcatctccag gctgcctgct gtctcctcgg tggcttgtgg ggcctctgtg gggtatgctg   1380
   tgaccaagga tggtcgtgtt ttcgcctggg gcatgggcac caactaccag ctgggcacag   1440
   ggcaggatga ggacgcctgg agccctgtgg agatgatggg caaacagctg gagaaccgtg   1500
   tggtcttatc tgtgtccagc gggggccagc atacagtctt attagtcaag gacaaagaac   1560
   agagctgatg aagcctctga gggcctggct tctgtcctgc acaacctccc tcacagaaca   1620
   gggaagcagt gacagctgca gatggcagcg ggcctctccc cagccctgag cactgtgtca   1680
   gttcctgcct tttctcatca gcagaacaga atccttttcc tctt                    1724
<210> 10
   <211> 1622
   <212> DNA
   <213> human
<400> 10
   cgttggcgtt tacgtgtgga agagcggaag agttttgctt ttcgtgcgcg ccttcgaaaa     60
   ctgcctgccg ctgtctgagg agtccacccg aaacctcccc tcctccgccg gcagccccgc    120
   gctgagctcg ccgacccaag ccagcgtggg cgaggtggga agtgcgcccg acccgcgcct    180
   ggagctgcgc ccccgagtgc ccatggctac aagggtgctg agcatgagcg cccgcctggg    240
   acccgtgccc cagccgccgg cgccgcagga cgagccggtg ttcgcgcagc tcaagccggt    300
   gctgggcgcc gcgaatccgg cccgcgacgc ggcgctcttc cccggcgagg agctgaagca    360
   cgcgcaccac cgcccgcagg cgcagcccgc gcccgcgcag gccccgcagc cggcccagcc    420
   gcccgccacc ggcccgcggc tgcctccaga ggacctggtc cagacaagat gtgaaatgga    480
   gaagtatctg acacctcagc ttcctccagt tcctataatt ccagagcata aaaagtatag    540
   acgagacagt gcctcagtcg tagaccagtt cttcactgac actgaagggt taccttacag    600
   tatcaacatg aacgtcttcc tccctgacat cactcacctg agaactggcc tctacaaatc    660
   ccagagaccg tgcgtaacac acatcaagac agaacctgtt gccattttca gccaccagag    720
   tgaaacgact gcccctcctc cggccccgac ccaggccctc cctgagttca ccagtatatt    780
   cagctcacac cagaccgcag ctccagaggt gaacaatatt ttcatcaaac aagaacttcc    840
   tacaccagat cttcatcttt ctgtccctac ccagcagggc cacctgtacc agctactgaa    900
   tacaccggat ctagatatgc ccagttctac aaatcagaca gcagcaatgg acactcttaa    960
   tgtttctatg tcagctgcca tggcaggcct taacacacac acctctgctg ttccgcagac   1020
   tgcagtgaaa caattccagg gcatgccccc ttgcacatac acaatgccaa gtcagtttct   1080
   tccacaacag gccacttact ttcccccgtc accaccaagc tcagagcctg gaagtccaga   1140
   tagacaagca gagatgctcc agaatttaac cccacctcca tcctatgctg ctacaattgc   1200
   ttctaaactg gcaattcaca atccaaattt acccaccacc ctgccagtta actcacaaaa   1260
   catccaacct gtcagataca atagaaggag taaccccgat ttggagaaac gacgcatcca   1320
   ctactgcgat taccctggtt gcacaaaagt ttataccaag tcttctcatt taaaagctca   1380
   cctgaggact cacactggtg aaaagccata caagtgtacc tgggaaggct gcgactggag   1440
   gttcgcgcga tcggatgagc tgacccgcca ctaccggaag cacacaggcg ccaagccctt   1500
   ccagtgcggg gtgtgcaacc gcagcttctc gcgctctgac cacctggccc tgcatatgaa   1560
   gaggcaccag aactgagcac tgcccgtgtg acccgttcca ggtcccctgg gctccctcaa   1620
   at                                                                   1622
<210> 11
   <211> 1221
   <212> DNA
   <213> human
<400> 11
   cgcaggctgg aaggaagacg aacctacgaa gcagagatct gaagacagca tgtacacagc     60
   cattccccag agtggctctc cattcccagg ctcagtgcag gatccaggcc tgcatgtgtg    120
   gcgggtggag aagctgaagc cggtgcctgt ggcgcaagag aaccagggcg tcttcttctc    180
   gggggactcc tacctagtgc tgcacaatgg cccagaagag gtttcccatc tgcacctgtg    240
   gataggccag cagtcatccc gggatgagca gggggcctgt gccgtgctgg ctgtgcacct    300
   caacacgctg ctgggagagc ggcctgtgca gcaccgcgag gtgcagggca atgagtctga    360
   cctcttcatg agctacttcc cacggggcct caagtaccag gaaggtggtg tggagtcagc    420
   atttcacaag acctccacag gagccccagc tgccatcaag aaactctacc aggtgaaggg    480
   gaagaagaac atccgtgcca ccgagcgggc actgaactgg gacagcttca acactgggga    540
   ctgcttcatc ctggacctgg gccagaacat cttcgcctgg tgtggtggaa agtccaacat    600
   cctggaacgc aacaaggcga gggacctggc cctggccatc cgggacagtg agcgacaggg    660
   caaggcccag gtggagattg tcactgatgg ggaggagcct gctgagatga tccaggtcct    720
   gggccccaag cctgctctga aggagggcaa ccctgaggaa gacctcacag ctgacaaggc    780
   aaatgcccag gccgcagctc tgtataaggt ctctgatgcc actggacaga tgaacctgac    840
   caaggtggct gactccagcc cctttgccct tgaactgctg atatctgatg actgctttgt    900
   gctggacaac gggctctgtg gcaagatcta tatctggaag ggcgaaaagc cgaatgagaa    960
   ggagcggcag gcagccctgc aggtggccga gggcttcatc tcgcgcatgc agtacgcccc   1020
   gaacactcag gtggagattc tgcctcaggg ccgtgagagt cccatcttca agcaattttt   1080
   caaggactgg aaatgagggt gggcgtcttc ctgccccatg ctcccctgcc ccccaccacc   1140
   tgcctgcttg cttctctggc tgcctggtca gtgcagaggt gccccctgca gatgttcaat   1200
   aaaggagaca agtgctttcc c                                             1221
<210> 12
   <211> 1460
   <212> DNA
   <213> human
<400> 12
   accccatctt catctggcct tgactctgcc cttgaggggc ctaggggtgc agccagcctg     60
   ctccgagctc ccctgcagat ggaggaggcc atcctggtcc cctgcgtgct ggggctcctg    120
   ctgctgccca tcctggccat gttgatggca ctgtgtgtgc actgccacag actgccaggc    180
   tcctacgaca gcacatcctc agatagtttg tatccaaggg gcatccagtt caaacggcct    240
   cacacggttg ccccctggcc acctgcctac ccacctgtca cctcctaccc acccctgagc   300
   cagccagacc tgctccccat cccaagatcc ccgcagcccc ttgggggctc ccaccggacg    360
   ccatcttccc ggcgggattc tgatggtgcc aacagtgtgg cgagctacga gaacgagggt    420
   gcgtctggga tccgaggtgc ccaggctggg tggggagtct ggggtccgtc ctggactagg    480
   ctgacccctg tgtcgttacc cccagaacca gcctgtgagg atgcagatga ggatgaggac    540
   gactatcaca acccaggcta cctggtggtg cttcctgaca gcaccccggc cactagcact    600
   gctgccccat cagctcctgc actcagcacc cctggcatcc gagacagtgc cttctccatg    660
   gagtccattg atgattacgt gaacgttccg gagagcgggg agagcgcaga agcgtctctg    720
   gatggcagcc gggagtatgt gaatgtgtcc caggaactgc atcctggagc ggctaagact    780
   gagcctgccg ccctgagttc ccaggaggca gaggaagtgg aggaagaggg ggctccagat 840
   tacgagaatc tgcaggagct gaactgaggg cctgtggagg ccgagtctgt cctggaacca 900
   ggcttgcctg ggacggctga gctgggcagc tggaagtggc tctggggtcc tcacatggcg 960
   tcctgccctt gctccagcct gacaacagcc tgagaaatcc ccccgtaact tattatcact 1020
   ttggggttcg gcctgtgtcc cccgaacgct ctgcaccttc tgacgcagcc tgagaatgac 1080
   ctgccctggc cccagcccta ctctgtgtaa tagaataaag gcctgcgtgt gtctgtgttg 1140
   agcgtgcgtc tgtgtgtgcc tgtgtgcgag tctgagtcag agatttggag atgtctctgt 1200
   gtgtttgtgt gtatctgtgg gtctccatcc tccatggggg ctcagccagg tgctgtgaca 1260
   ccccccttct gaatgaagcc ttctgacctg ggctggcact gctgggggtg aggacacatt 1320
   gccccatgag acagtcccag aacacggcag ctgctggctg tgacaatggt ttcaccatcc 1380
   ttagaccaag ggatgggacc tgatgacctg ggaggactct tttagttctt acctcttgtg 1440
   gttctcaata aaacagaacg 1460
<210> 13
   <211> 1403
   <212> DNA
   <213> human
<400> 13
   gcttccgctt tggggtggtg gtgccacccg ccgtggccgg cgcccggccg gagctgctgg 60
   tggtggggtc gcggcccgag ctggggcgtt gggagccgcg cggtgccgtc cgcctgaggc 120
   cggccggcac cgcggcgggc gacggggccc tggcgctgca ggagccgggc ctgtggctcg 180
   gggaggtgga gctggcggcc gaggaggcgg cgcaggacgg ggcggagccg ggccgcgtgg 240
   acacgttctg gtacaagttc ctgaagcggg agccgggagg agagctctcc tgggaaggca 300
   atggacctca tcatgaccgt tgctgtactt acaatgaaaa caacttggtg gatggtgtgt 360
   attgtctccc aataggacac tggattgagg ccactgggca caccaatgaa atgaagcaca 420
   caacagactt ctattttaat attgcaggcc accaagccat gcattattca aggccgagta 480
   cagatgctgc cccaggcggt gtgcctgctg catgcgctgc tggagaaggg acacatcgtg 540
   tacgtgcact gcaacgctgg ggtgggccgc tccaccgcgg ctgtctgcgg ctggctccag 600
   tatgtgatgg gctggaatct gaggaaggtg cagtatttcc tcatggccaa gaggccggct 660
   gtctacattg acgaagaggc cttggcccgg gcacaagaag atttttccca gaaatttggg 720
   aaggttcgtt cttctgtgtg tagcctgtag ctggtcagcc tgcttctgcc ccctcctgat 780
   ttccctaagg agcctgggat gatgttggtc aaatgaccta gaaacaagga ttctacctga 840
   actgaaagga ctgtgtgacc tcccccaagc caaccacttt cacctgggat gactttcgat 900
   tatgctttgt tttggggctg tatttttgaa atactctaca agaaagctgt ggctcaacac 960
   atgagaagaa gcacgaagca gttaggctgt acatcagaca gaagggtaat gcgtgcagtt 1020
   cctgctgcct gcaggcagac gaggcctttg ctttacagca ctgtatgtgt tgcacgatgg 1080
   atccgtgaca gcactttcct gttgcactga aactcttggc catgtagagg aaaagatatg 1140
   gagttatgtg gatttcatca ctagtatgtg tgcgtgagct ggtcagttgc caaaggagga 1200
   aataaggtta gaagcctgaa ccgttacaaa agaagagctc actatggtca aaaagtgatg 1260
   gctttcagga cttgtttttt atcctgcctc acagttgtta aagtctgttc caaggcatca 1320
   ccttccttct ctacccaaca accctgtgta acaactaaag tagaattatc tccaaaaaaa 1380
   aaaaaaaaaa aaaaaaaaaa aaa 1403
<210> 14
   <211> 3463
   <212> DNA
   <213> human
<400> 14
   atggctgagc cgactagtga tttcgagact cctatcgggt ggcatgcgtc tcccgagctg 60
   actcccacgt tagggcccct gagcgacact gccccgccgc gggacaggtg gatgttctgg 120
   gcaatgctgc cgccaccgcc accaccactt acgtcctcgc ttcccgcagc cgggtcaaag 180
   ccttcctctg agtcgcagcc ccccatggag gcccagtctc tccccggggc tccgcccccc 240
   ttcgacgccc agattcttcc cggggcgcaa ccccccttcg acgcccagtc tccccttgat 300
   tctcagcctc aacccagcgg ccagccttgg aatttccatg cttccacatc gtggtattgg 360
   agacagtctt ctgataggtt tcctcggcat cagaagtcct tcaaccctgc agttaaaaat 420
   tcttattatc cacgaaagta tgatgcaaaa ttcacagact tcagcttacc tcccagtaga 480
   aaacagaaaa aaaagaaaag aaaggaacca gtttttcact ttttttgtga tacctgtgat 540
   cgtggtttta aaaatcaaga aaagtatgac aaacacatgt ctgaacatac aaaatgccct 600
   gaattagatt gctcttttac tgcacacgag aagattgtcc agttccattg gagaaatatg 660
   catgctcctg gcatgaagaa gatcaagtta gacactccag aggaaattgc acggtggagg 720
   gaagaaagaa ggaaaaacta tccaactctg gccaatattg aaaggaagaa gaagttaaaa 780
   cttgaaaagg agaagagagg agcagtattg acaacaacac aatatggcaa gatgaagggg 840
   atgtccagac attcacaaat ggcaaagatc agaagtcctg gcaagaatca caaatggaaa 900
   aacgacaatt ctagacagag agcagtcact ggatcaggca gtcacttgtg tgatttgaag 960
   ctagaaggtc caccggaggc aaatgcagat cctcttggtg ttttgataaa cagtgattct 1020
   gagtctgata aggaggagaa accacaacat tctgtgatac ccaaggaagt gacaccagcc 1080
   ctatgctcac taatgagtag ctatggcagt ctttcagggt cagagagtga gccagaagaa 1140
   actcccatca agactgaagc agacgttttg gcagaaaacc aggttcttga tagcagtgct 1200
   cctaagagtc caagtcaaga tgttaaagca actgttagaa atttttcaga agccaagagt 1260
   gagaaccgaa agaaaagctt tgaaaaaaca aaccctaaga ggaaaaaaga ttatcacaac 1320
   tatcaaacgt tattcgaacc aagaacacac catccatatc tcttggaaat gcttctagct 1380
   ccggacattc gacatgaaag aaatgtgatt ttgcagtgtg ttcggtacat cattaaaaaa 1440
   gacttttttg gactggatac taattctgcg aaaagtaaag atgtataggc atctggtgtt 1500
   tcagcataca taactgaagc atgtgaaaca gtatcatcct cgttagtaga ggaaaaccaa 1560
   aacccttttt tccgtcaaaa ttggatttgt aattaaattg taagcctcgt aggatgtatg 1620
   ttggaatttt aagtctttcc tttggttcta tgcaaataaa aaaataactg attttttaag 1680
   actgtgtctg tattgttggg attgaatcta gtatttgctg ggagaatttt ttctttgtat 1740
   ttattttaat gtattgttct catgtaagaa tgactgatgt tgtgttagtt aagaattgaa 1800
   gataggttta gcagtaaaga agaaagcttt taaaaggatt gattcagcta agcaaagttg 1860
   ggcagagaaa tacagccatt ttgtttttaa tgcagaaaag gaagatgttc tgtagcaagg 1920
   gggaatattt taaaaataaa ccagatcaaa ttaatacaat cagaaggttt cgaaatgtaa 1980
   atattcctta tttaagacat gtttaaattc acctactagc acgacttaca tagctcaaat 2040
   attgaatgtt taaaatatta atacagatgg ggcctcttta tgtttagata aaattgaagt 2100
   acttaattga agctttttaa aaattgtaaa gtaaatgaaa gctattgaga tctttttgtc 2160
   tcctataata ccagggaatt tgagcttgtg ttctagtcat tgtactagct gtagctattg 2220
   gtctgtcctt ttgacataca gctaaaaggg actaaatttg taaaaaatta gtttgttata 2280
   gttgaagatt aacttttcct aacattgtga ttattgaagt tcatgaatct tgctgtcaag 2340
   gaagaaaggt aagaaagctg atagctcctc catgttggta aaatcctctc cagaatcttg 2400
   gaacacctgg catgtgaccc tagtgacgtc acagacctga gatgaagatt catgtttagc 2460
   cagtgttttc cagccttgta cccaccatac agatctgttt attctgtttc accctactcc 2520
   tccagtgagc cccatatttt gggaaattat ctgccttata cattaactaa ttcaattcat 2580
   gtaacactgt tgagtgctta ctctttgtac ctctattgtg cctatattaa aggtatacaa 2640
   ataaataagg ccatgtctga cttcaaggaa ctcagtttaa ttttgatata ttcaaagatg 2700
   tgattcccaa ccaactcagg atgaagtaac tagtgttaca actgagttga tattctaaaa 2760
   tataacccag tttgtacttt tattactagt tagcatacac attttatggc ttatgggtta 2820
   ataaatgaat tcatggactc ctggactact ttcattgatg accatatctc cagggatgtt 2880
   gttgatcccc acactgcctt aaggtatatt atagaaacag ttttattttc catttttctt 2940
   gtttcctgat aataaatgta tttaggactg aaaatactcc tgagtactcc cctggctgta 3000
   tgtctgacag tctttagcta tggtgactat tgtttatttt taatgggtat ttcagattcc 3060
   aagtgtattt aaaatttcta aggagatata atatagcctg tatggtttct actttatgga 3120
   attatatggt caatatttgt aaatattcta tgagttttgg gtgggtagag gggtgctttg 3180
   cctgttttgg gtacaggttt ttttggattt agcttgttaa ttgttcaaac tttctgcctt 3240
   ctacattcct atcttattgt tcgtttaatc agtttctgaa atgtaagcat tacatgacta 3300
   ttggtgagtt gtgcctttta taactgaaat actttacttt ttctcatatc ctctataatt 3360
   gacttctatt ttccttaatc aaaccagctc tgggaaattt aatacattta tattaattga 3420
   gattattaaa acatttggac tattaaaaaa aaaaaaaaaa aaa 3463
<210> 15
   <211> 5115
   <212> DNA
   <213> human
<400> 15
   gaattccggg agcgggcggg ctgcgaggcc gcggggcatg cgggaggcgg aggggtggga 60
   ccgggtggct gcgcccattc cacacccgcc gaaagcggac actgtcagct gaatcactcc 120
   ccttttagga ggagggaggg ggaaaaggtg tctagctaat ttctgcttaa aaaagcacag 180
   gagatcgcgg gtcagctttg cagtcgctgc cttctcgcgc ctgaccatgc acccctgcat 240
   cttcctgctg ggcacaggcg agcgctttat ttctggagct gagggctaaa acttttttca 300
   cttttcttct cctcaacatc tgaatcatgc catgtgccca gaggagctgg cttgcaaacc 360
   tttccgtggt ggctcagctc cttaactttg gggcgctttg ctatgggaga cagcctcagc 420
   caggcccggt tcgcttcccg gacaggaggc aagagcattt tatcaagggc ctgccagaat 480
   accacgtggt gggtccagtc cgagtagatg ccagtgggca ttttttgtca tatggcttgc 540
   actatcccat cacgagcagc aggaggaaga gagatttgga tggctcagag gactgggtgt 600
   actacagaat ttctcacgag gagaaggacc tgttttttaa cttgacggtc aatcaaggat 660
   ttctttccaa tagctacatc atggagaaga gatatgggaa cctctcccat gttaagatga 720
   tggcttcctc tgcccccctc tgccatctca gtggcacggt tctacagcag ggcaccagag 780
   ttgggacggc agccctcagt gcctgccatg gactgactgg atttttccaa ctaccacatg 840
   gagacttttt cattgaaccc gtgaagaagc atccactggt tgagggaggg taccacccgc 900
   acatcgttta caggaggcag aaagttccag aaaccaagga gccaacctgt ggattaaagg 960
   acagtgttaa catctcccag aagcaagagc tatggcggga gaagtgggag aggcacaact 1020
   tgccaagcag aagcctctct cggcgttcca tcagcaagga gagatgggtg gagacactgg    1080
   tggtggccga cacaaagatg attgaatacc atgggagtga gaatgtggag tcctacatcc    1140
   tcaccatcat gaacatggtc actgggttgt tccataaccc aagcattggc aatgcaattc    1200
   acattgttgt ggttcggctc attctactcg aagaagaaga gcaaggactg aaaatagttc    1260
   accatgcaga aaagacactg tctagcttct gcaagtggca gaagagtatc aatcccaaga    1320
   gtgacctcaa tcctgttcat cacgacgtgg ctgtccttct caccagaaag gacatctgtg    1380
   ctggtttcaa tcgcccctgc gagaccctgg gcctgtctca cctttcagga atgtgtcagc    1440
   ctcaccgcag ttgtaacatc aatgaagatt cgggactccc tctggctttc acaattgccc    1500
   atgagctagg acacagcttc ggcatccagc atgatgggaa agaaaatgac tgtgagcctg    1560
   tgggcagaca tccgtacatc atgtcccgcc agctccagta cgatcccact ccgctgacat    1620
   ggtccaagtg cagcgaggag tacatcaccc gcttcttgga ccgaggctgg gggttctgtc    1680
   ttgatgacat acctaaaaag aaaggcttga agtccaaggt cattgccccc ggagtgatct    1740
   atgatgttca ccaccagtgc cagctacaat atggacccaa tgctaccttc tgccaggaag    1800
   tagaaaacgt ctgccagaca ctgtggtgct ccgtgaaggg cttttgtcgc tctaagctgg    1860
   acgctgctgc agatggaact caatgtggtg agaagaagtg gtgtatggca ggcaagtgca    1920
   tcacagtggg gaagaaacca gagagcattc ctggaggctg gggccgctgg tcaccctggt    1980
   cccactgttc caggacctgt ggggctggag tccagagcgc agagaggctc tgcaacaacc    2040
   ccgagccaaa gtttggaggg aaatattgca ctggagaaag aaaacgctat cgcttgtgca    2100
   acgtccaccc ctgtcgctca gaggcaccaa catttcggca gatgcagtgc agtgaatttg    2160
   acactgttcc ctacaagaat gaactctacc actggtttcc catttttaac ccagcacatc    2220
   cttgtgagct ctactgccga cccatagatg gccagttttc tgagaaaatg ctggatgctg    2280
   tcattgatgg taccccttgc tttgaaggcg gcaacagcag aaatgtctgt attaatggca    2340
   tatgtaagat ggttggctgt gactatgaga tcgattccaa tgccaccgag gatcgctgcg    2400
   gtgtgtgcct gggagatggc tcttcctgcc agactgtgag aaagatgttt aagcagaagg    2460
   aaggatctgg ttatgttgac attgggctca ttccaaaagg agcaagggac ataagagtga    2520
   tggaaattga gggagctgga aacttcctgg ccatcaggag tgaagatcct gaaaaatatt    2580
   acctgaatgg agggtttatt atccagtgga acgggaacta taagctggca gggactgtct    2640
   ttcagtatga caggaaagga gacctggaaa agctgatggc cacaggtccc accaatgagt    2700
   ctgtgtggat ccagcttcta ttccaggtga ctaaccctgg catcaagtat gagtacacaa    2760
   tccagaaaga tggccttgac aatgatgttg agcagatgta cttctggcag tacggccact    2820
   ggacagagtg cagtgtgacc tgcgggacag gtatccgccg ccaaactgcc cattgcataa    2880
   agaagggccg cgggatggtg aaagctacat tctgtgaccc agaaacacag cccaatggga 2940
   gacagaagaa gtgccatgaa aaggcttgtc cacccaggtg gtgggcaggg gagtgggaag 3000
   catgctcggc gacatgcggg ccccacgggg agaagaagcg aaccgtgctg tgcatccaga 3060
   ccatggtctc tgacgagcag gctctcccgc ccacagactg ccagcacctg ctgaagccca 3120
   agaccctcct ttcctgcaac agagacatcc tgtgcccctc ggactggaca gtgggcaact 3180
   ggagtgagtg ttctgtttcc tgtggtggtg gagtgcggat tcgcagtgtc acatgtgcca 3240
   agaaccatga tgaaccttgc gatgtgacaa ggaaacccaa cagccgagct ctgtgtggcc 3300
   tccagcaatg cccttctagc cggagagttc tgaaaccaaa caaaggcact atttccaatg 3360
   gaaaaaaccc accaacacta aagcccgtcc ctccacctac atccaggccc agaatgctga 3420
   ccacacccac agggcctgag tctatgagca caagcactcc agcaatcagc agccctagtc 3480
   ctaccacagc ctccaaagaa ggagacctgg gtgggaaaca gtggcaagat agctcaaccc 3540
   aacctgagct gagctctcgc tatctcattt ccactggaag cacttcccag cccatcctca 3600
   cttcccaatc cttgagcatt cagccaagtg aggaaaatgt ttccagttca gatactggtc 3660
   ctacctcgga gggaggcctt gtagctacaa caacaagtgg ttctggcttg tcatcttccc 3720
   gcaaccctat cacttggcct gtgactccat tttacaatac cttgaccaaa ggtccagaaa 3780
   tggagattca cagtggctca ggggaagaaa gagaacagcc tgaggacaaa gatgaaagca 3840
   atcctgtaat atggaccaag atcagagtac ctggaaatga cgctccagtg gaaagtacag 3900
   aaatgccact tgcacctcca ctaacaccag atctcagcag ggagtcctgg tggccaccct 3960
   tcagcacagt aatggaagga ctgctcccca gccaaaggcc cactacttcc gaaactggga 4020
   cacccagagt tgaggggatg gttactgaaa agccagccaa cactctgctc cctctgggag 4080
   gagaccacca gccagaaccc tcaggaaaga cggcaaaccg taaccacctg aaacttccaa 4140
   acaacatgaa ccaaacaaaa agttctgaac cagtcctgac tgaggaggat gcaacaagtc 4200
   tgattactga gggctttttg ctaaatgcct ccaattacaa gcagctcaca aacggccacg 4260
   gctctgcaca ctggatcgtc ggaaactgga gcgagtgctc caccacatgt ggcctggggg 4320
   cctactggaa aagggtggag tgcaccaccc agatggattc tgactgtgcg gccatccaga 4380
   gacctgaccc tgcaaaaaga tgccacctcc gtccctgtgc tggctggaaa gtgggaaact 4440
   ggagcaagtg ctccagaaac tgcagtgggg gcttcaagat acgcgagatt cagtgcgtgg 4500
   acagccggga ccaccggaac ctgaggccat ttcactgcca gttcctggcc ggcattcctc 4560
   ccccattgag catgagctgt aacccggagc cctgtgaggc gtggcaggtg gagccttgga 4620
   gccagtgctc caggtcctgt ggaggtggag ttcaggagag aggagtgttc tgtccaggag 4680
   gcctctgtga ttggacaaaa agacccacat ccaccatgtc ttgcaatgag cacctgtgct   4740
   gtcactgggc cactgggaac tgggacctgt gttccacttc ctgtggaggt ggctttcaga   4800
   agaggattgt ccaatgtgtg ccctcagagg gcaataaaac tgaagaccaa gaccaatgtc   4860
   tatgtgatca caaacccaga cctccagaat tcaaaaaatg caaccagcag gcctgcaaga   4920
   aaagtgccga tttactttgc actaaggaca aactgtcagc cagtttctgc cagacactga   4980
   aagccatgaa gaaatgttct gtgcccaccg tgagggctga gtgctgcttc tcgtgtcccc   5040
   agacacacat cacacacacc caaaggcaaa gaaggcaacg gttgctccaa aagtcaaaag   5100
   aactctaagc ccaaa                                                    5115
<210> 16
   <211> 528
   <212> DNA
   <213> human
<400> 16
   cgccagggag ctgtgaggca gtgctgtgtg gttcctgccg tccggactct ttttcctcta     60
   ctgagattca tctgtgtgaa atatgagttg gcgaggaaga tcgacctatt attggcctag    120
   accaaggcgc tatgtacagc ctcctgaaat gattgggcct atgcggcccg agcagttcag    180
   tgatgaagtg gaaccagcaa cacctgaaga aggggaacca gcaactcaac gtcaggatcc    240
   tgcagctgct caggagggag aggatgaggg agcatctgca ggtcaagggc cgaagcctga    300
   agctgatagc caggaacagg gtcacccaca gactgggtgt gagtgtgaag atggtcctga    360
   tgggcaggag atggacccgc caaatccaga ggaggtgaaa acgcctgaag aaggtgaaaa    420
   gcaatcacag tgttaaaaga aggcacgttg aaatgatgca ggctgctcct atgttggaaa    480
   tttgttcatt aaaattctcc caataaagct ttacagcctt ctgcaaaa                 528
<210> 17
   <211> 2247
   <212> DNA
   <213> human
<400> 17
   tttcttgagc taggaaaggt ggttggctta cggcacagta gagagcttcc agggctggct     60
   ggcgtgggat acccgtacca cagaaatgca gggaccattg cttcttccag gcctctgctt    120
   tctgctgagc ctctttggag ctgtgactca gaaaaccaaa acttcctgtg ctaagtgccc    180
   cccaaatgct tcctgtgtca ataacactca ctgcacctgc aaccatggat atacttctgg    240
   atctgggcag aaactattca cattcccctt ggagacatgt aacgacatta atgaatgtac    300
   accaccctat agtgtatatt gtggatttaa cgctgtgtgt tacaatgtcg aaggaagttt    360
   ctactgtcaa tgtgtcccag gatatagact gcattctggg aatgaacaat tcagtaattc    420
   caatgagaac acctgtcagg acaccacctc ctcaaagaca accgagggca ggaaagagct 480
   gcaaaagatt gtggacaaat ttgagtcact tctcaccaat cagactttat ggagaacaga 540
   agggagacaa gaaatctcat ccacagctac cactattctc cgggatgtgg aatcgaaagt 600
   tctagaaact gccttgaaag atccagaaca aaaagtcctg aaaatccaaa acgatagtgt 660
   agctattgaa actcaagcga ttacagacaa ttgctctgaa gaaagaaaga cattcaactt 720
   gaacgtccaa atgaactcaa tggacatccg ttgcagtgac atcatccagg gagacacaca 780
   aggtcccagt gccattgcct ttatctcata ttcttctctt ggaaacatca taaatgcaac 840
   tttttttgaa gagatggata agaaagatca agtgtatctg aactctcagg ttgtgagtgc 900
   tgctattgga cccaaaagga acgtgtctct ctccaagtct gtgacgctga ctttccagca 960
   cgtgaagatg acccccagta ccaaaaaggt cttctgtgtc tactggaaga gcacagggca 1020
   gggcagccag tggtccaggg atggctgctt cctgatacac gtgaacaaga gtcacaccat 1080
   gtgtaattgc agtcacctgt ccagcttcgc tgtcctgatg gccctgacca gccaggagga 1140
   ggatcccgtg ctgactgtca tcacctacgt ggggctgagc gtctctctgc tgtgcctcct 1200
   cctggcggcc ctcacttttc tcctgtgtaa agccatccag aacaccagca cctcactgca 1260
   tctgcagctc tcgctctgcc tcttcctggc ccacctcctc ttcctcgtgg ggattgatcg 1320
   aactgaaccc aaggtgctgt gctccatcat cgccggtgct ttgcactatc tctacctggc 1380
   cgccttcacc tggatgctgc tggagggtgt gcacctcttc ctcactgcac ggaacctgac 1440
   agtggtcaac tactcaagca tcaatagact catgaagtgg atcatgttcc cagtcggcta 1500
   tggcgttccc gctgtgactg tggccatttc tgcagcctcc tggcctcacc tttatggaac 1560
   tgctgatcga tgctggctcc acctggacca gggattcatg tggagtttcc ttggcccagt 1620
   ctgtgccatt ttctctgcga atttagtatt gtttatcttg gtcttttgga ttttgaaaag 1680
   aaaactttcc tccctcaata gtgaagtgtc aaccatccag aacacaagga tgctggcttt 1740
   caaagcaaca gctcagctct tcatcctggg ctgcacatgg tgtctgggct tgctacaggt 1800
   gggtccagct gcccaggtca tggcctacct cttcaccatc atcaacagcc tccaaggctt 1860
   cttcatcttc ttggtctact gcctcctcag ccagcaggtc cagaaacaat atcaaaagtg 1920
   gtttagagag atcgtaaaat caaaatctga gtctgagaca tacacacttt ccagcaagat 1980
   gggtcctgac tcaaaaccca gtgaggggga tgtttttcca ggacaagtga agagaaaata 2040
   ttaaaactag aatattcaac tccatatgga aaatcatatc catggatctc tttggcatta 2100
   tgaagaatga agctaaggaa aagggaattc attaaacata tcatccttgg agaggaagta 2160
   atcaaccttt acttcccaag ctgtttgttc tccacaatag gctctcaaca aatgtgtggt 2220
   aaattgcatt tctcttcaaa aaaaaaa 2247
<210> 18
   <211> 1325
   <212> DNA
   <213> human
<400> 18
   accaatcctc acctctcacc tctgtgtccg ccctgctggg aaatattcca ggctttggcc 60
   aaggccagtg cagccccagg ttcccgagcg gcaggttggg tgcggaccat ggcctctcac 120
   aagctgctgg tgaccccccc caaggccctg ctcaagcccc tctccatccc caaccagctc 180
   ctgctggggc ctggtccttc caacctgcct cctcgcatca tggcagccgg ggggctgcag 240
   atgatcgggt ccatgagcaa ggatatgtac cagatcatgg acgagatcaa ggaaggcatc 300
   cagtacgtgt tccagaccag gaacccactc acactggtca tctctggctc gggacactgt 360
   gccctggagg ccgccctggt caatgtgctg gagcctgggg actccttcct ggttggggcc 420
   aatggcattt gggggcagcg agccgtggac atcggggagc gcataggagc ccgagtgcac 480
   ccgatgacca aggaccctgg aggccactac acactgcagg aggtggagga gggcctggcc 540
   cagcacaagc cagtgctgct gttcttaacc cacggggagt cgtccaccgg cgtgctgcag 600
   ccccttgatg gcttcgggga actctgccac aggtacaagt gcctgctcct ggtggattcg 660
   gtggcattcc tgggcgggac ccccctttac atggaccggc aaggcatcga catcctgtac 720
   tcgggctccc agaaggccct gaacgcccct ccagggacct cgctcatctc cttcagtgac 780
   aaggccaaaa agaagatgta ctcccgcaag acgaagccct tctccttcta cctggacatc 840
   aagtggctgg ccaacttctg gggctgtgac gaccagccca ggatgtacca tcacacaatc 900
   cccgtcatca gcctgtacag cctgagagag agcctggccc tcattgcgga acagggcctg 960
   gagaacagct ggcgccagca ccgcgaggcc gcggcgtatc tgcatgggcg cctgcaggca 1020
   ctggggctgc agctcttcgt gaaggacccg gcgctccggc ttcccacagt caccactgtg 1080
   gctgtacccg ctggctatga ctggagagac atcgtcagct acgtcataga ccacttcgac 1140
   attgagatca tgggtggcct tgggccctcc acggggaagg tgctgcggat cggcctgctg 1200
   ggctgcaatg ccacccgcga gaatgtggac cgcgtgacgg aggccctgag ggcggccctg 1260
   cagcactgcc ccaagaagaa gctgtgacct gcccactggc acacagctgg cactggcaca 1320
   cacct 1325
<210> 19
   <211> 2263
   <212> DNA
   <213> human
<400> 19
   agccagaggg acgagctagc ccgacgatgg cccaggggac attgatccgt gtgaccccag     60
   agcagcccac ccatgccgtg tgtgtgctgg gcaccttgac tcagcttgac atctgcagct    120
   ctgcccctga ggactgcacg tccttcagca tcaacgcctc cccaggggtg gtcgtggata    180
   ttgcccacag ccctccagcc aagaagaaat ccacaggttc ctccacatgg cccctggacc    240
   ctggggtaga ggtgaccctg acgatgaaag cggccagtgg tagcacaggc gaccagaagg    300
   ttcagatttc atactacgga cccaagactc caccagtcaa agctctactc tacctcaccg    360
   cggtggaaat ctccctgtgc gcagacatca cccgcaccgg caaagtgaag ccaaccagag    420
   ctgtgaaaga tcagaggacc tggacctggg gcccttgtgg acagggtgcc atcctgctgg    480
   tgaactgtga cagagacaat ctcgaatctt ctgccatgga ctgcgaggat gatgaagtgc    540
   ttgacagcga agacctgcag gacatgtcgc tgatgaccct gagcacgaag acccccaagg    600
   acttcttcac aaaccataca ctggtgctcc acgtggccag gtctgagatg gacaaagtga    660
   gggtgtttca ggccacacgg ggcaaactgt cctccaagtg cagcgtagtc ttgggtccca    720
   agtggccctc tcactacctg atggtccccg gtggaaagca caacatggac ttctacgtgg    780
   aggccctcgc tttcccggac accgacttcc cggggctcat taccctcacc atctccctgc    840
   tggacacgtc caacctggag ctccccgagg ctgtggtgtt ccaagacagc gtggtcttcc    900
   gcgtggcgcc ctggatcatg acccccaaca cccagccccc gcaggaggtg tacgcgtgca    960
   gtatttttga aaatgaggac ttcctgaagt cagtgactac tctggccatg aaagccaagt   1020
   gcaagctgac catctgccct gaggaggaga acatggatga ccagtggatg caggatgaaa   1080
   tggagatcgg ctacatccaa gccccacaca aaacgctgcc cgtggtcttc gactctccaa   1140
   ggaacagagg cctgaaggag tttcccatca aacgagtgat gggtccagat tttggctatg   1200
   taactcgagg gccccaaaca gggggtatca gtggactgga ctcctttggg aacctggaag   1260
   tgagcccccc agtcacagtc aggggcaagg aatacccgct gggcaggatt ctcttcgggg   1320
   acagctgtta tcccagcaat gacagccggc agatgcacca ggccctgcag gacttcctca   1380
   gtgcccagca ggtgcaggcc cctgtgaagc tctattctga ctggctgtcc gtgggccacg   1440
   tggacgagtt cctgagcttt gtgccagcac ccgacaggaa gggcttccgg ctgctcctgg   1500
   ccagccccag gtcctgctac aaactgttcc aggagcagca gaatgagggc cacggggagg   1560
   ccctgctgtt cgaagggatc aagaaaaaaa aacagcagaa aataaagaac attctgtcaa   1620
   acaagacatt gagagaacat aattcatttg tggagagatg catcgactgg aaccgcgagc   1680
   tgctgaagcg ggagctgggc ctggccgaga gtgacatcat tgacatcccg cagctcttca   1740
   agctcaaaga gttctctaag gcggaagctt ttttccccaa catggtgaac atgctggtgc   1800
   tagggaagca cctgggcatc cccaagccct tcgggcccgt catcaacggc cgctgctgcc 1860
   tggaggagaa ggtgtgttcc ctgctggagc cactgggcct ccagtgcacc ttcatcaacg 1920
   acttcttcac ctaccacatc aggcatgggg aggtgcactg cggcaccaac gtgcgcagaa 1980
   agcccttctc cttcaagtgg tggaacatgg tgccctgagc ccatcttccc tggcgtcctc 2040
   tccctcctgg ccagatgtcg ctgggtcctc tgcagtgtgg caagcaagag ctcttgtgaa 2100
   tattgtggct ccctgggggc ggccagccct cccagcagtg gcttgctttc ttctcctgtg 2160
   atgtcccagt ttcccactct gaagatccca acatggtcct agcactgcac actcagttct 2220
   gctctaagaa gctgcaataa agttttttta agtcactttg tac 2263
<210> 20
   <211> 2772
   <212> DNA
   <213> human
<400> 20
   cagtcggcac cggcgaggcc gtgctggaac ccgggcctca gccgcagccg cagcggggcc 60
   gacatgacga cagctcccca ggagcccccc gcccggcccc tccaggcggg cagtggagct 120
   ggcccggcgc ctgggcgcgc catgcgcagc accacgctcc tggccctgct ggcgctggtc 180
   ttgctttact tggtgtctgg tgccctggtg ttccgggccc tggagcagcc ccacgagcag 240
   caggcccaga gggagctggg ggaggtccga gagaagttcc tgagggccca tccgtgtgtg 300
   agcgaccagg agctgggcct cctcatcaag gaggtggctg atgccctggg agggggtgcg 360
   gacccagaaa ccaactcgac cagcaacagc agccactcag cctgggacct gggcagcgcc 420
   ttctttttct cagggaccat catcaccacc atcggctatg gcaatgtggc cctgcgcaca 480
   gatgccgggc gcctcttctg catcttttat gcgctggtgg ggattccgct gtttgggatc 540
   ctactggcag gggtcgggga ccggctgggc tcctccctgc gccatggcat cggtcacatt 600
   gaagccatct tcttgaagtg gcacgtgcca ccggagctag taagagtgct gtcggcgatg 660
   cttttcctgc tgatcggctg cctgctcttt gtcctcacgc ccacgttcgt gttctgctat 720
   atggaggact ggagcaagct ggaggccatc tactttgtca tagtgacgct taccaccgtg 780
   ggctttggcg actatgtggc cggcgcggac cccaggcagg actccccggc ctatcagccg 840
   ctggtgtggt tctggatcct gctcggcctg gcttacttcg cctcagtgct caccaccatc 900
   gggaactggc tgcgagtagt gtcccgccgc actcgggcag agatgggcgg cctcacggct 960
   caggctgcca gctggactgg cacagtgaca gcgcgcgtga cccagcgagc cgggcccgcc 1020
   gccccgccgc cggagaagga gcagccactg ctgcctccac cgccctgtcc agcgcagccg 1080
   ctgggcaggc cccgatcccc ttcgcccccc gagaaggctc agccgccttc cccgcccacg 1140
   gcctcggccc tggattatcc cagcgagaac ctggccttca tcgacgagtc ctcggatacg 1200
   cagagcgagc gcggctgccc gctgccccgc gcgccgagag gtcgccgccg cccaaatccc 1260
   cccaggaagc ccgtgcggcc ccgcggcccc gggcgtcccc gagacaaagg cgtgccggtg 1320
   taggggcagg atccctggcc gggcctctca agggcttcgt ttctgctctc cccggcatgc 1380
   ctggcttgtt tgaccaaaga gccctctttc cacgagactg aagtctgggg aggaggctac 1440
   agttgcctct ccgcctcctc cctggccccg gcccttccct cacttccatc catctctaga 1500
   cccccccaag gctttctgtg tcgctgcccc gggcgggtgt atccctcaca gcacctcacg 1560
   actgtgcctc aaagcctgca tcaataaatg aaaacggtct gcaccgctgc gggcgtgacg 1620
   ctcccggacg cgagtgggtg tggaattgct ttcctcgggc caccgtgggg gcacctctgg 1680
   cctcccgtga cccccaggcc gagggtcccc gggcacccag gtcggtcaag tctcggccct 1740
   ctcaggcccg cgtctctgcc tggaggagac tgtgtagggt ccggcgtggg gatcagccgg 1800
   gatgggctgc gcgtctccag cctctgcaca cacattggcg ggtggggtgc agggagggag 1860
   aggcagggga gagagaatgg catctcgcgt ggagggctgt cgtttgaact ctcccagcgc 1920
   gagagaccct gccccgcccc cttcctggag cgttgactcc cttctcgtct cgaggcctgt 1980
   ggcgtctggg tccgttgggg cagaaccatg gaggaaaagc cttcgaaagt gtcgctcaag 2040
   tcttccgacc gccaaggctc ggacgaggag agcgtgcata gcgacactcg ggacctgtgg 2100
   accacgacca cgctgtccca ggcacagctg aacatgccgc tgtccgaggt ctgcgagggc 2160
   ttcgacgagg agggccgcaa cattagcaag acccgcgggt ggcacagccc ggggcggggc 2220
   tcgttggacg aggggtacaa ggccagccac aagccggagg aactggacga gcacgcgctg 2280
   gtggagctgg agttgcaccg cggcagctcc atggaaatca atctggggga gaaggacact 2340
   gcatcccaga tcgaggccga aaagtcttcc tcaatgtcat cactcaatat tgcgaagcac 2400
   atgccccatc gagcctactg ggcagagcag cagagcaggc tgccactgcc cctgatggaa 2460
   ctcatggaga atgaagctct ggaaatcctc accaaagccc tccggagcta ccagttaggg 2520
   atcggcaggg accacttcct gactaaggag ctgcagcgat acatcgaagg gctcaagaag 2580
   cgccggagca agaggctgta cgtgaattaa aaacgccacc ttgggctcga gcagcgaccc 2640
   gaaccagccc cgtgccagcc cggtccccag acccaagcct gaccccatcc gagtggaatt 2700
   tgagtcctaa agaaataaaa gagtcgatgc atgaaaaaaa aaaaaaaaaa aaaaaaaaaa 2760
   aaaaaaaaaa aa 2772
<210> 21
   <211> 7909
   <212> DNA
   <213> human
<400> 21
   ttcaagtatg gcagacaaag gatgttctgc gtggggaaat gtggtgacac ccatttcaca 60
   aggacagctc acatagattg agtgctcagg aaggaccagc accataccca gtgcctgatg 120
   tgtatcatct caattagtcc ttgcctcaga tgcaaaagga aaccatcgcc atcatcatca 180
   ccaccatcat catcttcctc ctgtgcagat ggaaaggctg aggcatagag aggtgacgga 240
   gtctgcccag gactgcaagc ctgctggtgg cagagccagg ttccaatgga atgaaggctg 300
   tcatcctcag atggcagggt aggcaggtgg ctagagctca cttgggagaa ggggaaagga 360
   cactgacttt ggctagggat ggagcagagc ttgggctggc tttccatgca cgggcagggg 420
   gcgtggctca tggctacgct ccagccccgg gtgtggacat tgaatcttcc aggtctaccc 480
   taggctatgg gtctggacag cactgtgatg gaaagaagac actctatgtc ctgcattctg 540
   tgaccaatga tgtgactgtg ggaatggcgc tggcatctgg ctgccactct gggacgggtg 600
   gccagctgcc atcaggcccc acccaggatg ggaccaccat gcgacttctt ccctcgctcc 660
   tcctggtcat gtccagagcc ccaggaggac cagcaaagcc tctcgagccg atggcagctc 720
   acgttctgcc ttgtcagcta ctcctctcct gggcaatatt ggctgcttgc tgtggctctc 780
   cccggggtat gtgactgcct ctgtgctggg cacctggcct gggctttcct tctgggcctg 840
   ggcagctggg ctcagcttgg acccaggcag cagccacaga ggggcccatg gaggtgacag 900
   agttgcttct atgatggtga acgggcagct gtgacacgga ggaggcgacc actcctcagt 960
   ttccaagtgc tgcggtcagg gccggggcca gcaaagtccc tcccatattc aaagagcggg 1020
   tttgggtttg tcccaggagg acatagtcag gagcccatgc tgggacatgc ctcctccaaa 1080
   gttcagcctg gatccccagc ctctgccaac ggccccgctc cttagctaac ccagcttgct 1140
   cctgggttcc acggcggagt cagatgtttc tgggcagttt cacctttgtg ccttaaatgc 1200
   atgttgagga ctttaaggaa ttgtggagaa atagggctgt ggcaaaggca agtgacaact 1260
   gggaacaatg atcccgcaga ggctgctgag gcctgggccc caggggcgtg ggttcatcct 1320
   tctgcctggg ctttggtggg aggggcagac tctgtggtct gagacacaaa aaaacccaaa 1380
   acatatgtgt gtacagacac acagcagagc cacacacaca cttgtgccca tgcacacact 1440
   cacaggaggc ccgtggactc cgcacaggga agaaactcct ccggtcgaca gtggacggcg 1500
   ctgcagcagg gactcacccc caagccctgc ctgcctccca ttgcccacct ggccctggct 1560
   tgatgggctt atctcatgct gtggccgggg acctcttgct tcctgcaacc ccttgctgga 1620
   ctggggcctg ggcctctcct gggctgtgcc tagggtttgt aacccagggc ctgtgccggc 1680
   gtgcacagag catctctccc tgggaggctc agggctgcct cctcgagctc tgtgggcctg 1740
   cactggccgg tgagcttgtg gtgtgggttt tcaggctgta tccttctacc tcctgagccc 1800
   aggggtccca ggcgccctgc agctgtctcc tcggccatcc tgtggggccc cgaggccttg 1860
   ccctcacttc agtgcctggg tgctcaggct ttgcccaggt gccaggagaa ggtgtgagca 1920
   tgagcctatt ggacacacct ggcgacgtat accaggtgtc ccacccctgc caccatgggg 1980
   cctcccgata cggcaaccac cacggacctg tggggaccaa tgaggaaaga gagaggcagg 2040
   tctgggccag gctcacaggg actccggcat agcagaccct gccccagcag gcccccttgt 2100
   ccttcctggg tcctggtcct tcatgaggaa ctagcccatc cctggtgggg ctcccacccc 2160
   gcttctcagt gggctctatg cttgcctcgt cggagtcacc cctcaggcag tcctgggatc 2220
   ctctccttta gacccactgt gccttcccgg cctcccgggc ttctgctggg ggcagaagaa 2280
   atgcctcccc aggtctgtct ctggaggctc tgagggagat gggcttgggg gctgtaggag 2340
   gaggcaggga ttccagggtg tcaggaaggc aggggtgcca ggtcccacct agtgaagtaa 2400
   taaaccgtgg gtggtgatag tgacccagtg ccctcactgc ccagccccgc ctgtcctcag 2460
   ccagcactgc agggatccca ggcccagact ctggaggcct tcactgatcc cagccacccc 2520
   agaaaagctg cagcctgcag gcaccagccg ggccatatgc ccagtgccag ctagggccca 2580
   ccgcccatcc tgcacacggg gccgctgggc aggtgcccct cacaccccca ggatgtcagt 2640
   gctcacctcg agcaaagcgc cccagctcgg ccttgggagg tggtcatgtc cagggggatg 2700
   atggagagct gtccaaccaa gagagcggga gggagggaag gagggaggga gagagataga 2760
   gagagagaga gagagagagg aagtgtgggc cctaaggctg ccttagtgga ggtgcgcgtg 2820
   gcctgcacct caccaagcct agccactctc gcggctctga gtggctcaca ggcttgtgag 2880
   ggccccgtcg ctgcctgctg ggtccccacc agggctccct ctaggaatgc gccatggctg 2940
   ctatgacaat ttgcacagcc cagtggctta aacaccattt ataccacagg tccagatgaa 3000
   tcctgcaggg ccaaggtctg ggggtgctgg aggccatgct ccctccaggc ttgcggggag 3060
   aacttccctg cctcctccag tctctccatc cctgagctct cggctcctcc tccgtcttca 3120
   gggccagggc gtagcgtctg ctctctcggc ctctgcctcc gcttcccacc tcacctggct 3180
   tctgtctatg tcagtctccc tctgccaacc tcctagaagg acacttgtga ttacattagg 3240
   gctcacccct ttaatccagg ggagcctctc cacttcatga ttttcagcta acttgcttct 3300
   gcacagaccc cctttcccta taagggcaca cattcactgg tcccggggct aaggaccttg 3360
   ctccaagtcc ctccacccat gatgctgtgc cttccagaaa cctgtcctct gcagctcggt 3420
   cttgacccca agcctgctgg tgacctgaac ttcacagggt tatccccttg gactgtgtgc 3480
   agcacgatgc aatttctggg cctgaatgtc atgctccctg gggcaggacc ttgagcctgc 3540
   agcacacact aggccacctg cagtctcaca ggccatgccc tgggtagaca gggaggtgct 3600
   caaccccagc tcgggtcctc tagtctgcct ggctaccatg cttctcactc tcctgcatct 3660
   gcagaccctg cgttgccatg tgaggcaggg gtggggtggg gctgagggcg tggctttggt 3720
   ccctggctgt ccggatgaag taccagagtg acgccacagc ccatcccggt gacatgctca 3780
   cccccaaccc ccgtgtccgg gaccccggtc ttgtgtggtc cctgatgtgg agtcctcagt 3840
   ccttaagata catccagaaa gtcctggcca tgaattggag gtgcagagtc ctgcagagcc 3900
   tctgggctgg gctggtgccc ccaggagatg gagggcctgg tggatgccct cctccctcag 3960
   agctggggca gctgcctccc aggggtggga ctctgggctc agagagaggc ccttgagctg 4020
   cagctcaggg ggatgcgagg cttcgtggac tgtgtcctgg tccatgtggt gcacgtgtct 4080
   ccacctccaa ggagaggctc ctcagtgtgc acctccccca catccgtcct ctctgccggc 4140
   cccgggcgtc tgagcagtca ttccatgcca gcacctctgc agcctgctgg gcctcaggtt 4200
   ctctgtgagg gacctccccg gccttcggcg gaggtggagt aagctccgtc aaggcaggtg 4260
   gcttcgtccc ttcctgtgag tgacaccagt gatgaaatgg acccctccac acaggcatcc 4320
   tcagggcaca gggccctggg ggcaccttcc tcctttcgta tttgttgaga aaaaaagtgg 4380
   cattgcgctc acaccaggat gctggagcag agctgacatg ctcgggaaag ggcagaggtc 4440
   actgggggtg ggaaggtcat ccagtccaga ctcagcacct cgtgggctgg taaactgagg 4500
   ctcaaagtgc tggtgccagg cctgaggcct cgcggtgacc cctctctctg gttcccagca 4560
   cctgcctgag acctgcccca ggcacccata acctggaatt ccctgtttcc ttgtccaggg 4620
   cctgaggaaa tggctcccca ggtctgtctc tggatgctct gaggcagatg ggcttggggg 4680
   ctctaggagg aggcagggac tccagggtgt caggaaggca ggggtgccgg gtcccaccca 4740
   gtggagtaac aaactgtggg tggcgtttgg gcctccccgc cttccccact gggtgtgctg 4800
   gtgctggcgc tgctgggtca gggctgcccg tgaccccaga caccactgtc catcctgtga 4860
   ggctcccgtc tgggcatgtc ctgggtggat tcctcctttc tgttaagtag ctacatgagg 4920
   caggggctcc tggatccaaa gcaaatgaca ggaattccag agccaggtgc atccactcag 4980
   ggcagccagt gttggtggag ctgcctctag cacatggagg agagtgaaag tcagcctgcc 5040
   cctctcacga gaaaagaacc tggggatacc tctcagcctc cagcgttgca agtgcaaggc 5100
   cagtggagtt aatctgcaac gtgcacgagg gcgtgtgtca gtggctgtgt gcaggagtgt 5160
   gagtgagcaa gagcaagagc gcatggctcc tgctgtacct caaggtgtgg gctcctggtg 5220
   gctgctcagt gttcccaggg gtgagaggcc tcatgtatcc taggctgcct gagatttctg 5280
   tgtgctgatc gcatcctcag tttcttgtcc accgcttcac tggcaagagt cccaggctcc 5340
   aaggacaccc tccctgcaca tgattgggtg ttaatggtgg cctgggttgt gtcttcccct 5400
   ggggatgagg gttgggtgtc catggtgccc tgggctgtgt cctcccctag ggatgagggt 5460
   cgggcctcca cgatgccctg ggctgtgtgc tcttatggga atgagggttg ggtgtccaag 5520
   atgccctggg ctgtgtcctt ccctggggat gagggttgga tgtccaagat gccctgggct 5580
   gtgtactccc ctaggaatga gggctgggtg tccaagatac cctgggctgt gtcctcccct 5640
   ggggatgagg gttgggtgtc catggtgccc tgggctgtgt cctcccctgg ggatgacggt 5700
   tgggtgtcca tggtgccctg ggctgtgttt ccttggggat gagggttggg tgctatggca 5760
   tcctgggcag gtgcttcctt tctgcacaag ggttgggtga ccatgatgtc ctggcaatgg 5820
   cttccctggg ttgcctcttt tctgccatgt gggaagagca ggggaggttt agttggtctc 5880
   agcacatcat tctctcagga taagtagaag agtgtctgag ctgtgaggcc agtgctccag 5940
   ctttggaatt gtcttcccca ccctcacctc catcccatca aagcccgaca tgtcgtgtgg 6000
   cagcagcgag gtgggtgttg gctgttctct tgggctgggg gttagtcgtg gacggggaaa 6060
   ggagagatgc tggtcaaagg gcatgaagtt tctgctgatg ggaggagtca gttcttttga 6120
   tctgttgcac agcatggtga ctatagttaa caataatgac tatttcaaaa ttgctaaaag 6180
   atgagatttt aaatgttctc accacaaaat gataagtgtg tgaggtgatg gatatgccac 6240
   ttaccttgtt ttaatcatcc cacaatatag acaggcattg tcactttgca ttgtacccca 6300
   ggaatcttca catttgcttt tttgtcaatt aaaaatagag acacaaaagg agagagggga 6360
   gagcaataga ctcttcacgg aaccgtgggc ttctgcctcc gggtaaaata aactgcaaaa 6420
   aggattccca ggaaaccgtt ccctctttca gcccttggtt acaggaagcc ggatttggga 6480
   aatctgcctg gatgacattc acatgaacgg gcacatacag gaaaacacgg taatgtaatt 6540
   agaatagtca gagaaaagta gccagaaatg acattcacat gaacgggcac atacaggaga 6600
   aaacacggta acgtaattag aatagtcaga gaaaagtagc cagaaatgac attcacatga 6660
   acgggcacat ataggagaaa ccatggtaac gtaattagaa tagtcagaga aaagtagcca 6720
   gaaatgacat tcacatgaac gggcacatac aggaaaacac ggtaatgtaa ttagaatagt 6780
   cagagaaaag tagccagaaa tgacattcac atgaacgggc acatacagga gaaaacacgg 6840
   taacgtaatt agaatagtca gagaaaagta gccagaaatg acattcacat gaacgggcac 6900
   atacaggaga aaacacggta acgtaattag aatagtcaga gaaaagtagc cagaagaatt 6960
   tgcaacgtgc ccttgtaaca ccaaatttga tcagtttttt aaaaaatgat cgttatgtag 7020
   gtgattgaga agtaaatgta ttctttttta aggtaaaaat ttggaccctt atcatgcata 7080
   cccccctctg tgctcttcaa atcaacatca ttattaatat ctgtacattt ttgctcatct 7140
   gagccagcac aggctgaggc tgtcagaatg gacacctttt ggttgttggg tttctgtcag 7200
   tttctggggt gaagctgcgt gattgagaac gtagctcttg gctgccatct cggggattat 7260
   taaggactgt gaactctatc cacaagccat ggcaatatct gtcccaccga atgctccctc 7320
   taacacactc ttactcccgt gatgtgtgtt aagggctccg atgatgctga aaacagcaca 7380
   ggatgtgaaa aggcaggaac agttctgaag tcaaaggctg atgtcctgtt tctctttccc 7440
   tctgtgaccg actcccttcc cagtggtaac aagtacccac agcttggttt gaatttctgc 7500
   acgctgttgt ctgtgcactc gctcacactt acgcacacag caggcatgtg ggcgatgctg 7560
   ggtattttgt gtatgagtgg gatgcacata cacacatcta catccatatc atgcccatgc 7620
   atctgtaact tgcttttccc gtgtaagaac acttcttaga gtttgttcaa tgcatgtgtc 7680
   tgtgtgaatg attgaaggca tttctaaccc attttaaaga tggctactta ggaccatatg 7740
   gatgttgtac tgatgtcatt tgaccacgtc cattgtttcc atcttttggg ctgttcttgt 7800
   gtattttact ttccatgtaa cactgtgaca ttgagaattg gtacctacaa cagtctattt 7860
   gctttacatt aaatttgtag gctaatttgt gtaaaaaaaa aaaaaaaaa 7909
<210> 22
   <211> 1072
   <212> DNA
   <213> human
<400> 22
   agtcagtgaa acggcagaat cagaagaggt tccacaacca gaaaatcttg gctggaattt 60
   caccatcagg aataaaacag aaaaactaaa agagtgcccc agatagcctt tcttaggggc 120
   ctgtgacagg tcgcaggaat cttgttggtg atccatccag atgttgtgtg ttctggaagt 180
   ggacatcgcg gctctgtgtt tttgaagtca gatctcattg ctgtggtttc tatgcctgac 240
   cccccgaagt tcttgctcct gttgccacag ggagccggga gagcacagag cgctgctccc 300
   ggtgccctgc agccacacaa acatgctcct gctcctggcg gaggcagagc tgctgggaaa 360
   gacatttcgg aagtttcctg tggctgcaac aaattgttca aatctgcact ggagcaccgc 420
   tgtgacctgt ctttctccat cttagggcaa acagctcctg aaactggaaa ctccccagca 480
   cctactcacc ctacccctca ggctctcctt gtgggggtgg ggcaggggga gttgtctgga 540
   atgcctggcc tctctgtcca agcatggcag ccttgcccca tgggtggtgc agactcagtt 600
   tcccatgcac cttgccccag ggaggaggta ggggttcctt ccatagagat ggtgaagaat 660
   aagggaggta gtgatcgtct ctgggatcca gttagatctg cgtttgcagg cagaaagagg 720
   ctggggcaca tggagagagt gatcaactgg aagattctag ggtcctcaat tttgaaaggt 780
   gacatgatac cctggaaagg gcatgaactt agttgtcagt tcgtccttgc cttttccaat 840
   caatgctgtg tggccacggc aaattaatga acatctctga gtttcggtct cctgtctaaa 900
   atgaggtgat aatagcttct tgaaggttgt aaggccccaa acatgctgcc tggcacatag 960
   atggctaatc aatattttcc tacccttccc ttccttccct tctctggagt tgctacctgt 1020
   cttctcctgg ggccttgcaa ataaacttct gaattaaaaa aaaaaaaaaa aa 1072
<210> 23
   <211> 417
   <212> DNA
   <213> human
<400> 23
   acctcccaac caagccctcc agcaaggatt caggagtgcc cctcgggcct cgccatgagg 60
   ctcttcctgt cgctcccggt cctggtggtg gttctgtcga tcgtcttgga aggcccagcc 120
   ccagcccagg ggaccccaga cgtctccagt gccttggata agctgaagga gtttggaaac 180
   acactggagg acaaggctcg ggaactcatc agccgcatca aacagagtga actttctgcc 240
   aagatgcggg agtggttttc agagacattt cagaaagtga aggagaaact caagattgac 300
   tcatgaggac ctgaagggtg acatccagga ggggcctctg aaatttccca caccccagcg 360
   cctgtgctga ggactcccgc catgtggccc caggtgccac caataaaaat cctaccg 417
<210> 24
   <211> 1011
   <212> DNA
   <213> human
<400> 24
   ttcctcatta aagtttcaca aataaagcac agcaagactt gtctgcagac acacaggagg 60
   cacacggaca gcccgtcaac cagagatgga gacgaaggcc agcatggctc tcacagggca 120
   gcgcttctca gaacccctgg cccccctcgt gccaaggctg gcctgtgtca ggcctcgccc 180
   acgccgcctt atgacaaata gargccggtg ccaaggaggt ggctacagag caggggcaag 240
   gaagttatcc tcatgttctg ataatgaccc tgcaaatccc accccaccct caggcacctc 300
   cgtctaaggt gtccggttac tccaggtaag gaggttccca ggagggccgt gttttcccta 360
   gggctgatga aacttgctcc gacaagccag gccactggga ggcacctcag gatggaaaag 420
   atgctgagag gctttgctgg ctttcaggat gccgggwgcc ccacgggggc aaaaggggag 480
   gaaggaaaga rattctaaag acagattgct gctggtctgt cccgacccag ggtcacagtg 540
   tcagcaaaga gaacagcatg attctgacag ggttggattt tgtttcaccc tcggaatgag 600
   cagacattca aacacttgca ttttcacgga aatcaacaag agagacagct agcaggacac 660
   gaggctcctg ccagttctgt gtggaaaggc accagatggt ttgttatgaa acacattttg 720
   gtcagaaaat agctggggtt ttttggttcc tgggaggaca acaaagctag aagaaaarga 780
   ggtgtgagtt gcgtgaggag gaggcagaga agaaagcagc tttggcatca gacctgggtt 840
   ctactcttca ctctacccct cmacgcttga ggcctcagtt tcctcatctg taaagtggtc 900
   atagaatatt tccaaataaa tctaggtgtc aggtttcaca catymtccca ggaagtatgg 960
   ggaggcgggg cgcagacact caaacggaca cacagaaacc agaggaagag c 1011
<210> 25
<211> 2123
<212> DNA
<213> human
<400> 25
   tagctgatca tgtgacaatc caagatggcg gtgcccggcg aggcggagga ggaggcgaca     60
   gtttacctgg tagtgagcgg tatcccctcc gtgttgcgct cggcccattt acggagctat    120
   tttagccagt tccgagaaga gcgcggcggt ggcttcctct gtttccacta ccggcatcgg    180
   cctgagcggg cccctccgca ggccgctcct aactctgccc taattcctac cgacccagcc    240
   gctgagggcc agcttctctc tcagacttcg gccaccgatg tccggcctct ctccactcga    300
   gactctactc caatccagac ccgcacctgc tgctgcgtca tctcggtaag ggggttggct     360
   caagctcaga ggcttattcg catgtactcg ggccgccggt ggctggattc tcacgggact    420
   tggctaccgg gtcgctgtct catccgcaga cttcggctac ctacggaggc atcaggtctg    480
   ggcccctttc ccttcaagac ccggaaggaa ctgcagagtt ggaaggcaga gaatgaagcc    540
   ttcaccctgg ctgacctgaa gcaactgccg gagctgaacc caccagtgct gatgcccaga    600
   gggaatgtgg ggactcccct gcgggtcttt ttggagttga tccgggcctg ccgcctaccc    660
   cctcggatca tcacccagct gcagctccag ttccccaaga caggttcctc ccggcgctac    720
   ggcaatgtgc cttttgagta tgaggactca gagactgtgg agcaggaaga gcttgtgtgt    780
   acagcagagg gtgaagaaat accccaagga acctacctgg cagatatacc agccagcccc    840
   tgtggagagc ctgaggaaga agtggggaag gaagaggaag aagagtctca ctcagatgag    900
   gacgatgacc ggggtgagga atgggaacgg catgaagcgc tgcatgagga cgtgaccggg    960
   caggagcgga ccactgagca gctctttgag gaggagattg agctcaagtg ggagaagggt   1020
   ggctctggcc tggtgtttta tactgatgcc cagttctggc aggaggaaga aggagatttt   1080
   gatgaacaga cagccgatga ctgggatgtg gacatgagtg tgtactatga cagagatggt   1140
   ggagacaagg atgcccgaga ctctgtccaa atgcgtctag aacagagact ccgagatgga   1200
   caggaagatg gctctgtgat cgaacgccag gtgggcacct ttgagcgcca caccaagggc   1260
   attgggcgga aggtgatgga gcggcagggc tgggctgagg gccagggcct gggctgcagg   1320
   tgctcagggg tgcctgaggc cctggatagt gatggccaac accccagatg caagcgtgga   1380
   ttggggtacc atggagagaa gctacagcca tttgggcaac tgaagaggcc ccgtagaaat   1440
   ggcttggggc tcatctccac catctatgat gagcctctac cccaagacca gacggagtca   1500
   ctgctccgcc gccagccacc caccagcatg aagtttcgga cagacatggc ctttgtgagg 1560
   ggttccagtt gtgcttcaga cagcccctca ttgcctgact gaccgggttg ggggcttcct 1620
   ttcatagcta catgatgaaa accctctgcc ctggcctcat ctaccactga agcagaaagg 1680
   agtctgggag cagcagtctt cgtggctggt tcagggtgtt ttgttccgag cctgcctgcc 1740
   tgccggttct atacctcagg ggcattttta caaaaagccc cctcccgtcc cctccccttg 1800
   gatattaggg gtaacgaccg cttgtctttg gtctctaacc ctaatctctg ggcttgccct 1860
   ttgcctcctg cagaactttg aaaagctggg ttgagtgagg ctatcagcac agccttcctt 1920
   ggggactctg aaggtgtccc cacgaaggcc agaaaggggg aaagggacct gggcgaggag 1980
   aggatttgtg gtgcttggaa gagccggcct tgggtgggcc ctccaccgcc tctaccctca 2040
   ctgggtggga ctgccagcgg agagtccgcg ggaggtggct tgggtgtgcg acgtcacgga 2100
   agaataaaga cgtttactac tgg 2123
<210> 26
   <211> 1276
   <212> DNA
   <213> human
<400> 26
   ggaatccacc cggggtgtgt ggattcctgc cctgttccca caggacagcc ctcaaccaat 60
   ggagacagga acctggagtt aaatgcttct ccctttttca ctgagagaga gacatgcaca 120
   gtctgatgca ctttctttcc ttctttcttt ttctttcttt ttttttctta agacagagtc 180
   tctctctgtc accaaggctg gagtgcaggg gcacgatctg ggctcactgc cacctccacc 240
   tcccgggttc aagcaattct cccacctcag cctcccgagt agctgggatt acaggcacta 300
   gttaccacgc ccagctaatt tttgtatttt tagtagagat gcggtttcac catattggtc 360
   aggctggtct cagactcctg atctcaggta atctgtctgc ctcagcctcc caaggtgctg 420
   gaattacagg catgagccac cacacctggc cgtgatgcac tttctagatg ctgtcctaga 480
   gatcacactg tgttaagcct cagttgcctt caatgtggtc atctctacag tataccctta 540
   gcttttttct cctccgttac tttcccagac cctcactctg ctccctggat tcacttttcg 600
   aaatagtcct cctgctgcaa agtcctgggc acctgcccta ctttcagcat tggaaggggg 660
   gcccaggcta agaccatgag gccccactgt gggcgcccac agccccgttc ctccctctat 720
   tcccaccaca gtcacatcct cctgtccctc agtgcttcct cgcctttccc tccagcccac 780
   cgtgagatcc caggggacgg agcagcccct tctctgcccc agtgcagggc ttggccttag 840
   cacacggtca gtctgtgctg gggtgaagtg atgaatgagt gagtggttga gtgataatgc 900
   atcatcagat ctgtcttttc cacatgtctc tatctccacc cagaaccagt tttctcatcc 960
   acaaatgggc atttgaggct gggtgctcct aaaccctaca aaattcagag ctggcacagt 1020
   tggggactga ccttccttga tctcacctca ctttctgtat ctataaaatg gggtaccttt 1080
   ctctaagagt aaaaaggagg cctggcatag ggaaagaaac tcagctcgag catccagaac 1140
   atccatcttg ctctcaaata cctaatacag gggaccatgt tttctgctat aattggtatt 1200
   ggagctggta ccatttatta aaggtaattc agttacaaag cttcaaaaaa aaaaaaaaaa 1260
   aaaaaaaaaa aaaaaa 1276
<210> 27
   <211> 7764
   <212> DNA
   <213> human
<400> 27
   ccctgggatg gaggatctgt ctctctctct ctctctcctt tttttttttt tggtggagat 60
   gaaggggtgg gtctatggta catcacctga gttgtggggt aaatgtagag agtgtcaatc 120
   aaaggcagag ctctcagagc tgggaaggag gctctagatg gcggctgtgc cttagagaga 180
   gcgcgctctg ctccctgcct ttgcctcact ttacgcaact ttccctaact ttcgggcagc 240
   ctcagggggc ccccgtagcc ccctgccttt cctagggact tactggggtc gattcgaacc 300
   tttttttggg agaaaagcag cttttaggag ctttcttttc gtgccttgtt ggaaagaagc 360
   agccgtactg agagcccagg tcgttgtttt ttccagctta gaagccatgg cgcacctcca 420
   tttttgtgcg ctctcctaat gaggtttttt ttctttcgga cctgttttag tattaattat 480
   tgctttattt ttttgaccag ttaacatatt tgagggttat tttatttatt tttcgttttt 540
   taacggagga ttttgccttt atttttaatt atttgggatc tgatattttt ctactagtag 600
   ataggactct tggtttggac atactacatg gatcagtaaa tacctgggca caggacttca    660
   aagcaaacac agattccccc tcccccttaa tatttaagaa ttaaaagatg atgagaaata    720
   aggacaaaag ccaagaggag gacagttcgc tacacagcaa tgcatcgagt cactcagcct 780
   ctgaagaagc ttcgggttca gactcaggca gtcagtcgga aagtgagcag ggaagtgatc 840
   caggaagtgg acatggcagc gagtcgaaca gcagctctga atcttctgag agtcagtcgg 900
   aatctgagag cgaatcagca ggttccaaat cccagccagt cctcccagaa gccaaagaga 960
   agccagcctc taagaaggaa cggatagctg atgtgaagaa gatgtgggaa gaatatcctg 1020
   atgtttatgg ggtcaggcgg tcaaaccgaa gcagacaaga accatcgcga tttaatatta 1080
   aggaagaggc aagtagcggg tctgagagtg ggagcccaaa aagaagaggc cagaggcagc 1140
   tgaaaaaaca agaaaaatgg aaacaggaac cctcagaaga tgaacaggaa caaggcacca 1200
   gtgcagagag tgagccagaa caaaaaaaag taaaagccag aagacctgtc cccagaagaa 1260
   cagtgcccaa acctcgtgtt aaaaagcagc cgaagactca gcgtggaaag agaaaaaagc 1320
   aagattcttc tgatgaggat gatgatgatg acgaagctcc caaaaggcag actcgtcgaa 1380
   gagcggctaa aaacgttagt tacaaagaag atgatgactt tgagactgac tcagatgatc 1440
   tcattgaaat gactggagaa ggagttgatg aacagcaaga taatagtgaa actattgaaa 1500
   aggtcttaga ttcaagactg ggaaagaaag gagccactgg agcatctact actgtatatg 1560
   cgattgaagc taatggcgac cctagtggtg actttgacac tgaaaaggat gaaggtgaaa 1620
   tccagtacct catcaagtgg aagggttggt cttacatcca cagcacatgg gagagtgaag 1680
   aatccttaca gcaacagaaa gtgaagggcc taaaaaaact agagaacttc aagaaaaaag 1740
   aggacgaaat caaacaatgg ttagggaaag tttctcctga agatgtagaa tatttcaatt 1800
   gccaacagga gctggcttca gagttgaata aacagtatca gatagtagaa agagtaatag 1860
   ctgtgaagac aagtaaatct acattgggtc aaacagattt tccagctcat agtcggaagc 1920
   cggcaccctc aaatgagccc gaatatctat gtaaatggat gggactcccc tattcagagt 1980
   gtagctggga agatgaagcc ctcattggaa agaaattcca gaattgcatt gacagcttcc 2040
   acagtaggaa caactcaaaa accatcccaa caagagaatg caaggccctg aagcagagac 2100
   cacgatttgt agctttaaag aaacaacctg catatttagg aggggagaat ctggaacttc 2160
   gagattatca gctagaaggt ctaaactggc tagctcattc ctggtgcaaa aataatagtg 2220
   taatccttgc tgatgaaatg ggcctaggaa agaccatcca gaccatatca ttcctctcct 2280
   acctgttcca ccaacaccag ctgtatggcc cctttcttat agtcgtccct ttatccaccc 2340
   tcacctcatg gcagagagag tttgaaatct gggcaccaga gattaacgta gtggtttaca 2400
   taggtgacct gatgagcaga aatacgatac gggaatatga atggattcat tcccaaacca 2460
   aaagattgaa gttcaacgca cttataacaa catatgagat cctcttgaaa gataagactg 2520
   tgctgggcag tattaactgg gcctttctgg gagtggatga agcccatcgg ttgaagaatg 2580
   atgactcttt attgtataaa actctgattg atttcaagtc caaccatagg ctcctgatta 2640
   cggggacccc tcttcagaat tccctcaaag agctctggtc cttgctgcac tttattatgc 2700
   cggagaagtt tgaattttgg gaagattttg aagaagacca tgggaagggg agagagaatg 2760
   gctaccagag tcttcataag gtgctagagc ctttccttct ccggagagtc aaaaaagatg 2820
   tggagaaatc ccttcctgct aaagtggaac agattctcag ggtggagatg tcagcccttc 2880
   agaaacagta ttacaagtgg attctgacca ggaattacaa ggctcttgcc aaaggaacaa 2940
   gaggcagcac atctggtttt cttaatattg tgatggaact gaaaaaatgt tgcaaccact 3000
   gctatctgat taaaccccct gaagaaaatg aaagggaaaa tggacaggag attcttctgt 3060
   ccctcataag gagcagtggg aagttgattt tattagacaa actgttgaca agacttcgag 3120
   aaagggggaa tcgagtgctt atcttctctc agatggtgag aatgttggat atcctggctg 3180
   aatacctaac tattaaacac tatcctttcc agcgtctgga tggttccatc aagggagaaa 3240
   tccgaaaaca ggcactggac cacttcaatg cagatgggtc tgaggacttc tgtttcctgc 3300
   tctcgacaag ggctggtggc ctgggaatca atttggcttc agcggacaca gtcgtcatct 3360
   ttgactctga ctggaacccc cagaatgact tgcaggcaca agcccgagcg catagaattg 3420
   gtcagaagaa gcaggtaaat atttaccgct tagttacaaa ggggactgtg gaggaggaga 3480
   tcatagaacg ggccaaaaag aagatggtat tagatcatct ggtgattcag cgcatggaca 3540
   ccactggccg gacgatcctg gaaaacaact caggaaggtc caactcaaat ccttttaata 3600
   aagaagagct gacagctatt ttgaaatttg gagcagagga tctcttcaaa gaactggaag 3660
   gggaggaatc agaacctcag gaaatggata tagatgaaat tttgcggttg gctgaaacga 3720
   gagagaatga agtgtcaaca agtgcaacag atgaacttct atcacagttt aaggttgcca 3780
   actttgcaac aatggaagat gaagaagagc tagaagagcg tcctcacaag gactgggatg 3840
   agatcattcc agaggaacaa aggaaaaaag tagaggagga agagcggcag aaggagctag 3900
   aagaaattta tatgctgcct cgaattcgga gttccactaa aaaggctcag acaaatgaca 3960
   gtgactctga cactgagtct aagaggcagg cccagagatc ctctgcttct gagagtgaaa 4020
   cggaagactc tgatgatgac aagaagccaa agcgcagagg gcgtccgagg agtgtgcgga 4080
   aggacctcgt ggagggattt actgatgcag agatccgaag gttcatcaag gcttataaga 4140
   agtttggtct ccctcttgaa cggctggagt gcttagcacg tgatgctgag ctggtagata 4200
   agtcggtggc agatctgaag cgcctgggtg aactgatcca caacagctgt gtgtcagcaa 4260
   tgcaggaata tgaagagcag ctgaaagaaa atgccagcga gggaaaagga ccagggaaaa 4320
   ggagaggtcc aacaatcaag atatccggag ttcaggttaa tgtgaaatcc attatccaac 4380
   atgaagagga gtttgagatg ctgcataaat ctatccctgt ggaccctgaa gaaaaaaaaa 4440
   aatactgctt aacctgtcgt gtcaaagctg cacattttga tgtagagtgg ggggtggaag 4500
   atgattctcg cctgttgctg gggatttatg aacatggcta tggaaactgg gagttaatta 4560
   aaacagaccc agagcttaaa ttaactgaca aaattctgcc ggtggagaca gataaaaagc 4620
   ctcaggggaa gcagctacag acccgagcgg attacttgtt gaagctgctc agaaagggtc 4680
   tggagaagaa gggggctgtg acaggtgggg aggaggccaa attaaagaag cggaagcctc 4740
   gggtaaagaa ggaaaacaaa gtgcccaggc tgaaagagga gcatggaatt gagctttcat 4800
   ctcctaggca ttcagataat ccatcagaag agggagaagt gaaagatgat ggcttggaaa 4860
   aaagtccaat gaaaaaaaaa cagaagaaga aagagaacaa ggagaacaag gagaaacaaa 4920
   tgagttctag gaaagacaaa gaaggggaca aggaaagaaa gaagtcaaaa gataagaaag   4980
   agaagcctaa aagtggtgat gccaaatctt cgagtaaatc aaagcgatct cagggtcctg   5040
   tccatattac agcaggaagt gaacctgtcc ccattggaga ggatgaggat gatgatctgg   5100
   accaggagac attcagcata tgtaaggaga ggatgaggcc cgtgaaaaag gcactgaaac   5160
   agctcgacaa acctgacaag gggctcaacg tgcaagaaca gctggaacac acccggaact   5220
   gcctgctgaa aatcggagac cggatagccg agtgccttaa agcctactca gatcaggagc   5280
   acatcaaact ctggaggagg aacctatgga tttttgtttc caagtttaca gaatttgatg   5340
   ctcgaaaact gcataagtta tacaagatgg ctcataagaa aaggtctcaa gaagaagagg   5400
   agcaaaagaa gaaagacgac gtgactgggg gtaagaaacc atttcgtcca gaggcctcag   5460
   gctccagccg ggactctctg atatctcagt cccatacctc acacaacctt caccctcaga   5520
   agcctcattt gcctgcctcc catggcccac agatgcatgg acacccaaga gataactaca   5580
   atcaccccaa caagagacac ttcagtaatg cagatcgagg agactggcag agggaaagaa   5640
   agttcaacta tggtggtggc aacaacaatc caccatgggg aagcgacagg caccatcagt   5700
   atgagcagca ctggtacaag gaccaccatt atggggaccg gcgacatatg gatgcccacc   5760
   gttccggaag ctatcgaccc aacaacatgt ccagaaagag gccttatgac cagtacagca   5820
   gtgaccgaga ccaccgggga cacagagatt attatgacag gtatgcaaaa ggctgtgaga   5880
   caccaggtgc caacctttgc caggagctgt ttctagggag aaagtgacgt atacatgaat   5940
   gtatttatct atcaaattac tgaagatctc atcatgcatg tgtcagccac agcgaatccc   6000
   atgtcttggt tataggtttt atgttttgtt ttctgggtca tagggagcac atttcacctg   6060
   tgcaggaaaa gagttttctg ccgtcttttg aggaaatcta gtgaagaggt cgccataaaa   6120
   tattagagtc aacaaccaaa attattaagc tctgtgcgag gctgtcagcc acactaggta   6180
   tcagggatcc cgagatgggt accagcccac agtccttacc tgccacgagc ccataattga   6240
   agagtcaaag tcttctgaag ctgcaccctc tttacttcag tacaatgcca ccagtagtac   6300
   gatgagccaa agctttacat tgtgagagta gcaagtccag ggagagctaa agaggtttta   6360
   tctgtatttc ctaatttcaa atcttggata atttaacctc atagcagctt tggttttccc   6420
   tgggctgatg atgtgcgtca tttgcactgt accttgaatt tacagtggga aaatttcata   6480
   taaacgtgtc aaagtcgtgc tttgtttttg gaagatctgg taacagcagc ccgcattagc   6540
   agagagctgt agctgagtag ctgccacctc gttgggagac tgcccctcgc tcccaccctt   6600
   ctctattgtc tggacccagt gggcatcttg ccctgcgttc ttctagtagg tctgtatttc   6660
   tatttgatgt cactttcctt ttgcctgaag gactttttct gctggtgata aactctttca   6720
   gtgtttgtat atatgcctga aaaagtattt tgccttcatt tttgaaagta gtttttgctg 6780
   agtgtataca tttttggctt tacagtttct ttcagtgctt taaagatgta cctctgctat 6840
   ttacttgcat tgttttgtga tgaaaaatct gtcatcctta tctttgttcc tctttacata 6900
   atgttccttt taaaaaaaat cactgattat gatgtgcctt ggtgtatttt tccttggttt 6960
   cttgtgcttg gaaatttttg aacttcttgg atctgtgggt ttattgtttc cataaaattt 7020
   ggaaattttt acaatcttct tcaaatattt tttctgatcc cccactctct cttcttcttt 7080
   ggagattctc attacaccta tattagcttg cttgaagttg tctcacagct cacttgtatt 7140
   ctgttgactt ttaaaaaatt atgctttctg tttcactgtg gatagtttct attgctacct 7200
   cttcaagttc actaatactt tccttttcaa tgtcaagact gctgtgaggc ccatccagtg 7260
   tactttgcat tttatacatt gtagttctaa aagttcggaa agttgttttt gggtcttttt 7320
   atatatgttc tgtgtctaac cttttaaaac ctggaacaca gatataacaa tggttttgat 7380
   gtccttgtct gcgaatctta tcacttgggt cagtttcagt tgatacctcc tcactgtggg 7440
   tcttgctccc ctggtgcttt ctgtgcctag taatttttgt cagatgccag atgtaacatt 7500
   taccttgttg ggtgctggat atttctgtat tcctgtaagt attctggagc tttgttatga 7560
   gttgcaggtt atttggaagc agtttccttt ttcaggtctt gctgttaaga ttcgttaggt 7620
   agaaccagag cagtgctcag tcaagggcta atgattgccc acccccaagg taaagagcct 7680
   cattgcactc tacccaattg cgttagtctg ttttgcagga atacctgagg ctgggtaatt 7740
   tatagagaaa agagttttat ttgg 7764
<210> 28
   <211> 3000
   <212> DNA
   <213> human
<400> 28
   ggcagcgtcc gcgggaggtg aggtggctgt ggggacccag gtggcctctt ccctggggcc 60
   ttgctaatga cggcaaaatc cgggttctgc caaaatatat ttaaaaaggt ttattcctag 120
   tcagtatgag tgactgtggc ccaggttatt cagcctcaag aggtcctgtg aaagtgcccg 180
   agatggtcag gcttgcaggt taattttata caattcaggg agacaggaat ttcaggtaaa 240
   gtcataaatc aggctgagca gtgtggctca tgcctgtggt cccagcactt tgggaggcca 300
   ggagttccag agcagcctgg gcagcacagc aagaccctgt ctctacatga aattagaaaa 360
   ataaaaaaat tagcggggcg tggtgtccca tgcctgtggc ctcagctact tgggaggccc 420
   agtcagttga gtccaggagg tggaggctgt aaccagctat gttggctgca ctgcacgcta 480
   gcctgggtaa cacagcgaga tcctgcctcc aaaaagaaaa tcataaatca ataagagaaa 540
   gatatacacg ggttcctccc aaaaagctgg tatatctcca aagggtttac acctcatggg 600
   ggcacttagg gattctttag tggacagttg gttgagagac ttaagctact gcctgaagac 660
   tggaatcaga agcatgccag agttaagggg attgcgtaga tcaaagttct tattatgtag 720
   atgaagcctc ttagttggca actctcagaa tagatggtaa atgtctgttt tcagtttttt 780
   gggtttttgt gtttttgttt ttgtttttag agagagtctt gctctgtcgc ccaggctaga 840
   gtgcagtggc gtgatctcag ctcactgcaa cctccacctc ccaggtttga gcggttctcc 900
   tgcctcggcc tcctgggtag ctgggactac gggcgcccgc caccacgcct ggctaatttt 960
   tgtattttta gtggagatgg ggtttcacca tgttgctgag gctggtcttg acttcctgac 1020
   ctcaggtgat ccgcccacct ctacctccca aagtgctggg attacaggcg tgggccaccg 1080
   cgcgtcaggc tggctgtctc ttccagacct aagaaaggct tagaacaaag gaggtctggc 1140
   tacattaatg gagattcgct gcagatgcaa attttcccac taaagatagc tttgcggggc 1200
   tatccatttc aatctgttgc ccctgtggca gccacttcaa aacatgtcaa agaagtatat 1260
   tttggggtaa aataatttcc ttcagcatct gctgtcatgt gatgctgtac cagagtcagg 1320
   ttggaaagtg agcctcatta tataagagta ataaaactca tctgatgaga ttttatggtt 1380
   tctcgggcag gattccccaa gcctcataca taggcatttg ggcaagggaa aaaaggtgaa 1440
   tttagtcctc accaggttgg tagggcttcc tcggttattg gagtgggagt aacagcaacc 1500
   attgggccca gcagtttttt taaatgtctc tggggctgtg gactgaccat ccaaataact 1560
   gattttaatc atttcattat ggaaaaattg tcagcagaac ccccaagtag agagacccat 1620
   cagtcaagat atacctcatg accttgcaag ctaatctagc ttgacccaga tcccctccta 1680
   atctgtgcag attcattgag gaatgtcata gccatgccta ctggttaaga catagtcctt 1740
   tacagtgaga gttgaaaccc aagctctatc actttcttgg ctgtgttgct ttgagaaagg 1800
   catttaaatg ttttgtgcct gtttcctcat ctgaaattgg tgggtaatag tcacttcata 1860
   ggacagttgt gaagattgaa tgcagaaaaa tttgtgccac gcctggaacc gtccctggca 1920
   tatattaaat tctaaaaaag tgttaaatat tataatgaat atcaacactt ccttattctg 1980
   gaagcaccga caggatatgc tgtgtttagt gttagcatca tgtcaggaca gggtctgttg 2040
   cgatgcccac actcaggatc tgttcccagg aacctgcgta aagttttctt ctctggaaga 2100
   ctttgggtcc ttttttttta acaagaagag gctctaccct gggactggga atttccaagg 2160
   ccacctttga ggatcgcaga gctcatttta gagccatttt agtccccagc tcctcttcct 2220
   ccactcccac gttacccgtg agaggactgt ctgcagggta agggaggaca gcccaacccc 2280
   aggtggggac ttcttatgta ttgccttcct gcagtgcctt ctctgcccta aaccatggtg 2340
   ggtttccttt gctaatgtct gacatcttgt gccctacact gtcccatctg aggctcagaa 2400
   cctctcagcc ggttctcatg gggaacgttc cccagatctg atgccctcat tcaggacact 2460
   tccatcattg tccctacatt tcttctctca gtgctttatt caggctgctg cattcgtggt 2520
   gcagaccagg tcttgtaaaa aattattcag tcagcatgtg ctgagccatt gtcctgtccc 2580
   agggacaggg ctttatagtc attgccctat tcatctcttc aaccaatgtg gaagttagga 2640
   attggaatcc ccatttcaca gactaagaag tggcgtgtta atcagttgaa ataattttta 2700
   cggcttggcg tggtggccca tacctgtaat cccagcactt tgggaggccg gggcgggcgg 2760
   attacctgag gccaggggtt cgagaccagc ctggccaaca tggtgaaacc tcatctctgc 2820
   tgggaataca gaaattagcc aggcatggtg gctcacgcct gtagtcccaa ctgctctgga 2880
   gcctgaagca ggataatcgc ttgaatccag gagatggagg ttgcagtgag cagagagcat 2940
   gccactgcac tacagcctga gcaagagtga gactccgtca caaaaaaaaa aaaaaaaaaa 3000
<210> 29
   <211> 489
   <212> DNA
   <213> human
<400> 29
   agagccgcag gtcagtcgtg aagagggagc tctattgcca ccatgagttt ctccggcaag 60
   taccaactgc agagccagga aaactttgaa gccttcatga aggcaatcgg tctgccggaa 120
   gagctcatcc agaaggggaa ggatatcaag ggggtgtcgg aaatcgtgca gaatgggaag 180
   cacttcaagt tcaccatcac cgctgggtcc aaagtgatcc aaaacgaatt cacggtgggg 240
   gaggaatgtg agctggagac aatgacaggg gagaaagtca agacagtggt tcagttggaa 300
   ggtgacaata aactggtgac aactttcaaa aacatcaagt ctgtgaccga actcaacggc 360
   gacataatca ccaataccat gacattgggt gacattgtct tcaagagaat cagcaagaga 420
   atttaaacaa gtctgcattt catattattt tagtgtgtaa aattaatgta ataaagtgaa 480
   ctttgtttt 489
<210> 30
   <211> 1699
   <212> DNA
   <213> human
<400> 30
   aggtgagcgg ttgctcgtcg tcggggcggc cggcagcggc ggctccaggg cccagcatgc 60
   gcgggggacc ccgcggccac catgtatgtg ggctatgtgc tggacaagga ttcgcccgtg 120
   taccccggcc cagccaggcc agccagcctc ggcctgggcc cggcaaacta cggccccccg 180
   gccccgcccc cggcgccccc gcagtacccc gacttctcca gctactctca cgtggagccg 240
   gcccccgcgc ccccgacggc ctggggggcg cccttccctg cgcccaagga cgactgggcc 300
   gccgcctacg gcccgggccc cgcggcccct gccgccagcc cagcttcgct ggcattcggg 360
   ccccctccag actttagccc ggtgccggcg ccccctgggc ccggcccggg cctcctggcg 420
   cagcccctcg ggggcccggg cacaccgtcc tcgcccggag cgcagaggcc gacgccctac 480
   gagtggatgc ggcgcagcgt ggcggccgga ggcggcggtg gcagcggtaa gactcggacc 540
   aaggacaagt accgcgtggt ctacaccgac caccaacgcc tggagctgga gaaggagttt 600
   cattacagcc gttacatcac aatccggcgg aaatcagagc tggctgccaa tctggggctc 660
   actgaacggc aggtgaagat ctggttccaa aaccggcggg caaaggagcg caaagtgaac 720
   aagaagaaac agcagcagca acagccccca cagccgccga tggcccacga catcacggcc 780
   accccagccg ggccatccct ggggggcctg tgtcccagca acaccagcct cctggccacc 840
   tcctctccaa tgcctgtgaa agaggagttt ctgccatagc cccatgccca gcctgtgcgc 900
   cgggggacct ggggactcgg gtgctgggag tgtggctcct gtgggcccag gaggtctggt 960
   ccgagtctca gccctgacct tctgggacat ggtggacagt cacctatcca ccctctgcat 1020
   ccccttggcc cattgtgtgc agtaagcctg ttggataaag accttccagc tcctgtgttc 1080
   tagacctctg ggggataagg gagtccaggg tggatgatct caatctcccg tgggcatctc 1140
   aagccccaaa tggttggggg aggggcctag acaaggctcc aggccccacc tcctcctcca 1200
   tacgttcaga ggtgcagctg gaggcctgtg tggggaccac actgatcctg gagaaaaggg 1260
   atggagctga aaaagatgga atgcttgcag agcatgacct gaggagggag gaacgtggtc 1320
   aactcacacc tgcctcttct gcagcctcac ctctacctgc ccccatcata agggcactga 1380
   gcccttccca ggctggatac taagcacaaa gcccatagca ctgggctctg atggctgctc 1440
   cactgggtta cagaatcaca gccctcatga tcattctcag tgagggctct ggattgagag 1500
   ggaggccctg ggaggagaga agggggcaga gtcttcccta ccaggtttct acacccccgc 1560
   caggctgccc atcagggccc agggagcccc cagaggactt tattcggacc aagcagagct 1620
   cacagctgga caggtgttgt atatagagtg gaatctcttg gatgcagctt caagaataaa 1680
   tttttcttct cttttcaaa 1699
<210> 31
   <211> 2612
   <212> DNA
   <213> human
<400> 31
   gctgatagca cagttctgtc cagagaagga aggcggaata aacttattca ttcccaggaa 60
   ctcttggggt aggtgtgtgt ttttcacatc ttaaaggctc acagaccctg cgctggacaa 120
   atgttccatt cctgaaggac ctctccagaa tccggattgc tgaatcttcc ctgttgccta     180
   gaagggctcc aaaccacctc ttgacaatgg gaaactgggt ggttaaccac tggttttcag     240
   ttttgtttct ggttgtttgg ttagggctga atgttttcct gtttgtggat gccttcctga     300
   aatatgagaa ggccgacaaa tactactaca caagaaaaat ccttgggtca acattggcct     360
   gtgcccgagc gtctgctctc tgcttgaatt ttaacagcac gctgatcctg cttcctgtgt     420
   gtcgcaatct gctgtccttc ctgaggggca cctgctcatt ttgcagccgc acactgagaa     480
   agcaattgga tcacaacctc accttccaca agctggtggc ctatatgatc tgcctacata     540
   cagctattca catcattgca cacctgttta actttgactg ctatagcaga agccgacagg     600
   ccacagatgg ctcccttgcc tccattctct ccagcctatc tcatgatgag aaaaaggggg     660
   gttcttggct aaatcccatc cagtcccgaa acacgacagt ggagtatgtg acattcacca     720
   gcgttgctgg tctcactgga gtgatcatga caatagcctt gattctcatg gtaacttcag     780
   ctactgagtt catccggagg agttattttg aagtcttctg gtatactcac caccttttta     840
   tcttctatat ccttggctta gggattcacg gcattggtgg aattgtccgg ggtcaaacag     900
   aggagagcat gaatgagagt catcctcgca agtgtgcaga gtcttttgag atgtgggatg     960
   atcgtgactc ccactgtagg cgccctaagt ttgaagggca tccccctgag tcttggaagt    1020
   ggatccttgc accggtcatt ctttatatct gtgaaaggat cctccggttt taccgctccc    1080
   agcagaaggt tgtgattacc aaggttgtta tgcacccatc caaagttttg gaattgcaga    1140
   tgaacaagcg tggcttcagc atggaagtgg ggcagtatat ctttgttaat tgcccctcaa    1200
   tctctctcct ggaatggcat ccttttactt tgacctctgc tccagaggaa gatttcttct    1260
   ccattcatat ccgagcagca ggggactgga cagaaaatct cataagggct ttcgaacaac    1320
   aatattcacc aattcccagg attgaagtgg atggtccctt tggcacagcc agtgaggatg    1380
   ttttccagta tgaagtggct gtgctggttg gagcaggaat tggggtcacc ccctttgctt    1440
   ctatcttgaa atccatctgg tacaaattcc agtgtgcaga ccacaacctc aaaacaaaaa    1500
   agatctattt ctactggatc tgcagggaga caggtgcctt ttcctggttc aacaacctgt    1560
   tgacttccct ggaacaggag atggaggaat taggcaaagt gggttttcta aactaccgtc    1620
   tcttcctcac cggatgggac agcaatattg ttggtcatgc agcattaaac tttgacaagg    1680
   ccactgacat cgtgacaggt ctgaaacaga aaacctcctt tgggagacca atgtgggaca    1740
   atgagttttc tacaatagct acctcccacc ccaagtctgt agtgggagtt ttcttatgtg    1800
   gccctcggac tttggcaaag agcctgcgca aatgctgtca ccgatattcc agtctggatc    1860
   ctagaaaggt tcaattctac ttcaacaaag aaaatttttg agttatagga ataaggacgg    1920
   taatctgcat tttgtctctt tgtatcttca gtaattgagt tataggaata aggacggtaa    1980
   tctgcatttt gtctctttgt atcttcagta atttacttgg tctcgtcagg tttgagcagt 2040
   cactttagga taagaatgtg cctctcaagc cttgactccc tggtattctt tttttgattg 2100
   cattcaactt cgttacttga gcttcagcaa cttaagaact tctgaagttc ttaaagttct 2160
   gaagttctta aagcccatgg atcctttctc agaaaaataa ctgtaaatct ttctggacag 2220
   ccatgactgt agcaaggctt gatagcagag gtttggtggt tcagagttat acaactaatc 2280
   ccaggtgatt ttatcaattc cagtgttacc atctcctgag ttttggtttg taatcttttg 2340
   tccctcccac ccccacagaa gatttcctaa gtagggtgac tttttaaata aaaatttatt 2400
   gaataattaa tgataaaaca taataataaa cataaataat aaacaaaatt accgagaacc 2460
   ccatccccat ataacaccaa cagtgtacat gtttactgtc acttttgata tggtcttatc 2520
   cagtgtgaac agcaatttat tatttttgct catcaaaaaa taaaggattt tttttcactt 2580
   gaaaaaaaaa aaaaaaaaaa aaaaaaaaaa aa 2612
<210> 32
   <211> 3345
   <212> DNA
   <213> human
<400> 32
   gaattccgtc tcgaccactg aatggaagaa aaggactttt aaccaccatt ttgtgactta     60
   cagaaaggaa tttgaataaa gaaaactatg atacttcagg cccatcttca ctccctgtgt    120
   cttcttatgc tttatttggc aactggatat ggccaagagg ggaagtttag tggacccctg    180
   aaacccatga cattttctat ttatgaaggc caagaaccga gtcaaattat attccagttt    240
   aaggccaatc ctcctgctgt gacttttgaa ctaactgggg agacagacaa catatttgtg    300
   atagaacggg agggacttct gtattacaac agagccttgg acagggaaac aagatctact    360
   cacaatctcc aggttgcagc cctggacgct aatggaatta tagtggaggg tccagtccct    420
   atcaccatag aagtgaagga catcaacgac aatcgaccca cgtttctcca gtcaaagtac    480
   gaaggctcag taaggcagaa ctctcgccca ggaaagccct tcttgtatgt caatgccaca    540
   gacctggatg atccggccac tcccaatggc cagctttatt accagattgt catccagctt    600
   cccatgatca acaatgtcat gtactttcag atcaacaaca aaacgggagc catctctctt    660
   acccgagagg gatctcagga attgaatcct gctaagaatc cttcctataa tctggtgatc    720
   tcagtgaagg acatgggagg ccagagtgag aattccttca gtgataccac atctgtggat    780
   atcatagtga cagagaatat ttggaaagca ccaaaacctg tggagatggt ggaaaactca    840
   actgatcctc accccatcaa aatcactcag gtgcggtgga atgatcccgg tgcacaatat    900
   tccttagttg acaaagagaa gctgccaaga ttcccatttt caattgacca ggaaggagat    960
   atttacgtga ctcagccctt ggaccgagaa gaaaaggatg catatgtttt ttatgcagtt 1020
   gcaaaggatg agtacggaaa accactttca tatccgctgg aaattcatgt aaaagttaaa 1080
   gatattaatg ataatccacc tacatgtccg tcaccagtaa ccgtatttga ggtccaggag 1140
   aatgaacgac tgggtaacag tatcgggacc cttactgcac atgacaggga tgaagaaaat 1200
   actgccaaca gttttctaaa ctacaggatt gtggagcaaa ctcccaaact tcccatggat 1260
   ggactcttcc taatccaaac ctatgctgga atgttacagt tagctaaaca gtccttgaag 1320
   aagcaagata ctcctcagta caacttaacg atagaggtgt ctgacaaaga tttcaagacc 1380
   ctttgttttg tgcaaatcaa cgttattgat atcaatgatc agatccccat ctttgaaaaa 1440
   tcagattatg gaaacctgac tcttgctgaa gacacaaaca ttgggtccac catcttaacc 1500
   atccaggcca ctgatgctga tgagccattt actgggagtt ctaaaattct gtatcatatc 1560
   ataaagggag acagtgaggg acgcctgggg gttgacacag atccccatac caacaccgga 1620
   tatgtcataa ttaaaaagcc tcttgatttt gaaacagcag ctgtttccaa cattgtgttc 1680
   aaagcagaaa atcctgagcc tctagtgttt ggtgtgaagt acaatgcaag ttcttttgcc 1740
   aagttcacgc ttattgtgac agatgtgaat gaagcacctc aattttccca acacgtattc 1800
   caagcgaaag tcagtgagga tgtagctata ggcactaaag tgggcaatgt gactgccaag 1860
   gatccagaag gtctggacat aagctattca ctgaggggag acacaagagg ttggcttaaa 1920
   attgaccacg tgactggtga gatctttagt gtggctccat tggacagaga agccggaagt 1980
   ccatatcggg tacaagtggt ggccacagaa gtaggggggt cttccttaag ctctgtgtca 2040
   gagttccacc tgatccttat ggatgtgaat gacaaccctc ccaggctagc caaggactac 2100
   acgggcttgt tcttctgcca tcccctcagt gcacctggaa gtctcatttt cgaggctact 2160
   gatgatgatc agcacttatt tcggggtccc cattttacat tttccctcgg cagtggaagc 2220
   ttacaaaacg actgggaagt ttccaaaatc aatggtactc atgcccgact gtctaccagg 2280
   cacacagact ttgaggagag ggcgtatgtc gtcttgatcc gcatcaatga tgggggtcgg 2340
   ccacccttgg aaggcattgt ttctttacca gttacattct gcagttgtgt ggaaggaagt 2400
   tgtttccggc cagcaggtca ccagactggg atacccactg tgggcatggc agttggtata 2460
   ctgctgacca cccttctggt gattggtata attttagcag ttgtgtttat ccgcataaag 2520
   aaggataaag gcaaagataa tgttgaaagt gctcaagcat ctgaagtcaa acctctgaga 2580
   agctgaattt gaaaaggaat gtttgaattt atatagcaag tgctatttca gcaacaacca 2640
   tctcatccta ttacttttca tctaacgtgc attataattt tttaaacaga tattccctct 2700
   tgtcctttaa tatttgctaa atatttcttt tttgaggtgg agtcttgctc tgtcgcccag 2760
   gctggagtac agtggtgtga tcccagctca ctgcaacctc cgcctcctgg gttcacatga 2820
   ttctcctgcc tcagcttcct aagtagctgg gtttacaggc acccaccacc atgcccagct 2880
   aatttttgta tttttaatag agacggggtt tcgccatttg gccaggctgg tcttgaactc 2940
   ctgacgtcaa gtgatctgcc tgccttggtc tcccaataca ggcatgaacc actgcaccca 3000
   cctacttaga tatttcatgt gctatagaca ttagagagat ttttcatttt tccatgacat 3060
   ttttcctctc tgcaaatggc ttagctactt gtgtttttcc cttttggggc aagacagact 3120
   cattaaatat tctgtacatt ttttctttat caaggagata tatcagtgtt gtctcataga 3180
   actgcctgga ttccatttat gttttttctg attccatcct gtgtcccctt catccttgac 3240
   tcctttggta tttcactgaa tttcaaacat ttgtcagaga agaaaaaagt gaggactcag 3300
   gaaaaataaa taaataaaag aacagccttt tgcggccgcg aattc 3345
<210> 33
   <211> 1201
   <212> DNA
   <213> human
<220>
   <221> misc_feature
   <222> (532)..(532)
   <223> w equals a or t
<400> 33
   wttatwahaa atttattttt aacccaatag aaaagcaaat ttggaatcta tttacaagta 60
   ctatatattt acatatatac agttagagtg ggagatttaa agaaaatggg cagagaaaca 120
   caatataaat caaagaatat gccactgtac aaggcattat tatcattatc atggtcctta 180
   atgttactga acctttacta tagtaataaa tacagttcta tatttacaca tcttataaaa 240
   catctcataa atgtattttt tcaaatccaa gthaaaacat ctgatcaaaa taaacatgct 300
   tatataaaaa taaatctacc taacagccat ttggtttgga tgtattgarg ctaatatagg 360
   ataatagagg gtaagrbtta atactttgac ttttcttatt taataacttg cttcttaaaa 420
   tacctaacac agtattaata tggaatargc rgagargtaa tgttcctaac atcaagtggg 480
   ttatccagag agaacacagc taaaaccaag ctaaataaac aggataatac gttactgagt 540
   ctcttgagtc caaagtggtg tcagatattg ggtttgccag agctactaga gatacatgtg 600
   tgagaggttg tatcagtgga cttaatttat gtgatgtgca catttgatca ttaagatgca 660
   catcagtttg aatcaactga taaaacttat tgcaaaaatt ctttactaac ccagaaaaaa 720
   aatcccagat tgcttacttt cttttccagg tatgtycatt gctggcagtg gaattccctt 780
   ctgagctttg ggcmcaagga gttaaaaaca aatcagataa gacatacgtc acctgtscat 840
   gattscctta gtaacaattt aagaattttg gtcagttttt ctttcaaaat acttgtaagc 900
   agttttatcc catgakggtg gaccatctag tgctgataca taaamctggt atctctaaaa 960
   wtgatctcaa tatgagtgag taacaatacy twacattacc ayctaaggga ttgtscttag 1020
   aaggatcttt cysmkkaags aaasgwggat haaaathtca awkktattwt attwatccaw 1080
   ttwaaaychm haaaataaat ttttattwaa ccawatttcy aatcccmaaa ccyttttttt 1140
   tttttaaaaa aattttatat tamcbkktcm tkyyktaaam dttttttaaa atttaaattw 1200
   a 1201
<210> 34
   <211> 2778
   <212> DNA
   <213> human
<400> 34
   ctcattttga tgtctagaat caggggatcc aggatcatca ccaaggtcat tttcccaggt 60
   atggaggggt ctttctgctt ctttcttgtc atgcacagct gctgaggaag gggctgggag 120
   taaagacagt gaaatgggga ggaggagtcc attcaaaccg agaaacaaag tgtttggttt    180
   ttcttacccc tggtgtagaa gctaccaacc ttttccaaga aagagggcct ggcccccttc    240
   tcgggtctgg ctgggtgcct gctgtgcctc tctggcctcc cctccgaagg gcaccattcc 300
   ctcgggtgag tactaccggc ctgcaccgtc ttccagtggg gacagcctga gaagagagtc 360
   tggggcctta cttcagtacc ttccttcact ggcctcaccc tgtgcaaatc atgccacacg 420
   ctgcagcctc cttttcccta tctataaaat aaaaatgacc ctgctctatc tcactgggct 480
   ggcaagaaca cactgttgtt gccttgcaga cagatgtgct gaggctgtag aaagtgcttt 540
   ttatttggtt gggagcttgt gcataaatgc gagaggggct gcacatctga cggactagag 600
   gtgactcatg gctgaaccgg aacaggacat cggggagaag ccagcagcca tgctgaactc 660
   tccacagggc cctgtgaaaa gctcttcacc tcctctgccc tctggatcta gtgaagccta    720
   ttcatccttc agatgtcagc tcaaataatc aaccttcatg gaggcctccc ttgaccccta    780
   acatgctttc aaagtactgt gtatttcaca ttcatcatgc cccgacaact gtgatttccc 840
   atttattaat atctgtctct tctgctggcc tgcaaactcc aggagcacag agacatcttt 900
   gggatttttg aacatgattt ccccagggct tagcccagtg cctggtgcaa agcaggcttt 960
   caacatgttc agtggatatt gtaagaaaga aagaaataca caaaaggcct ggcatatgca 1020
   aagcactcta aatattcact cctttccctt ccctctgggt gagaaaattt ctccttataa 1080
   agacaccctc ctaactgtat ctctgctaga gaactgaaga cataaagcac tctgtgccaa 1140
   aaatatttaa gtaaaaactt gagctaagca cagagattat aaatatttct tccccagatt 1200
   acgcaccatt taaaaatact gtctcagctc cttttcatga tttgggtggt gattaaagaa 1260
   aattactctt caagactgaa agtcattact gcccttttcc tgacttgcct tttcccttga 1320
   gaaggggagg ataagctgca gggcaggaag tggaagtggg gcatccttgt cctttgtctg 1380
   gcagacagcc aactggtcag gtactgctcc ttctcaactc tttcctgatt cccaggtgaa 1440
   tataaacaag aaggcacaaa tccacacttg ccaacaacgg acccaagtga taacaagaaa 1500
   cccagtgaca cctgtctagg tgaagactca gcccctatgt gaccaggttg caaagccaaa 1560
   ctgaccatct gctttccatt tggactttta gttcatactg tatcttctca ggacagttaa 1620
   gttggaatac aatgccactg tcctgaaaga tggtagaatt atcctatttc tggaggagtg 1680
   ggggtggtgg gtaggaatct caagagcgat ttgctcctct gcacaatagc ttctttaagg 1740
   acaccagggc ccccagggct atacatttcc ctgaagcttt ccagataagc aacaaggtat 1800
   gagcacctgc tatgtattgc ccaagggtga tgtgtttaaa tatccattgc atattttaaa 1860
   tccttggctg gcttaaagct gcaagctttc tgtcttcagt ggatataatg ggggcataca 1920
   tcccagagct tgcccaacac tccaagaaaa gaaccctcag ctaatgcaaa gtgtgtatgt 1980
   gcccatgaaa gctccatgtc tacttaacat tcagttttta ggattattta tgctgtaata 2040
   atagatatga aaatctctga caggtatttt gtttccttta caaactgtat ttgaatttat 2100
   gggtgattta gagcttgtgt ttaaagtcag aattcagaac cccaaagaaa atgacttcat 2160
   tgaaattgaa ctgaagagac aagaactgag ttaccaaaac ctactaaacg tgagttgctg 2220
   tgaactgggg attaaaccag aacgagtgga gaagatcaga aagctaccaa acacactgct 2280
   cagaaaggac aaagacattc gaagactgcg ggactttcag gaagtggaac tcattttaat 2340
   gaaaaatgga agctccagat tgacagaata tgtgccatct ctgacagaaa ggccctgcta 2400
   tgatagcaaa gctgcaaaaa tgacttatta aatactccca ggaatggccg cgcatggtgg 2460
   ctcaccccct gtaatcccag cactttggga agccaaggtg ggcggatcac ctgaggtcag 2520
   gagttctaga ccagcctggc caacatatag tgaaacccag tctctactaa aaaaaataca 2580
   aaaattagct aggtgtggtg gcgcacacct gtagtagtcc cagctacatg ggaagctgag 2640
   gcaggagaat cacctgaacc caggaggcag aggttgcagt gagctgagat tgcgccactg 2700
   cactccagcc tggcgacaga gcaagactct gtctctcaaa ataaataaat aaataaataa 2760
   ataaataaat aaataatc 2778
<210> 35
   <211> 2973
   <212> DNA
   <213> human
<400> 35
   attctggggc tcgggggatc ccggacaccc tctcagctcc tgcccggggg cccatgtagt      60
   cccttctgcc ctgtgcctcg gtgcctgtga cctgagcccc ttggttgacc ctgcactcgt     120
   ccaacttggg ccaaacgact gcccctcctt ctggcagtgg gctggaccag ccggccagcg     180
   ggagccccct tggcagaagc cggtcgtaaa ggatcataaa ctggcggcgt ctggctgggg    240
   cgaaggtcgc tgaggtagga actgcgccag tcctagacgc cagacccgct cagaccctcc     300
   tgccaggtga cagccgccaa gatggggtct tgggccctgc tgtggcctcc cctgctgttc     360
   accgggctgc tcgtccgacc cccggggacc atggcccagg cccagtactg ctctgtgaac     420
   aaggacatct ttgaagtaga ggagaacaca aatgtcaccg agccgctggt ggacatccac     480
   gtcccggagg gccaggaggt gaccctcgga gccttgtcca ccccctttgc atttcggatc     540
   cagggaaacc agctgtttct caacgtgact cctgattacg aggagaagtc actgcttgag     600
   gctcagctgc tgtgtcagag cggaggcaca ttggtgaccc agctaagggt gttcgtgtca     660
   gtgctggacg tcaatgacaa tgcccccgaa ttccccttta agaccaagga gataagggtg     720
   gaggaggaca cgaaagtgaa ctccaccgtc atccccgaga cgcaactgca ggctgaggac     780
   cgcgacaagg acgacattct gttctacacc ctccaggaaa tgacagcagg tgccagtgac     840
   tacttctccc tggtgagtgt aaaccgtccc gccctgaggc tggaccggcc cctggacttc     900
   tacgagcggc cgaacatgac cttctggctg ctggtgcggg acactccggg ggagaatgtg     960
   gaacccagcc acactgccac cgccacacta gtgctgaacg tggtgcccgc cgacctgcgg    1020
   cccccgtggt tcctgccctg caccttctca gatggctacg tctgcattca agctcagtac    1080
   cacggggctg tccccacggg gcacatactg ccatctcccc tcgtcctgcg tcccggaccc    1140
   atctacgctg aggacggaga ccgcggcatc aaccagccca tcatctacag catctttagg    1200
   ggaaacgtga atggtacatt catcatccac ccagactcgg gcaacctcac cgtggccagg    1260
   agtgtcccca gccccatgac cttccttctg ctggtgaagg gccaacaggc cgaccttgcc    1320
   cgctactcag tgacccaggt caccgtggag gctgtggctg cggccgggag cccgccccgc    1380
   ttcccccaga gcctgtatcg tggcaccgtg gcgcgtggcg ctggagcggg cgttgtggtc    1440
   aaggatgcag ctgccccttc tcagcctctg aggatccagg ctcaggaccc ggagttctcg    1500
   gacctcaact cggccatcac atatcgaatt accaaccact cacacttccg gatggaggga    1560
   gaggttgtgc tgaccaccac cacactggca caggcgggag ccttctacgc agaggttgag    1620
   gcccacaaca cggtgacctc tggcaccgca accacagtca ttgagataca agtttccgaa    1680
   caggagcccc cctccacaga tgtcccccca tccccagagg ctggaggaac aactgggccc    1740
   tggaccagca ccacttccga ggtccccaga ccccctgagc cctcccaggg accctccacg    1800
   accagctctg ggggaggcac aggccctcat ccaccctctg gcacaactct gaggccacca     1860
   acctcgtcca cacccggggg gtccccgggt gcagaaaaca gcacctccca ccaaccagcc   1920
   actcccggtg gggacacagc acagacccca aagccaggaa cctctcagcc gatgcccccc   1980
   ggtgtgggaa ccagcacctc ccaccaacca gccacaccca gtgggggcac agtacagacc   2040
   ccagagccag gaacctctca gccgatgccc cccagtatgg gaaccagcac ctcccaccaa   2100
   ccagccacac ccggtggggg cacagcacag accccagagg caggaacctc tcagccgatg   2160
   ccccccggta tgggaaccag cacctcccac caaccaacca cacccggtgg gggcacagca   2220
   cagaccccag agccaggaac ctctcagccg atgcccctca gcaagagcac cccatcttca   2280
   ggtggcggcc cctcggagga caagcgcttc tcggtggtgg atatggcggc cctgggcggg   2340
   gtgctgggtg cgctgctgct gctggctctc cttggcctcg ccgtccttgt ccacaagcac   2400
   tatggccccc ggctcaagtg ctgctctggc aaagctccgg agccccagcc ccaaggcttt   2460
   gacaaccagg cgttcctccc tgaccacaag gccaactggg cgcccgtccc cagccccacg   2520
   cacgacccca agcccgcgga ggcaccgatg cccgcagagc ccgcaccccc cggccctgcc   2580
   tccccaggcg gtgcccctga gccccccgca gcggcccgag ctggcggaag ccccacggcg   2640
   gtgaggtcca tcctgaccaa ggagcggcgg ccagagggcg ggtacaaggc tgtctggttt   2700
   ggcgaggaca tcgggacgga ggcagacgtg gtcgttctca acgcgcccac cctggacgtg   2760
   gatggcgcca gtgactccgg cagcggcgat gagggcgagg gcgcggggag gggtgggggt   2820
   ccctacgatg cgcccggtgg tgatgactcc tacatctaag tggcccctcc accctctccc   2880
   ccagccgcac gggcactgga ggtctcgctc ccccagcctc cgacccgagg cagaataaag   2940
   caaggctccc gaaacccaaa aaaaaaaaaa aaa                                2973
<210> 36
   <211> 1930
   <212> DNA
   <213> human
<400> 36
   ggagagagag aggacagaga gcaagtcact cccggctgcc tttttcacct ctgacagagc     60
   ccagacacca tgaacgcaag tgaattccga aggagaggga aggagatggt ggattacgtg    120
   gccaactaca tggaaggcat tgagggacgc caggtctacc ctgacgtgga gcccgggtac    180
   ctgcggccgc tgatccctgc cgctgcccct caggagccag acacgtttga ggacatcatc    240
   aacgacgttg agaagataat catgcctggg gtgacgcact ggcacagccc ctacttcttc    300
   gcctacttcc ccactgccag ctcgtacccg gccatgcttg cggacatgct gtgcggggcc    360
   attggctgca tcggcttctc ctgggcggca agcccagcat gcacagagct ggagactgtg    420
   atgatggact ggctcgggaa gatgctggaa ctaccaaagg catttttgaa tgagaaagct    480
   ggagaagggg gaggagtgat ccagggaagt gccagtgaag ccaccctggt ggccctgctg    540
   gccgctcgga ccaaagtgat ccatcggctg caggcagcgt ccccagagct cacacaggcc    600
   gctatcatgg agaagctggt ggcttactca tccgatcagg cacactcctc agtggaaaga    660
   gctgggttaa ttggtggagt gaaattaaaa gccatcccct cagatggcaa cttcgccatg    720
   cgtgcgtctg ccctgcagga agccctggag agagacaaag cggctggcct gattcctttc    780
   tttatggttg ccaccctggg gaccacaaca tgctgctcct ttgacaatct cttagaagtc    840
   ggtcctatct gcaacaagga agacatatgg ctgcacgttg atgcagccta cgcaggcagt    900
   gcattcatct gccctgagtt ccggcacctt ctgaatggag tggagtttgc agattcattc    960
   aactttaatc cccacaaatg gctattggtg aattttgact gttctgccat gtgggtgaaa   1020
   aagagaacag acttaacggg agcctttaga ctggacccca cttacctgaa gcacagccat   1080
   caggattcag ggcttatcac tgactaccgg cattggcaga taccactggg cagaagattt   1140
   cgctctttga aaatgtggtt tgtatttagg atgtatggag tcaaaggact gcaggcttat   1200
   atccgcaagc atgtccagct gtcccatgag tttgagtcac tggtgcgcca ggatccccgc   1260
   tttgaaatct gtgtggaagt cattctgggg cttgtctgct ttcggctaaa gggttccaac   1320
   aaagtgaatg aagctcttct gcaaagaata aacagtgcca aaaaaatcca cttggttcca   1380
   tgtcacctca gggacaagtt tgtcctgcgc tttgccatct gttctcgcac ggtggaatct   1440
   gcccatgtgc agcgggcctg ggaacacatc aaagagctgg cggccgacgt gctgcgagca   1500
   gagagggagt aggagtgaag ccagctgcag gaatcaaaaa ttgaagagag atatatctga   1560
   aaactggaat aagaagcaaa taaatatcat cctgccttca tggaactcag ctgtctgtgg   1620
   cttcccatgt ctttctccaa agccatccag agggttgtga ttttgtctgc ttagtatctc   1680
   atcaacaaag aaatattatt tgctaattaa aaagttaatc ttcatggcca tagcttttat   1740
   tcattagctg tgatttttgt tgattaaaac attatagatt ttcatgttct tgcagtcatc   1800
   agaagtggta ggaaagcctc actgatatat tttccagggc aatcaatgtt cacgcaactt   1860
   gaaattatat ctgtggtctt caaattgtct tttgtcatgt ggctaaatgc ctaataaaca   1920
   attcaagtga                                                          1930
<210> 37
   <211> 1745
   <212> DNA
   <213> human
<400> 37
   gcgcccctgg cagccttcaa cgtcggtccc caggcagcat ggtgaggtct gctcccggac     60
   cctcgccacc atgtacgtga gctacctcct ggacaaggac gtgagcatgt accctagctc    120
   cgtgcgccac tctggcggcc tcaacctggc gccgcagaac ttcgtcagcc ccccgcagta 180
   cccggactac ggcggttacc acgtggcggc cgcagctgca gcgcagaact tggacagcgc 240
   gcagtccccg gggccatcct ggccggcagc gtatggcgcc ccactccggg aggactggaa 300
   tggctacgcg cccggaggcg cggccgccgc caacgccgtg gctcacgcgc tcaacggtgg 360
   ctccccggcc gcagccatgg gctacagcag ccccgcagac taccatccgc accaccaccc 420
   gcatcaccac ccgcaccacc cggccgccgc gccttcctgc gcttctgggc tgctgcaaac 480
   gctcaacccc ggccctcctg ggcccgccgc caccgctgcc gccgagcagc tgtctcccgg 540
   cggccagcgg cggaacctgt gcgagtggat gcggaagccg gcgcagcagt ccctcggcag 600
   ccaagtgaaa accaggacga aagacaaata tcgagtggtg tacacggacc accagcggct 660
   ggagctggag aaggagtttc actacagtcg ctacatcacc atccggagga aagccgagct 720
   agccgccacg ctggggctct ctgagaggca ggttaaaatc tggtttcaga accgcagagc 780
   aaaggagagg aaaatcaaca agaagaagtt gcagcagcaa cagcagcagc agccaccaca 840
   gccgcctccg ccgccaccac agcctcccca gcctcagcca ggtcctctga gaagtgtccc 900
   agagcccttg agtccggtgt cttccctgca agcctcagtg tctggctctg tccctggggt 960
   tctggggcca actggggggg tgctaaaccc caccgtcacc cagtgaccca ccggggtctg 1020
   cagcggcaga gcaattccag gctgagccat gaggagcgtg gactctgcta gactcctcag 1080
   gagagacccc tcccctccca cccacagcca tagacctaca gacctggctc tcagaggaaa 1140
   aatgggagcc aggagtaaga caagtgggat ttggggcctc aagaaatata ctctcccaga 1200
   tttttacttt ttccatctgg ctttttctgc cactgaggag acagaaagcc tccgctgggc 1260
   ttcattccgg actggcagaa gcattgcctg gactgaccac accaaccagc ttcatctatc 1320
   cgactcttct cttcctagat ctgcaggctg cacctctggc tagagccgag ggagagaggg 1380
   gactcaaggg aaaggcaagc ttgaggccaa gatggctgct gcctgctcat ggccctcgga 1440
   ggtccagctg ggcctcctgc ctccgggcag caaggtttac actgcggaac gcaaaggcag 1500
   ctaagataga aagctggact gaccaaagac tgcagaaccc ccaggtggcc ctgcgtcttt 1560
   tttctcttcc ctttcccaga ccaggaaagg cttggctggt gtatgcacag ggtgtggtat 1620
   gagggggtgg ttattggact ccaggcctga ccagggggcc cgaacaggac ttgttagaga 1680
   gcctgtcacc agagcttctc tgggctgaat gtatgtcagt gctataaatg ccagagccaa 1740
   cctgg 1745
<210> 38
   <211> 1881
   <212> DNA
   <213> human
<400> 38
   ggacctctcc agaatccgga ttgctgaatc ttccctgttg cctagaaggg ctccaaacca 60
   cctcttgaca atgggaaact gggtggttaa ccactggttt tcagttttgt ttctggttgt 120
   ttggttaggg ctgaatgttt tcctgtttgt ggatgccttc ctgaaatatg agaaggccga 180
   caaatactac tacacaagaa aaatccttgg gtcaacattg gcctgtgccc gagcgtctgc 240
   tctctgcttg aattttaaca gcacgctgat cctgcttcct gtgtgtcgca atctgctgtc 300
   cttcctgagg ggcacctgct cattttgcag ccgcacactg agaaagcaat tggatcacaa 360
   cctcaccttc cacaagctgg tggcctatat gatctgccta catacagcta ttcacatcat 420
   tgcacacctg tttaactttg actgctatag cagaagccga caggccacag atggctccct 480
   tgcctccatt ctctccagcc tatctcatga tgagaaaaag gggggttctt ggctaaatcc 540
   catccagtcc cgaaacacga cagtggagta tgtgacattc accagcattg ctggtctcac 600
   tggagtgatc atgacaatag ccttgattct catggtaact tcagctactg agttcatccg 660
   gaggagttat tttgaagtct tctggtatac tcaccacctt tttatcttct atatccttgg 720
   cttagggatt cacggcattg gtggaattgt ccggggtcaa acagaggaga gcatgaatga 780
   gagtcatcct cgcaagtgtg cagagtcttt tgagatgtgg gatgatcgtg actcccactg 840
   taggcgccct aagtttgaag ggcatccccc tgagtcttgg aagtggatcc ttgcaccggt 900
   cattctttat atctgtgaaa ggatcctccg gttttaccgc tcccagcaga aggttgtgat 960
   taccaaggtt gttatgcacc catccaaagt tttggaattg cagatgaaca agcgtggctt 1020
   cagcatggaa gtggggcagt atatctttgt taattgcccc tcaatctctc tcctggaatg 1080
   gcatcctttt actttgacct ctgctccaga ggaagatttc ttctccattc atatccgagc 1140
   agcaggggac tggacagaaa atctcataag ggctttcgaa caacaatatt caccaattcc 1200
   caggattgaa gtggatggtc cctttggcac agccagtgag gatgttttcc agtatgaagt   1260
   ggctgtgctg gttggagcag gaattggggt cacccccttt gcttctatct tgaaatccat   1320
   ctggtacaaa ttccagtgtg cagaccacaa cctcaaaaca aaaaagatct atttctactg 1380
   gatctgcagg gagacaggtg ccttttcctg gttcaacaac ctgttgactt ccctggaaca 1440
   ggagatggag gaattaggca aagtgggttt tctaaactac cgtctcttcc tcaccggatg 1500
   ggacagcaat attgttggtc atgcagcatt aaactttgac aaggccactg acatcgtgac 1560
   aggtctgaaa cagaaaacct cctttgggag accaatgtgg gacaatgagt tttctacaat 1620
   agctacctcc caccccaagt ctgtagtggg agttttctta tgtggccctc ggactttggc 1680
   aaagagcctg cgcaaatgct gtcaccgata ttccagtctg gatcctagaa aggttcaatt 1740
   ctacttcaac aaagaaaatt tttgagttat aggaataagg acggtaatct gcattttgtc 1800
   tctttgtatc ttcagtaatt tacttggtct cgtcaggttt gagcagtcac tttaggataa 1860
   gaatgtgcct ctcaagcctt g 1881
<210> 39
   <211> 3745
   <212> DNA
   <213> human
<400> 39
   cgcaaagcaa gtgggcacaa ggagtatggt tctaacgtga ttggggtcat gaagacgttg     60
   ctgttggact tggctttgtg gtcactgctc ttccagcccg ggtggctgtc ctttagttcc    120
   caggtgagtc agaactgcca caatggcagc tatgaaatca gcgtcctgat gatgggcaac    180
   tcagcctttg cagagcccct gaaaaacttg gaagatgcgg tgaatgaggg gctggaaata    240
   gtgagaggac gtctgcaaaa tgctggccta aatgtgactg tgaacgctac tttcatgtat    300
   tcggatggtc tgattcataa ctcaggcgac tgccggagta gcacctgtga aggcctcgac    360
   ctactcagga aaatttcaaa tgcacaacgg atgggctgtg tcctcatagg gccctcatgt    420
   acatactcca ccttccagat gtaccttgac acagaattga gctaccccat gatctcagct    480
   ggaagttttg gattgtcatg tgactataaa gaaaccttaa ccaggctgat gtctccagct    540
   agaaagttga tgtacttctt ggttaacttt tggaaaacca acgatctgcc cttcaaaact    600
   tattcctgga gcacttcgta tgtttacaag aatggtacag aaactgagga ctgtttctgg    660
   taccttaatg ctctggaggc tagcgtttcc tatttctccc acgaactcgg ctttaaggtg    720
   gtgttaagac aagataagga gtttcaggat atcttaatgg accacaacag gaaaagcaat    780
   gtgattatta tgtgtggtgg tccagagttc ctctacaagc tgaagggtga ccgagcagtg    840
   gctgaagaca ttgtcattat tctagtggat cttttcaatg accagtactt ggaggacaat    900
   gtcacagccc ctgactatat gaaaaatgtc cttgttctga cgctgtctcc tgggaattcc    960
   cttctaaata gctctttctc caggaatcta tcaccaacaa aacgagactt tgctcttgcc   1020
   tatttgaatg gaatcctgct ctttggacat atgctgaaga tatttcttga aaatggagaa   1080
   aatattacca cccccaaatt tgctcatgct ttcaggaatc tcacttttga agggtatgac   1140
   ggtccagtga ccttggatga ctggggggat gttgacagta ccatggtgct tctgtatacc    1200
   tctgtggaca ccaagaaata caaggttctt ttgacctatg atacccacgt aaataagacc   1260
   tatcctgtgg atatgagccc cacattcact tggaagaact ctaaacttcc taatgatatt   1320
   acaggccggg gccctcagat cctgatgatt gcagtcttca ccctcactgg agctgtggtg   1380
   ctgctcctgc tcgtcgctct cctgatgctc agaaaatata gaaaagatta tgaacttcgt   1440
   cagaaaaaat ggtcccacat tcctcctgaa aatatctttc ctctggagac caatgagacc 1500
   aatcatgtta gcctcaagat cgatgatgac aaaagacgag atacaatcca gagactacga 1560
   cagtgcaaat acgacaaaaa gcgagtgatt ctcaaagatc tcaagcacaa tgatggtaat 1620
   ttcactgaaa aacagaagat agaattgaac aagttgcttc agattgacta ttacaacctg 1680
   accaagttct acggcacagt gaaacttgat accatgatct tcggggtgat agaatactgt 1740
   gagagaggat ccctccggga agttttaaat gacacaattt cctaccctga tggcacattc 1800
   atggattggg agtttaagat ctctgtcttg tatgacattg ctaagggaat gtcatatctg 1860
   cactccagta agacagaagt ccatggtcgt ctgaaatcta ccaactgcgt agtggacagt 1920
   agaatggtgg tgaagatcac tgattttggc tgcaattcca ttttacctcc aaaaaaggac 1980
   ctgtggacag ctccagagca cctccgccaa gccaacatct ctcagaaagg agatgtgtac 2040
   agctatggga tcatcgcaca ggagatcatt ctgcggaaag aaaccttcta cactttgagc 2100
   tgtcgggacc ggaatgagaa gattttcaga gtggaaaatt ccaatggaat gaaacccttc 2160
   cgcccagatt tattcttgga aacagcagag gaaaaagagc tagaagtgta cctacttgta 2220
   aaaaactgtt gggaggaaga tccagaaaag agaccagatt tcaaaaaaat tgagactaca 2280
   cttgccaaga tatttggact ttttcatgac caaaaaaatg aaagctatat ggataccttg 2340
   atccgacgtc tacagctata ttctcgaaac ctggaacatc tggtagagga aaggacacag 2400
   ctgtacaagg cagagaggga cagggctgac agacttaact ttatgttgct tccaaggcta 2460
   gtggtaaagt ctctgaagga gaaaggcttt gtggagccgg aactatatga ggaagttaca 2520
   atctacttca gtgacattgt aggtttcact actatctgca aatacagcac ccccatggaa 2580
   gtggtggaca tgcttaatga catctataag agttttgacc acattgttga tcatcatgat 2640
   gtctacaagg tggaaaccat cggtgatgcg tacatggtgg ctagtggttt gcctaagaga 2700
   aatggcaatc ggcatgcaat agacattgcc aagatggcct tggaaatcct cagcttcatg 2760
   gggacctttg agctggagca tcttcctggc ctcccaatat ggattcgcat tggagttcac 2820
   tctggtccct gtgctgctgg agttgtggga atcaagatgc ctcgttattg tctatttgga 2880
   gatacggtca acacagcctc taggatggaa tccactggcc tccctttgag aattcacgtg 2940
   agtggctcca ccatagccat cctgaagaga actgagtgcc agttccttta tgaagtgaga 3000
   ggagaaacat acttaaaggg aagaggaaat gagactacct actggctgac tgggatgaag 3060
   gaccagaaat tcaacctgcc aacccctcct actgtggaga atcaacagcg tttgcaagca 3120
   gaattttcag acatgattgc caactcttta cagaaaagac aggcagcagg gataagaagc 3180
   caaaaaccca gacgggtagc cagctataaa aaaggcactc tggaatactt gcagctgaat 3240
   accacagaca aggagagcac ctatttttaa acctaaatga ggtataagga ctcacacaaa 3300
   ttaaaataca gctgcactga ggcagcgacc tcaagtgtcc tgaaagctta cattttcctg 3360
   agacctcaat gaagcagaaa tgtacttagg cttggctgcc ctgtctggaa catggacttt 3420
   cttgcatgaa tcagatgtgt gttctcagtg aaataactac cttccactct ggaaccttat 3480
   tccagcagtt gttccaggga gcttctacct ggaaaagaaa agaaatgaat agactatcta 3540
   gaacttgaga agattttatt cttatttcat ttattttttg tttgtttatt tttatcgttt 3600
   ttgtttactg gctttccttc tgtattcata agatttttta aattgtcata attatatttt 3660
   aaatacccat cttcattaaa gtatatttaa ctcataattt ttgcagaaaa tatgctatat 3720
   attaggcaag aataaaagct aaagg 3745
<210> 40
   <211> 2793
   <212> DNA
   <213> human
<400> 40
   ctaccccttt gtgagcagtc taggactttg tacacctgtt aagtagggag aaggcagggg 60
   aggtggctgg tttaagggga acttgaggga agtagggaag actcctcttg ggacctttgg 120
   agtaggtgac acatgagccc agccccagct cacctgccaa tccagctgag gagctcacct 180
   gccaatccag ctgaggctgg gcagaggtgg gtgagaagag ggaaaattgc agggacctcc 240
   agttgggcca ggccagaagc tgctgtagct ttaaccagac agctcagacc tgtctggagg 300
   ctgccagtga caggttaggt ttagggcaga gaagaagcaa gaccatggtg gggaagatgt 360
   ggcctgtgtt gtggacactc tgtgcagtca gggtgaccgt cgatgccatc tctgtggaaa 420
   ctccgcagga cgttcttcgg gcttcgcagg gaaagagtgt caccctgccc tgcacctacc 480
   acacttccac ctccagtcga gagggactta ttcaatggga taagctcctc ctcactcata 540
   cggaaagggt ggtcatctgg ccgttttcaa acaaaaacta catccatggt gagctttata 600
   agaatcgcgt cagcatatcc aacaatgctg agcagtccga tgcctccatc accattgatc 660
   agctgaccat ggctgacaac ggcacctacg agtgttctgt ctcgctgatg tcagacctgg 720
   agggcaacac caagtcacgt gtccgcctgt tggtcctcgt gccaccctcc aaaccagaat 780
   gcggcatcga gggagagacc ataattggga acaacatcca gctgacctgc caatcaaagg 840
   agggctcacc aacccctcag tacagctgga agaggtacaa catcctgaat caggagcagc 900
   ccctggccca gccagcctca ggtcagcctg tctccctgaa gaatatctcc acagacacat 960
   cgggttacta catctgtacc tccagcaatg aggaggggac gcagttctgc aacatcacgg 1020
   tggccgtcag atctccctcc atgaacgtgg ccctgtatgt gggcatcgcg gtgggcgtgg 1080
   ttgcagccct cattatcatt ggcatcatca tctactgctg ctgctgccga gggaaggacg 1140
   acaacactga agacaaggag gatgcaaggc cgaaccggga agcctatgag gagccaccag 1200
   agcagctaag agaactttcc agagagaggg aggaggagga tgactacagg caagaagagc 1260
   agaggagcac tgggcgtgaa tccccggacc acctcgacca gtgacaggcc agcagcagag 1320
   ggcggcggag gaagggttag gggttcattc tcccgcttcc tggcctccct tctcctttct 1380
   aagccctgtt ctcctgtccc tccatcccag acattgatgg ggacatttct tccccagtgt 1440
   cagctgtggg gaacatggct ggcctggtaa gggggtccct gtgctgatcc tgctgacctc 1500
   actgtcctgt gaagtaaccc ctcctggctg tgacacctgg tgcgggcctg gccctcactc 1560
   aagaccaggc tgcagcctcc acttccctcg tagttggcag gagctcctgg aagcacagcg 1620
   ctgagcatgg ggcgctccca ctcagaactc tccagggagg cgatgccagc cttggggggt 1680
   gggggctgtc ctgctcacct gtgtgcccag cacctggagg ggcaccaggt ggagggtttg 1740
   cactccacac atctttcttg aatgaatgaa agaataagtg agtatgcttg ggccctgcat 1800
   tggcctggcc tccagctccc actccctttc caacctcact tcccgtagct gccagtatgt 1860
   tccaaaccct cctgggaagg ccacctccca ctcctgctgc acaggccctg gggagctttt 1920
   gcccacacac tttccatctc tgcctgtcaa tatcgtacct gtccctccag gcccatctca 1980
   aatcacaagg atttctctaa ccctatccta attgtccaca tacgtggaaa caatcctgtt 2040
   actctgtccc acgtccaatc atgggccaca aggcacagtc ttctgagcga gtgctctcac 2100
   tgtattagag cgccagctcc ttggggcagg gcctgggcct catggctttt gctttccctg 2160
   aagccctagt agctggcgcc catcctagtg ggcacttaag cttaattggg gaaactgctt 2220
   tgattggttg tgccttccct tctctggtct ccttgagatg atcgtagaca cagggatgat 2280
   tcccacccaa acccacgtat tcattcagtg agttaaacac gaattgattt aaagtgaaca 2340
   cacacaaggg agcttgcttg cagatggtct gagttcttgt gtcctggtaa ttcctctcca 2400
   ggccagaata attggcatgt ctcctcaacc cacatggggt tcctggttgt tcctgcatcc 2460
   cgatacctca gccctggccc tgcccagccc atttgggctc tggttttctg gtggggctgt 2520
   cctgctgccc tcccacagcc tccttctgtt tgtcgagcat ttcttctact cttgagagct 2580
   caggcagcgt tagggctgct taggtctcat ggaccagtgg ctggtctcac ccaactgcag 2640
   tttactattg ctatcttttc tggatgatca gaaaaataat tccataaatc tattgtctac 2700
   ttgcgatttt ttaaaaaatg tatattttta tatatattgt taaatccttt gcttcattcc 2760
   aaatgctttc agtaataata aaattgtggg tgg 2793
<210> 41
   <211> 1734
   <212> DNA.
   <213> human
<400> 41
   ggacctctcc agaatccgga ttgctgaatc ttccctgttg cctagaaggg ctccaaacca      60
   cctcttgaca atgggaaact gggtggttaa ccactggttt tcagttttgt ttctggttgt     120
   ttggttaggg ctgaatgttt tcctgtttgt ggatgccttc ctgaaatatg agaaggccga     180
   caaatactac tacacaagaa aaatccttgg gtcaacattg gcctgtgccc gagcgtctgc     240
   tctctgcttg aattttaaca gcacgctgat cctgcttcct gtgtgtcgca atctgctgtc     300
   cttcctgagg ggcacctgct cattttgcag ccgcacactg agaaagcaat tggatcacaa     360
   cctcaccttc cacaagctgg tggcctatat gatctgccta catacagcta ttcacatcat     420
   tgcacacctg tttaactttg actgctatag cagaagccga caggccacag atggctccct     480
   tgcctccatt ctctccagcc tatctcatga tgagaaaaag gggggttctt ggctaaatcc     540
   catccagtcc cgaaacacga cagtggagta tgtgacattc accagcattg ctggtctcac     600
   tggagtgatc atgacaatag ccttgattct catggtaact tcagctactg agttcatccg     660
   gaggagttat tttgaagtct tctggtatac tcaccacctt tttatcttct atatccttgg     720
   cttagggatt cacggcattg gtggaattgt ccggggtcaa acagaggaga gcatgaatga     780
   gagtcatcct cgcaagtgtg cagagtcttt tgagatgtgg gatgatcgtg actcccactg     840
   taggcgccct aagtttgaag ggcatccccc tgagtcttgg aagtggatcc ttgcaccggt     900
   cattctttat atctgtgaaa ggatcctccg gttttaccgc tcccagcaga aggttgtgat     960
   taccaaggtt gttatgcacc catccaaagt tttggaattg cagatgaaca agcgtggctt    1020
   cagcatggaa gtggggcagt atatctttgt taattgcccc tcaatctctc tcctggaatg    1080
   gcatcctttt actttgacct ctgctccaga ggaagatttc ttctccattc atatccgagc    1140
   agcaggggac tggacagaaa atctcataag ggctttcgaa caacaatatt caccaattcc    1200
   caggattgaa gtggatggtc cctttggcac agccagtgag gatgttttcc agtatgaagt    1260
   ggctgtgctg gttggagcag gaattggggt cacccccttt gcttctatct tgaaatccat    1320
   ctggtacaaa ttccagtgtg cagaccacaa cctcaaaaca aaaaaggttg gtcatgcagc    1380
   attaaacttt gacaaggcca ctgacatcgt gacaggtctg aaacagaaaa cctcctttgg    1440
   gagaccaatg tgggacaatg agttttctac aatagctacc tcccacccca agtctgtagt    1500
   gggagttttc ttatgtggcc ctcggacttt ggcaaagagc ctgcgcaaat gctgtcaccg    1560
   atattccagt ctggatccta gaaaggttca attctacttc aacaaagaaa atttttgagt    1620
   tataggaata aggacggtaa tctgcatttt gtctctttgt atcttcagta atttacttgg    1680
   tctcgtcagg tttgagcagt cactttagga taagaatgtg cctctcaagc cttg          1734
<210> 42
   <211> 3941
   <212> DNA
   <213> human
<400> 42
   accatctact ccacagtcag ctcatccaca actgccatca cctcaccttt cactaccgca     60
   gagactgggg tgacttccac accttcatcc ccatcttctc tgagtacaga catcccgacc    120
   acatccctaa gaactctcac cccattatct ttgagcacca gcacttcatt gactacaacc    180
   acagaccttc cctctatacc cactgatatc agtagcttac caaccccaat acacatcatt    240
   tcatcttctc cctccatcca aagtacagaa acctcatccc ttgtgggcac cacctctccc    300
   accatgtcca ctgtgagagc gaccctcaga agtactgaga acaccccaat cagttccttt    360
   agcacaagta ttgttgttac acctgaaacc ccaacaacac aggcccctcc tgtactgatg    420
   tctgccactg ggacccaaac atcccctgta cctactactg tcacctttgg aagtatggat    480
   tcctctacgt ccactcttca tactcttact ccatcaacag ccttgagcaa gatcatgtca    540
   acatcacagt ttcctattcc tagcacacat tcctccaccc ttcaaacaac tccttcaatc    600
   ccctctttgc aaacttcact cacatctaca agtgagttca ctacagaatc tttcactagg    660
   ggaagtacgt ctacaaatgc aatcttgact tcttttagta ccatcatctg gtcctcaaca    720
   cccactatta tcatgtcctc ttctccatct tctgccagca taactccagt gttcgctact    780
   accattcatt ctgttccttc gtcaccatac attttcagta cagaaaatgt gggctccgct    840
   tctatcacag cctttcctag tctctcttcc tcttcaacta ccagcacttc tccaaccagc    900
   tcctctctga ccacagctct cactgaaata accccctttt cttatatttc ccttccctcc    960
   accacaccct gtccaggaac tataacaatt accatagtcc ctgcctcccc cactgatcca   1020
   tgtgttgaaa tggatcccag cactgaagct acttctcctc ccaccactcc attaacagtc   1080
   tttcccttta ctactgaaat ggtcacctgt cctagctcca tcagtatgca aactactctt   1140
   gctacacata tggacacttc ttccatgacg ccagaaagtg agtccagcat catacctaat   1200
   gcttccagtt ccactggcac tgggactgta cccacaaaca cagttttcac aagtactcga   1260
   ctgcccacca gtgagacctg gctgagcaac aactctgtga tccccacacc tcttcctggc   1320
   gtctctacca tcccgctcac catgaaacca agcagtagcc tcccgaccat cctgaggact   1380
   tcaagcaagt caacacaccc atccccaccc accgccagga cttcagagac atcagtggcc   1440
   actacccaga ctcctaccac ccttacaacg cgcaggacaa ctcccatcac ttcttggatg   1500
   accacacagt ccacgttgac caccactgca ggcacctgtg acaatggtgg cacctgggaa   1560
   cagggccagt gtgcttgcct tccggggttt tctggggacc gctgtcagct ccagaccaga   1620
   tgccagaacg ggggccagtg ggatggcctc aagtgccagt gccccagcac cttctatggt   1680
   tccagttgtg agtttgctgt ggaacaggtg gatctagatg tagtggagac cgaggtgggc   1740
   atggaagtgt ctgtggatca gcagttctcg ccggacctca atgacaacac ttcccaggcc   1800
   tacagggatt tcaacaagac cttctggaat cagatgcaga agatttttgc agacatgcag   1860
   ggcttcacct tcaagggtgt ggagatcctg tccctgagga atggcagcat cgtggtggac   1920
   tacctggtcc tgctggagat gcccttcagc ccccagctgg agagcgagta tgagcaggtg   1980
   aagaccacgc tgaaggaggg gctccagaac gccagccagg atgcgaacag ctgccaggac   2040
   tcccagaccc tgtgttttaa gcctgactcc atcaaggtga acaacaacag caagacagag   2100
   ctgaccccgg aagccatctg ccgccgcgcc gctcccacgg gctatgaaga gttctacttc   2160
   cctctggtgg aggccacccg gctccgctgt gtcaccaaat gcacgtcggg cgtggacaac   2220
   gccatcgact gtcaccaggg ccagtgcgtt ctagagacga gcggtcccgc gtgtcgctgc   2280
   tactccaccg acacgcactg gttctctggc ccgcgctgcg aggtggccgt ccactggagg   2340
   gcgctggtcg ggggcctgac ggccggcgcc gcgctgctgg tgctgctgct gctggcgctg   2400
   ggcgtccggg cggtgcgctc cggatggtgg ggcggccagc gccgaggccg gtcctgggac   2460
   caggacagga aatggttcga gacctgggat gaggaagtcg tgggcacttt ttcaaactgg   2520
   ggtttcgagg acgacggaac agacaaggat acaaatttcc atgtggcctt ggagaacgtg   2580
   gacaccacta tgaaggtgca catcaagaga cccgagatga cctcgtcctc agtgtgagcc   2640
   ctgcggggcc ccttcaccac cccctccgcc ctgccccgga cacaagggtc tgcattgcgt   2700
   ccatttcaag aggtggcccc aggacgcggg cagcccaggc tcctgctgtt cttgggcaag   2760
   atgagactgt tcccccaaat cccatccttc tccttccaac ttggctgaaa cccacctgga   2820
   gacgcagttc acgtccaggc tcttccactg tggaatcttg ggcaagtcag taacgagcct   2880
   cagtttcctc acctgcaaaa cgggtacagc attcctgtat gatagctcac gccgttgttg   2940
   tgaaaaccac atagacttgg tcaattctcg gtcctactct gccctcccgt ctcagccctc   3000
   gtgttgccat tgcctctctc ggatcctcca atcctcacgt ccttcacctg gtctctggcc   3060
   ctggttctta ttttctctca attccctact gcctgtttct tactttgaac ctggaggcag   3120
   cctgcagccc catcccatct cctgccctct cctgatctaa ctccctgctg catctcttgc   3180
   tctcattcct tagacgtcct ccccttttga ccccgttcct tcatccatcc tgcaccccag   3240
   tcccccagcc ctaaatcctc cctcctctcc tcacatcctg gtccctagca aggtatagat   3300
   agcctctgtg tcttaggata ccccgggtgc tgttccctcg gtcaccctgt tgcccagttc   3360
   cccgtttctc ttgctctcat tccttgtatc ttctcccctt ctgagcccgt ccattcatcg   3420
   gttctgcccc cgactccccc agccctaaat accccagctc ctaattcccc cctcaccccg   3480
   ttgctcaatt ccccgtttct cttgctctca ttccttgtat cttctcccct tctgagcctg 3540
   tccattcatc ggtggttctg cccctactcc cccagcccta aataccccag ctgctgttcc 3600
   tccccatcac ccagccaccg gattctccat tcaccccttt ctctcacccc tggagccccg 3660
   tgggtggggg cagggcatga gttccccagt ccccaaggaa aggcagcccc ctcagtctcc 3720
   ctcctcctca ttcccttcca tctccctccc ctctgccttt taaacccatc ccctccgatt 3780
   cccctcctcc cccctctctc cctggtgtca actcgattcc tgcggtaact ctgagccctg 3840
   aaatcctcag tctccttggc ggggaagatt ggctttgggg acaggaagtc ggcacatctc 3900
   caggtctcca tgtgcacaat atagagttta ttgtaaaaag c 3941
<210> 43
   <211> 1126
   <212> DNA
   <213> human
<400> 43
   cagatactct gacccatgga tcccctgggc ccggccaagc cacagtggtc gtggcgctgc 60
   tgtctgacca cgctgctgtt tcagctgctg atggctgtgt gtttcttctc ctatctgcgt 120
   gtgtctcaag acgatcccac tgtgtaccct aatgggtccc gcttcccaga cagcacaggg 180
   acccccgccc actccatccc cctgatcctg ctgtggacgt ggccttttaa caaacccata 240
   gctctgcccc gctgctcaga gatggtgcct ggcacggctg actgcaacat cactgccgac 300
   cgcaaggtgt atccacaggc agacgcggtc atcgtgcacc accgagaggt catgtacaac 360
   cccagtgccc agctcccacg ctccccgagg cggcaggggc agcgatggat ctggttcagc 420
   atggagtccc caagccactg ctggcagctg aaagccatgg acggatactt caatctcacc 480
   atgtcctacc gcagcgactc cgacatcttc acgccctacg gctggctgga gccgtggtcc 540
   ggccagcctg cccacccacc gctcaacctc tcggccaaga ccgagctggt ggcctgggca 600
   gtgtccaact gggggccaaa ctccgccagg gtgcgctact accagagcct gcaggcccat 660
   ctcaaggtgg acgtgtacgg acgctcccac aagcccctgc cccagggaac catgatggag 720
   acgctgtccc ggtacaagtt ctatctggcc ttcgagaact ccttgcaccc cgactacatc 780
   accgagaagc tgtggaggaa cgccctggag gcctgggccg tgcccgtggt gctgggcccc 840
   agcagaagca actacgagag gttcctgccg cccgacgcct tcatccacgt ggacgacttc 900
   cagagcccca aggacctggc ccggtacctg caggagctgg acaaggacca cgcccgctac 960
   ctgagctact ttcgctggcg ggagacgctg cggcctcgct ccttcagctg ggcactcgct 1020
   ttctgcaagg cctgctggaa actgcaggag gaatccaggt accagacacg cggcatagcg 1080
   gcttggttca cctgagaggc ccggcatggg gcctgggctg ccaggg 1126
<210> 44
   <211> 6129
   <212> DNA
   <213> human
<400> 44
<210> 45
   <211> 330
   <212> DNA
   <213> human
<400> 45
   gcggccgcag gtacccgggc tccacgtcag ggtagacctg gcgtccctca atgccttcca 60
   tgtagttggc cacgtaatcc accatctcct tccctctcct tcggaattca cttgcgttca 120
   tggtgtctgg gctctgtcag aggtgaaaaa tgctggaaat tcgaattcct tacagggcta 180
   ctctccttga tgggattctc caactttggg gactgaagag catgtggaga agctgctgag 240
   gcactcggca ctgagacagt cactcttctt gaaactccaa gccacacgtt tccctcttct 300
   tgcatttcca gccacatgtg cccctcgtgc 330
<210> 46
   <211> 2400
   <212> DNA
   <213> human
<400> 46
   ctgtagggga ggatattttg attgaacaca ggcttgacag aatcttcttt tcttcttaga 60
   aatcctagaa aacagaaagc aacaggaaga tgtcttattg ggaactaccc ccatcaactt 120
   caccatgagt caaacaagga agaaaacttc ctcagaagga gaaactaagc cccagacttc 180
   aactgtcaac aaatttctca ggggctccaa tgctgaaagc agaaaagagg acaatgacct 240
   taaaacaagt gattcccaac ccagcgactg gatacagaag acagccacct cagagactgc 300
   taagcctctc agttcagaaa tggaatggag atccagtatg gagaaaaatg agcatttcct 360
   gcagaagctg ggcaaaaagg ctgtcaacaa gtgtctagat ttgaataact gtggattaac 420
   aacagcggac atgaaagaaa tgggagaagc atttgagatg attcctgaac ttgaagagct 480
   aaatttgtct tggaacagta aagtgggagg aaatttgcct ctgatccttc agaagttcca 540
   aaaagggagc aagatacaaa tgattgagct tgtggattgc tccctcacgt cagaagatgg 600
   gacatttctg ggtcaactgc tacctatgct gcaaagtctc gaagtacttg atctttccat 660
   taacagagac attgttggca gtctgaacag tattgctcag ggattaaaaa gcacctcaaa 720
   tctgaaagta ctgaagttac attcatgtgg attatcacaa aagagtgtca aaatattgga 780
   tgctgctttt aggtatttgg gtgagctgag gaaattagat ctttcctgca ataaggatct 840
   aggtggaggt tttgaagact cgccggctca gttggtcatg ctaaagcatc tacaagtcct 900
   agatcttcac cagtgctcac taacagcaga tgacgtgatg tcactgaccc aggtcattcc 960
   tttactttca aatcttcaag aattggattt atcagccaac aaaaagatgg gcagttcttc 1020
   tgaaaactta ctcagcaggc tccgattttt accagcattg aagtcattag ttatcaacaa 1080
   ctgtgctttg gagagtgaga cttttacagc tcttgctgaa gcctctgttc acctctctgc 1140
   tctggaagta ttcaaccttt cttggaacaa gtgtgttggt ggcaacttgg agctgcttct 1200
   ggaaacacta aagctttcca tgtctcttca agtgctgagg ctgagcagct gttccctggt 1260
   gacagaggat gtggctctcc tggcatcggt catacagacg ggtcatctgg ccaaactgca 1320
   aaagctggac ctgagctaca atgacagcat ctgtgatgca gggtggacca tgttctgcca 1380
   aaacgtgcgg ttcctcaaag agctaatcga gctggatatt agccttcgac catcaaattt 1440
   tcgagattgt ggacaatggt ttagacactt gttatatgct gtgaccaagc ttcctcagat 1500
   cactgagata ggaatgaaaa gatggattct cccagcttca caggaggaag aactagaatg 1560
   ctttgaccaa gaaaaaaaaa agaagcattc actttgacca tggtgggttt cagtaaactg 1620
   atttcccatg tcctactaag ctacaaacca ttctccaaag gaaaagaaca tgaacgaatt 1680
   ccagagtcat gaactgaatt tcaacttctg ggccatttaa tgggacttat attacaagag 1740
   ctttgtaaat atatatatat attacatata tatatgtaat atacatatat acacatatat 1800
   ataatataca tatataatac acatatatat gtaaatatat atataatatc taatatgagc 1860
   atgccattat tctctgtcta tgaaacaaaa atggcatttt tcaatggatt tgttttggat 1920
   atataattag ttcatttgct gtttagaagc cttgccaaaa gtgtttagat tttggtactg 1980
   caactgcttt cctcttgccc agaaatgttt tgcctcttct tttcctacaa gttaaatgtt 2040
   ctaaatataa aggggtatgt gtgtgtgtgt gtaattctaa tgtgaaaggc actagctgtc 2100
   taatagtttc atgtatcatt actattacta tatgtatctt aatgtagtct atgtaggttt 2160
   ttatcagaaa gtgtaccttt ctatggttta ttattttata ttctggtgcc ttttatctca 2220
   gatataaacc atgaacagta atgatagtca ctgacatata aatcttagta aaaagtgatt 2280
   aaaaatctaa aactcagtat gaaaaacata tcttgttaga ataaattaaa accttttatt 2340
   gtttaaaaaa ttgttaaaaa aaaaaaaaaa aaaaaaaaaa aaaaaaaaaa aaaaaaaaaa 2400
<210> 47
   <211> 2308
   <212> DNA
   <213> human
<400> 47
   aagccacttt gacaacgttt ctgagccagg ggtgaccatg acctgctgcg aaggatggac 60
   atcctgcaat ggattcagcc tgctggttct actgctgtta ggagtagttc tcaatgtgat 120
   acctctaatt gtcagcttag ttgaggaaga ccaattttct caaaacccca tctcttgctt 180
   tgagtggtgg ttcccaggaa ttataggagc aggtctgatg gccattccag caacaacaat 240
   gtccttgaca gcaagaaaaa gagcgtgctg caacaacaga actggaatgt ttctttcatc 300
   acttttcagt gtgatcacag tcattggtgc tctgtattgc atgctgatat ccatccaggc 360
   tctcttaaaa ggtcctctca tgtgtaattc tccaagcaac agtaatgcca attgtgaatt 420
   ttcattgaaa aacatcagtg acattcatcc agaatccttc aacttgcagt ggtttttcaa 480
   tgactcttgt gcacctccta ctggtttcaa taaacccacc agtaacgaca ccatggcgag 540
   tggctggaga gcatctagtt tccacttcga ttctgaagaa aacaaacata ggcttatcca 600
   cttctcagta tttttaggtc tattgcttgt tggaattctg gaggtcctgt ttgggctcag 660
   tcagatagtc atcggtttcc ttggctgtct gtgtggagtc tctaagcgaa gaagtcaaat 720
   tgtgtagttt aatgggaata aaatgtaagt atcagtagtt tgaattaatt tgagaagtac 780
   acttgttttc aaagtcatct ttgagatgat ttaaaaaatc aacccttcac gtagaaagca 840
   cgttgtaaat gcataacact ctcatatcag tggttgattt gggaaaggtg gagagaattt 900
   tcaattagtt ttgtgttgta ctattcaaat tttttacctc ttcactgtgt gtagagaaag 960
   gagaagggaa ggaggatgag aaggaacgga agtcatcctg aaaataaaag tacaggactt 1020
   tttttttttt tttttgagac agggtctcaa aaaaggctgg agtacagtag tacagtggtg 1080
   ctatctcagc ttactgcagc ctcaacctcc tgggctcagg tgattctccc atctcagcct 1140
   ccctagtagc tgggactaca ggtgcgtgcc actatgccaa gctaattttt gtatttttag 1200
   tagagatggg ggttttccat attgcccagg ctggtcccga actcatggac tcaagtgatc 1260
   tgcctgcctc agcctcctaa agtgctgcga ttacaggcat gagccatcgc gcctaaagga 1320
   caggaccttt ttattgtatt tctttaaaga ataaatacat aacctgaatg caatcaagtc 1380
   tttagatcta attctcagct tgcagggaac actaggacaa atccaaaaag tgggtcagcg 1440
   ggcacagaat ggcccaattt tcaacaggaa aatgttataa aagaaaaata tttttgaggg 1500
   aactgttata gattaagaga atagaggcat gtttcagcta aacacatgta aactttgtca 1560
   gagataattg ggaggagtat gtagaagaat cggattattg ttaattttgg taggtctgat 1620
   aatggtttta tagtataaag gctgagtacc ccttatccaa aatgattaag atcagaagtg 1680
   ttttggcttt cacatttttt tggattttgg aattttgcct ataataatga gacatcttgg 1740
   ggatgggatg caagtctaac cacaaaattc atttatgtct catacacact ttgaacacct 1800
   ggcctgaagg taatttcaca caatatttta aataactttg tgcatgaaac acaattttga 1860
   ctgcattttg actgcaactc atcacatgag gtcaggtatg gaatttccca cttgtggtgt 1920
   tacgttactg gctcaaaaag ttttggatct cggagcattc tggattttga atttttggat 1980
   tagtgatgct caacctgtat acagaaatgt cctcattttt aaaaaaagaa atgcatattt 2040
   atatgtttta aaattacttc aaccaaaagc aacggggaga tgtttactgt tatatttagg 2100
   tgacaggtac atggcaattc attataccct cctattttcc tatgtttaca ttattcatta 2160
   atttaaaaac aatacctaga aaaacccaag actttcaaaa gctattttct atatgtgcca 2220
   atctttaaaa aacaggataa caagggtatt tatcacatta aaatgttgta aaacagcaaa 2280
   gctaaaatct aaaaaaaaaa aaaaaaaa 2308
<210> 48
   <211> 2880
   <212> DNA
   <213> human
<400> 48
   tgctgctctc cgcccgcgtc cggctcgtgg ccccctactt cgggcaccat ggacacctcc     60
   cggctcggtg tgctcctgtc cttgcctgtg ctgctgcagc tggcgaccgg gggcagctct    120
   cccaggtctg gtgtgttgct gaggggctgc cccacacact gtcattgcga gcccgacggc    180
   aggatgttgc tcagggtgga ctgctccgac ctggggctct cggagctgcc ttccaacctc    240
   agcgtcttca cctcctacct agacctcagt atgaacaaca tcagtcagct gctcccgaat    300
   cccctgccca gtctccgctt cctggaggag ttacgtcttg cgggaaacgc tctgacatac    360
   attcccaagg gagcattcac tggcctttac agtcttaaag ttcttatgct gcagaataat    420
   cagctaagac acgtacccac agaagctctg cagaatttgc gaagccttca atccctgcgt    480
   ctggatgcta accacatcag ctatgtgccc ccaagctgtt tcagtggcct gcattccctg 540
   aggcacctgt ggctggatga caatgcgtta acagaaatcc ccgtccaggc ttttagaagt 600
   ttatcggcat tgcaagccat gaccttggcc ctgaacaaaa tacaccacat accagactat 660
   gcctttggaa acctctccag cttggtagtt ctacatctcc ataacaatag aatccactcc 720
   ctgggaaaga aatgctttga tgggctccac agcctagaga ctttagattt aaattacaat 780
   aaccttgatg aattccccac tgcaattagg acactctcca accttaaaga actaggattt 840
   catagcaaca atatcaggtc gatacctgag aaagcatttg taggcaaccc ttctcttatt 900
   acaatacatt tctatgacaa tcccatccaa tttgttggga gatctgcttt tcaacattta 960
   cctgaactaa gaacactgac tctgaatggt gcctcacaaa taactgaatt tcctgattta 1020
   actggaactg caaacctgga gagtctgact ttaactggag cacagatctc atctcttcct 1080
   caaaccgtct gcaatcagtt acctaatctc caagtgctag atctgtctta caacctatta 1140
   gaagatttac ccagtttttc agtctgccaa aagcttcaga aaattgacct aagacataat 1200
   gaaatctacg aaattaaagt tgacactttc cagcagttgc ttagcctccg atcgctgaat 1260
   ttggcttgga acaaaattgc tattattcac cccaatgcat tttccacttt gccatcccta 1320
   ataaagctgg acctatcgtc caacctcctg tcgtcttttc ctataactgg gttacatggt 1380
   ttaactcact taaaattaac aggaaatcat gccttacaga gcttgatatc atctgaaaac 1440
   tttccagaac tcaaggttat agaaatgcct tatgcttacc agtgctgtgc atttggagtg 1500
   tgtgagaatg cctataagat ttctaatcaa tggaataaag gtgacaacag cagtatggac 1560
   gaccttcata agaaagatgc tggaatgttt caggctcaag atgaacgtga ccttgaagat 1620
   ttcctgcttg actttgagga agacctgaaa gcccttcatt cagtgcagtg ttcaccttcc 1680
   ccaggcccct tcaaaccctg tgaacacctg cttgatggct ggctgatcag aattggagtg 1740
   tggaccatag cagttctggc acttacttgt aatgctttgg tgacttcaac agttttcaga 1800
   tcccctctgt acatttcccc cattaaactg ttaattgggg tcatcgcagc agtgaacatg 1860
   ctcacgggag tctccagtgc cgtgctggct ggtgtggatg cgttcacttt tggcagcttt 1920
   gcacgacatg gtgcctggtg ggagaatggg gttggttgcc atgtcattgg ttttttgtcc 1980
   atttttgctt cagaatcatc tgttttcctg cttactctgg cagccctgga gcgtgggttc 2040
   tctgtgaaat attctgcaaa atttgaaacg aaagctccat tttctagcct gaaagtaatc 2100
   attttgctct gtgccctgct ggccttgacc atggccgcag ttcccctgct gggtggcagc 2160
   aagtatggcg cctcccctct ctgcctgcct ttgccttttg gggagcccag caccatgggc 2220
   tacatggtcg ctctcatctt gctcaattcc ctttgcttcc tcatgatgac cattgcctac 2280
   accaagctct actgcaattt ggacaaggga gacctggaga atatttggga ctgctctatg   2340
   gtaaaacaca ttgccctgtt gctcttcacc aactgcatcc taaactgccc tgtggctttc   2400
   ttgtccttct cctctttaat aaaccttaca tttatcagtc ctgaagtaat taagtttatc   2460
   cttctggtgg tagtcccact tcctgcatgt ctcaatcccc ttctctacat cttgttcaat   2520
   cctcacttta aggaggatct ggtgagcctg agaaagcaaa cctacgtctg gacaagatca   2580
   aaacacccaa gcttgatgtc aattaactct gatgatgtcg aaaaacagtc ctgtgactca   2640
   actcaagcct tggtaacctt taccagctcc agcatcactt atgacctgcc tcccagttcc   2700
   gtgccatcac cagcttatcc agtgactgag agctgccatc tttcctctgt ggcatttgtc   2760
   ccatgtctct aattaatatg tgaaggaaaa tgttttcaaa ggttgagaac ctgaaaatgt   2820
   gagattgagt atatcagagc agtaattaat aagaagagct gaggtgaaac tcggtttaaa   2880
<210> 49
   <211> 915
   <212> DNA
   <213> human
<400> 49
   atggatcccc tgggcccggc caagccacag tggtcgtggc gctgctgtct gaccacgctg     60
   ctgtttcagc tgctgatggc tgtgtgtttc ttctcctatc tgcgtgtgtc tcaagacgat    120
   cccactgtgt accctaatgg gtcccgcttc ccagacagca cagggacccc cgcccactcc    180
   atccccctga tcctgctgtg gacgtggcct tttaacaaac ccatagctct gccccgctgc    240
   tcagagatgg tgcctggcac ggctgactgc aacatcactg ccgaccgcaa ggtgtatcca    300
   caggcagacg cggtcatcgt gcaccaccga gaggtcatgt acaaccccag tgcccagctc    360
   ccacgctccc cgaggcggca ggggcagcga tggatctggt tcagcatgga gtccccaagc    420
   cactgctggc agctgaaagc catggacgga tacttcaatc tcaccatgtc ctaccgcagc    480
   gactccgaca tcttcacgcc ctacggctgg ctggagccgt ggtccggcca gcctgcccac    540
   ccaccgctca acctctcggc caagaccgag ctggtggcct gggcagtgtc caactggggg    600
   ccaaactccg ccagggtgcg ctactaccag agcctgcagg cccatctcaa ggtggacgtg    660
   tacggacgct cccacaagcc cctgccccag ggaaccatga tggagacgct gtcccggtac    720
   aagttctatc tggccttcga gaactccttg caccccgact acatcaccga gaagctgtgg    780
   aggaacgccc tggaggcctg ggccgtgccc gtggtgctgg gccccagcag aaggaacctc    840
   attttcctgg ggcctcacct gagtgggggc ctcatctacc taaggactcg tttgcctgaa    900
   gcttcacctg cctga                                                     915
<210> 50
   <211> 1095
   <212> DNA
   <213> human
<400> 50
   atggatcccc tgggcccggc caagccacag tggtcgtggc gctgctgtct gaccacgctg     60
   ctgtttcagc tgctgatggc tgtgtgtttc ttctcctatc tgcgtgtgtc tcaagacgat    120
   cccactgtgt accctaatgg gtcccgcttc ccagacagca cagggacccc cgcccactcc    180
   atccccctga tcctgctgtg gacgtggcct tttaacaaac ccatagctct gccccgctgc    240
   tcagagatgg tgcctggcac ggctgactgc aacatcactg ccgaccgcaa ggtgtatcca    300
   caggcagacg cggtcatcgt gcaccaccga gaggtcatgt acaaccccag tgcccagctc    360
   ccacgctccc cgaggcggca ggggcagcga tggatctggt tcagcatgga gtccccaagc    420
   cactgctggc agctgaaagc catggacgga tacttcaatc tcaccatgtc ctaccgcagc    480
   gactccgaca tcttcacgcc ctacggctgg ctggagccgt ggtccggcca gcctgcccac    540
   ccaccgctca acctctcggc caagaccgag ctggtggcct gggcagtgtc caactggggg    600
   ccaaactccg ccagggtgcg ctactaccag agcctgcagg cccatctcaa ggtggacgtg    660
   tacggacgct cccacaagcc cctgccccag ggaaccatga tggagacgct gtcccggtac    720
   aagttctatc tggccttcga gaactccttg caccccgact acatcaccga gaagctgtgg    780
   aggaacgccc tggaggcctg ggccgtgccc gtggtgctgg gccccagcag aagcaactac    840
   gagaggttcc tgccacccga cgccttcatc cacgtggacg acttccagag ccccaaggac    900
   ctggcccggt acctgcagga gctggacaag gaccacgccc gctacctgag ctactttcgc    960
   tggcgggaga cgctgcggcc tcgctccttc agctgggcac tcgctttctg caaggcctgc   1020
   tggaaactgc aggaggaatc cagtgggggc ctcatctacc taaggactcg tttgcctgaa   1080
   gcttcacctg cctga                                                    1095
<210> 51
   <211> 1182
   <212> DNA
   <213> human
<400> 51
   gtcctgagca gccaacacac cagcccagac agctgcaagt caccatggac gctgaaggcc 60
   tggcgctgct gctgccgccc gtcaccctgg cagccctggt ggacagctgg ctccgagagg 120
   actgcccagg gctcaactac gcagccttgg tcagcggggc aggcccctcg caggcggcgc 180
   tgtgggccaa atcccctggg gtactggcag ggcagccttt cttcgatgcc atatttaccc 240
   aactcaactg ccaagtctcc tggttcctcc ccgagggatc gaagctggtg ccggtggcca 300
   gagtggccga ggtccggggc cctgcccact gcctgctgct gggggaacgg gtggccctca 360
   acacgctggc ccgctgcagt ggcattgcca gtgctgccgc cgctgcagtg gaggccgcca 420
   ggggggccgg ctggactggg cacgtggcag gcacgaggaa gaccacgcca ggcttccggc 480
   tggtggagaa gtatgggctc ctggtgggcg gggccgcctc gcaccgctac gacctgggag 540
   ggctggtgat gttgaaggat aaccatgtgg tgccccccgg tggcgtggag aaggcggtgc 600
   gggcggccag acaggcggct gacttcgctc tgaaggtgga agtggaatgc agcagcctgc 660
   aggaggtcgt ccaggcagct gaggctggcg ccgaccttgt cctgctggac aacttcaagc 720
   cagaggagct gcaccccacg gccaccgcgc tgaaggccca gttcccgagt gtggctgtgg 780
   aagccagtgg gggcatcacc ctggacaacc tcccccagtt ctgcgggccg cacatagacg 840
   tcatctccat ggggatgctg acccaggcgg tcccagccct tgatttctcc ctcaagctgt 900
   ttgccaaaga ggtggctcca gtgcccaaaa tccactagtc ctaaaccgga agaggatgac 960
   accggccatg ggttaacgtg gctcctcagg accctctggg tcacacatct ttagggtcag 1020
   tgaacaatgg ggcacatttg gcactagctt gagcccaact ctggctctgc cacctgctgc 1080
   tcctgtgacc tgtcagggct gacttcacct ctgctcatct cagtttccta atctgtaaaa 1140
   tgggtctaat aaaggatcaa ccaaaaaaaa aaaaaaaaaa aa 1182
<210> 52
   <211> 3600
   <212> DNA
   <213> human
<400> 52
   gaatcaacag aatttgtctt tttgtgactg gtttatttca cttaacttca tcctcaaggt 60
   tcaacttaaa ggtgtatcca tgttgtagca cgtgtcagca ttttctttcg ttctcaggct 120
   aaatagtatt tcattgtgtg tgtacaccat gtttcatgca ttcattcatc ccttgaaaga 180
   ttggtgggtt gtttcctcct ttttgctttt gtgaacagtg ctacgaacat ggttgtacaa 240
   acatctcttg gagccccact agcagttcct ttgggtatat accccaaagt ggaattgctg 300
   gatctggtag ctcccttttt aattttttga ggaatcgcca cacagtttcc ataacagctg 360
   caccatttta cattcccaag accttttttt tttttttttt tttaagaaga aaagatgtgt 420
   ttctgcattt ctggaagtct atgctgcatt tccatttgtt gaaatttaag accagagtca 480
   tcttttctgc tgtaattata atggtcactg gcttgtgcct tttcctcctc tctctgcccc 540
   atctgcacgg ggtctttgaa caagtcccag caccttggtg gacaagcctg tgtccctggc 600
   ccatcatgga agccgctgcc tttcagagtg ggagtctgta ccctgttgcc tcattccttg 660
   ctgcgcccat gagtgagctt gtgcctgacc tctccttcca ggtggactta cacactgggc 720
   tgtcggagtt ctcggtgacg cagcgccggc tggcccatgg ctggaatgag tttgttgctg 780
   acaacagcga acctgtgtgg aagaaatacc tggatcagtt taagaacccc ctgatcctgc 840
   tgctgctggg ctctgccctg gtgagtgtcc tcaccaagga gtatgaggac gccgtcagca 900
   tcgccacggc agtgcttgtc gtggtcactg tcgccttcat ccaggagtac aggtcggaga 960
   aatctctgga agagctgacc aagctggttc ctccagaatg taactgccta agagaaggaa 1020
   aactccagca cctgcttgct cgagaactgg ttcctggtga tgtcgtatct ctctcgatcg 1080
   gagaccggat ccctgcagac atccgactca ctgaggtcac ggacctcttg gtggatgaat 1140
   ccagtttcac cggggaagcc gagccatgta gtaaaacaga cagccccttg acaggcggtg 1200
   gggacctcac caccctcagc aacatcgtct tcatggggac cctggtgcag tatgggaggg 1260
   gccagggggt cgtgattgga acaggggaaa gctctcagtt cggagaagtg tttaagatga 1320
   tgcaggctga agagacacct aaaactcctt tgcagaaaag catggacagg ctaggaaagc 1380
   aactgacact cttctccttt ggcataatcg gtctcatcat gctcattggc tggtcgcaag 1440
   ggaaacaact cctgagtatg ttcacgatcg gggtcagcct ggctgtggcg gctattccag 1500
   agggtctgcc catcgtcgtc atggtgacgc tggtcctggg agtgctgcgg atggccaaga 1560
   agcgggtcat cgtgaagaag ttacccatcg tggagacttt aggttgctgc agcgttctct 1620
   gttctgacaa gacggggact ctgactgcca atgaaatgac agtgacccag cttgtaacgt 1680
   cagatgggct tcgtgccgag gtcagcggag ttgggtatga cggtcaaggg actgtgtgtc 1740
   ttctaccatc caaggaagtc attaaggaat tttccaatgt ctcagtggga aagttagtgg 1800
   aggcgggctg tgttgccaac aatgcggtca tcagaaagaa cgccgtgatg gggcagccca 1860
   ccgagggtgc attgatggcc ctggcgatga agatggactt aagtgatatt aaaaattcat 1920
   atataagaaa aaaagagatt ccattcagtt cagagcagaa gtggatggcg gtgaaatgca 1980
   gtctgaagac tgaggatcag gaagacattt acttcatgaa aggggccttg gaagaggtga 2040
   tccgctactg caccatgtac aacaacgggg gcatccccct gccgctgacg ccccagcaga 2100
   ggtcattctg cctgcaggaa gagaagagga tggggtcgct cggtttgcgg gtgctggccc 2160
   tggcttctgg gcccgagctg gggcggctga cgtttctagg tcttgtgggc atcattgacc 2220
   ccccgagagt tggcgtgaag gaagcagtcc aggttctctc cgagtctggt gtgtctgtga 2280
   agatgataac gggggatgcc ctggagacgg ccttggccat aggaagaaac atcggcctgt 2340
   gcaacgggaa gctgcaagcc atgtccgggg aggaggtgga cagcgtggag aagggcgagc 2400
   tggccgaccg cgtggggaag gtgtccgtgt tcttcaggac cagcccaaag cacaagctca 2460
   aaatcatcaa ggctctgcag gagtcagggg cgatcgtggc catgactggg gatggggtga 2520
   acgacgcagt ggccctgaag tctgcagaca ttgggatcgc catggggcag acagggacgg 2580
   acgtcagcaa agaggccgcc aacatgatcc tggtggatga tgacttctca gccatcatga 2640
   atgcagtgga ggaaggcaag ggtatttttt acaacatcaa aaactttgtc cgattccagc 2700
   tgagcacgag catctccgcc ctgagtctca tcactctgtc caccgtgttc aacctgccca 2760
   gccccctcaa cgccatgcag atcctatgga tcaacatcat catggatggg ccaccggcgc 2820
   agagcttggg ggtagagccc gttgacaaag acgccttcag gcagccacca cggagtgtgc 2880
   gggacaccat cctcagcaga gccctcatcc tgaagatcct catgtccgcg gccatcatca 2940
   tcagcgggac cctctttatc ttctggaagg agatgcctga agacagagca agcactcccc 3000
   gcaccacgac gatgacgttc acttgttttg tgtttttcga tctcttcaac gccttgacct 3060
   gccgctctca gaccaagctg atatttgaga tcggctttct caggaaccac atgttcctct 3120
   actccgtcct ggggtccatc ctggggcagc tggcggtcat ttacatcccc ccgctgcaga 3180
   gggtcttcca gacggagaac ctgggagcgc ttgatttgct gtttttaact ggattggcct 3240
   catccgtctt cattttgtca gagctcctca aactatgtga aaaatactgt tgcagcccca 3300
   agagagtcca gatgcaccct gaagatgtgt agtggaccgc actccgcggc accttcccta 3360
   atcatctcga tctggttgtg actgtggccc ctgccgtgtc tcctcgtcag gggagacttt 3420
   taggaggccg cagccttcca tcaccggatc agtttttcct cttaggaaag ctgcaggaac 3480
   ctcgtgggct ccagggaccc aggcccacat ccatccagcg ttcccgctgg ctgtgggaca 3540
   gacagggagg ggcctgtaca gaaacaccac actgtttatt aaatcacaat gatttttatt 3600
<210> 53
   <211> 4192
   <212> DNA
   <213> human
<400> 53
   tccaagctca aagaagcaga ggccgctgtt cgtttccttt aggtctttcc actaaagtcg     60
   gagtatcttc ttccaagatt tcacgtcttg gtggccgttc caaggagcgc gaggtcggga    120
   tggatcttga aggggaccgc aatggaggag caaagaagaa gaactttttt aaactgaaca    180
   ataaaagtga aaaagataag aaggaaaaga aaccaactgt cagtgtattt tcaatgtttc    240
   gctattcaaa ttggcttgac aagttgtata tggtggtggg aactttggct gccatcatcc    300
   atggggctgg acttcctctc atgatgctgg tgtttggaga aatgacagat atctttgcaa    360
   atgcaggaaa tttagaagat ctgatgtcaa acatcactaa tagaagtgat atcaatgata    420
   cagggttctt catgaatctg gaggaagaca tgaccaggta tgcctattat tacagtggaa    480
   ttggtgctgg ggtgctggtt gctgcttaca ttcaggtttc attttggtgc ctggcagctg    540
   gaagacaaat acacaaaatt agaaaacagt tttttcatgc tataatgcga caggagatag    600
   gctggtttga tgtgcacgat gttggggagc ttaacacccg acttacagat gatgtctcca 660
   agattaatga aggaattggt gacaaaattg gaatgttctt tcagtcaatg gcaacatttt 720
   tcactgggtt tatagtagga tttacacgtg gttggaagct aacccttgtg attttggcca 780
   tcagtcctgt tcttggactg tcagctgctg tctgggcaaa gatactatct tcatttactg 840
   ataaagaact cttagcgtat gcaaaagctg gagcagtagc tgaagaggtc ttggcagcaa 900
   ttagaactgt gattgcattt ggaggacaaa agaaagaact tgaaaggtac aacaaaaatt 960
   tagaagaagc taaaagaatt gggataaaga aagctattac agccaatatt tctataggtg 1020
   ctgctttcct gctgatctat gcatcttatg ctctggcctt ctggtatggg accaccttgg 1080
   tcctctcagg ggaatattct attggacaag tactcactgt attttctgta ttaattgggg 1140
   cttttagtgt tggacaggca tctccaagca ttgaagcatt tgcaaatgca agaggagcag 1200
   cttatgaaat cttcaagata attgataata agccaagtat tgacagctat tcgaagagtg 1260
   ggcacaaacc agataatatt aagggaaatt tggaattcag aaatgttcac ttcagttacc 1320
   catctcgaaa agaagttaag atcttgaagg gtctgaacct gaaggtgcag agtgggcaga 1380
   cggtggccct ggttggaaac agtggctgtg ggaagagcac aacagtccag ctgatgcaga 1440
   ggctctatga ccccacagag gggatggtca gtgttgatgg acaggatatt aggaccataa 1500
   atgtaaggtt tctacgggaa atcattggtg tggtgagtca ggaacctgta ttgtttgcca 1560
   ccacgatagc tgaaaacatt cgctatggcc gtgaaaatgt caccatggat gagattgaga 1620
   aagctgtcaa ggaagccaat gcctatgact ttatcatgaa actgcctcat aaatttgaca 1680
   ccctggttgg agagagaggg gcccagttga gtggtgggca gaagcagagg atcgccattg 1740
   cacgtgccct ggttcgcaac cccaagatcc tcctgctgga tgaggccacg tcagccttgg 1800
   acacagaaag cgaagcagtg gttcaggtgg ctctggataa ggccagaaaa ggtcggacca 1860
   ccattgtgat agctcatcgt ttgtctacag ttcgtaatgc tgacgtcatc gctggtttcg 1920
   atgatggagt cattgtggag aaaggaaatc atgatgaact catgaaagag aaaggcattt 1980
   acttcaaact tgtcacaatg cagacagcag gaaatgaagt tgaattagaa aatgcagctg 2040
   atgaatccaa aagtgaaatt gatgccttgg aaatgtcttc aaatgattca agatccagtc 2100
   taataagaaa aagatcaact cgtaggagtg tccgtggatc acaagcccaa gacagaaagc 2160
   ttagtaccaa agaggctctg gatgaaagta tacctccagt ttccttttgg aggattatga 2220
   agctaaattt aactgaatgg ccttattttg ttgttggtgt attttgtgcc attataaatg 2280
   gaggcctgca accagcattt gcaataatat tttcaaagat tataggggtt tttacaagaa 2340
   ttgatgatcc tgaaacaaaa cgacagaata gtaacttgtt ttcactattg tttctagccc 2400
   ttggaattat ttcttttatt acatttttcc ttcagggttt cacatttggc aaagctggag 2460
   agatcctcac caagcggctc cgatacatgg ttttccgatc catgctcaga caggatgtga   2520
   gttggtttga tgaccctaaa aacaccactg gagcattgac taccaggctc gccaatgatg   2580
   ctgctcaagt taaaggggct ataggttcca ggcttgctgt aattacccag aatatagcaa   2640
   atcttgggac aggaataatt atatccttca tctatggttg gcaactaaca ctgttactct   2700
   tagcaattgt acccatcatt gcaatagcag gagttgttga aatgaaaatg ttgtctggac   2760
   aagcactgaa agataagaaa gaactagaag gtgctgggaa gatcgctact gaagcaatag   2820
   aaaacttccg aaccgttgtt tctttgactc aggagcagaa gtttgaacat atgtatgctc   2880
   agagtttgca ggtaccatac agaaactctt tgaggaaagc acacatcttt ggaattacat   2940
   tttccttcac ccaggcaatg atgtattttt cctatgctgg atgtttccgg tttggagcct   3000
   acttggtggc acataaactc atgagctttg aggatgttct gttagtattt tcagctgttg   3060
   tctttggtgc catggccgtg gggcaagtca gttcatttgc tcctgactat gccaaagcca   3120
   aaatatcagc agcccacatc atcatgatca ttgaaaaaac ccctttgatt gacagctaca   3180
   gcacggaagg cctaatgccg aacacattgg aaggaaatgt cacatttggt gaagttgtat   3240
   tcaactatcc cacccgaccg gacatcccag tgcttcaggg actgagcctg gaggtgaaga   3300
   agggccagac gctggctctg gtgggcagca gtggctgtgg gaagagcaca gtggtccagc   3360
   tcctggagcg gttctacgac cccttggcag ggaaagtgct gcttgatggc aaagaaataa   3420
   agcgactgaa tgttcagtgg ctccgagcac acctgggcat cgtgtcccag gagcccatcc   3480
   tgtttgactg cagcattgct gagaacattg cctatggaga caacagccgg gtggtgtcac   3540
   aggaagagat tgtgagggca gcaaaggagg ccaacataca tgccttcatc gagtcactgc   3600
   ctaataaata tagcactaaa gtaggagaca aaggaactca gctctctggt ggccagaaac   3660
   aacgcattgc catagctcgt gcccttgtta gacagcctca tattttgctt ttggatgaag   3720
   ccacgtcagc tctggataca gaaagtgaaa aggttgtcca agaagccctg gacaaagcca   3780
   gagaaggccg cacctgcatt gtgattgctc accgcctgtc caccatccag aatgcagact   3840
   taatagtggt gtttcagaat ggcagagtca aggagcatgg cacgcatcag cagctgctgg   3900
   cacagaaagg catctatttt tcaatggtca gtgtccaggc tggaacaaag cgccagtgaa   3960
   ctctgactgt atgagatgtt aaatactttt taatatttgt ttagatatga catttattca   4020
   aagttaaaag caaacactta cagaattatg aagaggtatc tgtttaacat ttcctcagtc   4080
   aagttcagag tcttcagaga cttcgtaatt aaaggaacag agtgagagac atcatcaagt   4140
   ggagagaaat catagtttaa actgcattat aaattttata acagaattaa ag           4192
<210> 54
   <211> 771
   <212> DNA
   <213> human
<400> 54
   gctgtctcta cacacgtggc cctcggggcc tacgccccgc tcacaaagca tgggacactg      60
   gtggtggagg atgtggtggc atcctgcttc gcggccgtgg ctgaccacca cctggctcag     120
   ttggccttct ggcccctgag actctttcac agcttggcat ggggcagctg gaccccgggg     180
   gagggtgtgc attggtaccc ccagctgctc taccgcctgg ggcgtctcct gctagaagag     240
   ggcagcttcc acccactggg catgtccggg gcagggagct gaaaggactc caccgctgcc     300
   ctcctggaac tgctgtactg ggtccagaag cctctcagcc aggagggagc tggccctgga     360
   agggacctga gctgggggac actggctcct gccatctcct ctgccatgaa gatacaccat     420
   tgagacttga ctgggcaaca ccagcgtccc ccacccgcgc gtggtgtagt catagagctg     480
   caagctgagc tggcgagggg atggttgttg acccctctct cctagagacc ttgaggctgg     540
   cacgggactc ccaactcagc ctgctctcac tacgagtttt catactctgc ctcccccatt     600
   ggggagggcc cattccatcc atctttaggc ccctttgggt gggcttgcgc ctcagtttga     660
   tgctgctaaa ttcccctggg agccagcatg gatctggtgg accgatgctg tcagaactgg     720
   gaaggcacca gggtggggca gcatcccggg cattctgagg tatgacattc c              771
<210> 55
   <211> 4446
   <212> DNA
   <213> human
<400> 55
   ttcttaaccc tttccagctt tcccaccctc tttggcttta gccatggcct tctgatctgt     60
   gtttctcagg ggacctgcag gccccagata tagccccatg ctgtcctcct accccagagc    120
   acactgttca ggctacttcc actggtactg aaatccagta tttcacttac tcttttcctt    180
   tccaatatcc tcatgacatt caatatttca cttactctag gtcctccctg cctaaggccc    240
   aagtcaactt tctgtccagt gggatttgta atccaatgcc tcctagccct agcagaatcc    300
   catgtggata atcagaaatg tgactggaaa aaggacagag ctctatggct gtgggtccca    360
   gtccccactg ctggcagtaa gtccccagca gtgagctgtg taagcacctt acattctgcg    420
   cttggttgaa aacagcaagg caagcatcca cttgagaaat gtcaacccct aggaaatccc    480
   agcctcaagt ctttctcatc ccttgggaag tgcaaattgg atagagaaga aaccaattaa    540
   aaacaaaaca aacaaatcat acttagatat tctggctttt ctcaccaggg ctggattaaa    600
   gcatgtactt caaaataata acaacttaag tcaataaata aatgtaagga agtccaaatg    660
   ttcacctgaa gacaactgtg gtcatttttt ggcaatccca ggttctcttt tctacctgtt    720
   tgctcaatcg tggtctccct ctccctctct tgttggggcc catgcccctg ctttactgtt 780
   gccagaggct tgtacttgtt tgccttttag gtaggagcag ttacttccac tcccctcacc 840
   tgccataaag catctttata aacaaagcaa gtagaagaaa cacatcctgg tatccaccac 900
   attcggcttt tgttgattct gttcacttgg gagcacctgc tgctagggaa taagaaggtt 960
   gaggctgaag agtgaggact cttcagctcc cctctggcag gacccgggag aggaaagagc 1020
   cctcagctgg tccatcctcc ccactcctgg tcagccttct gttctgagat caaagtggtg 1080
   gggtcacatt ctcgagaact gtgctcagcc ccctcatctc acaccctttc cctctccctg 1140
   tgtgcctgcc cccctcttac ataaccatgc tggtgattgg caccgtcata aatcaatact 1200
   ttgctcactt tcacatcaag taacactatc cagggaggtg gtttcaacaa aggaggaagt 1260
   ataaggagat ctaggttcaa attaatgttg cccctagtgg taaaggacag agaccctcag 1320
   actgatgaaa tgcactcaga attacttaga caaagcggat atttgccact ctcttcccct 1380
   tttcctgtgt ttttgtagtg aagagacctg aaagaaaaaa gtagggagaa cataatgaga 1440
   acaaatacgg taatctcttc atttgctagt tcaagtgctg gacttgggac ttaggagggg 1500
   caatggagcc gcttagtgcc tacatctgac ttggactgaa atataggtga gagacaagat 1560
   tgtctcatat ccggggaaat cataacctat gactaggacg ggaagaggaa gcactgcctt 1620
   tacttcagtg ggaatctcgg cctcagcctg caagccaagt gttcacagtg agaaaagcaa 1680
   gagaataagc taatactcct gtcctgaaca aggcagcggc tccttggtaa agctactcct 1740
   tgatcgatcc tttgcaccgg attgttcaaa gtggacccca ggggagaagt cggagcaaag 1800
   aacttaccac caagcagtcc aagaggccca gaagcaaacc tggaggtgag acccaaagaa 1860
   agctggaacc atgctgactt tgtacactgt gaggacacag agtctgttcc tggaaagccc 1920
   agtgtcaacg cagatgagga agtcggaggt ccccaaatct gccgtgtatg tggggacaag 1980
   gccactggct atcacttcaa tgtcatgaca tgtgaaggat gcaagggctt tttcaggagg 2040
   gccatgaaac gcaacgcccg gctgaggtgc cccttccgga agggcgcctg cgagatcacc 2100
   cggaagaccc ggcgacagtg ccaggcctgc cgcctgcgca agtgcctgga gagcggcatg 2160
   aagaaggaga tgatcatgtc cgacgaggcc gtggaggaga ggcgggcctt gatcaagcgg 2220
   aagaaaagtg aacggacagg gactcagcca ctgggagtgc aggggctgac agaggagcag 2280
   cggatgatga tcagggagct gatggacgct cagatgaaaa cctttgacac taccttctcc 2340
   catttcaaga atttccggct gccaggggtg cttagcagtg gctgcgagtt gccagagtct 2400
   ctgcaggccc catcgaggga agaagctgcc aagtggagcc aggtccggaa agatctgtgc 2460
   tctttgaagg tctctctgca gctgcggggg gaggatggca gtgtctggaa ctacaaaccc 2520
   ccagccgaca gtggcgggaa agagatcttc tccctgctgc cccacatggc tgacatgtca 2580
   acctacatgt tcaaaggcat catcagcttt gccaaagtca tctcctactt cagggacttg 2640
   cccatcgagg accagatctc cctgctgaag ggggccgctt tcgagctgtg tcaactgaga 2700
   ttcaacacag tgttcaacgc ggagactgga acctgggagt gtggccggct gtcctactgc 2760
   ttggaagaca ctgcaggtgg cttccagcaa cttctactgg agcccatgct gaaattccac 2820
   tacatgctga agaagctgca gctgcatgag gaggagtatg tgctgatgca ggccatctcc 2880
   ctcttctccc cagaccgccc aggtgtgctg cagcaccgcg tggtggacca gctgcaggag 2940
   caattcgcca ttactctgaa gtcctacatt gaatgcaatc ggccccagcc tgctcatagg 3000
   ttcttgttcc tgaagatcat ggctatgctc accgagctcc gcagcatcaa tgctcagcac 3060
   acccagcggc tgctgcgcat ccaggacata cacccctttg ctacgcccct catgcaggag 3120
   ttgttcggca tcacaggtag ctgagcggct gcccttgggt gacacctccg agaggcagcc 3180
   agacccagag ccctctgagc cgccactccc gggccaagac agatggacac tgccaagagc 3240
   cgacaatgcc ctgctggcct gtctccctag ggaattcctg ctatgacagc tggctagcat 3300
   tcctcaggaa ggacatgggt gccccccacc cccagttcag tctgtaggga gtgaagccac 3360
   agactcttac gtggagagtg cactgacctg taggtcagga ccatcagaga ggcaaggttg 3420
   ccctttcctt ttaaaaggcc ctgtggtctg gggagaaatc cctcagatcc cactaaagtg 3480
   tcaaggtgtg gaagggacca agcgaccaag gataggccat ctggggtcta tgcccacata 3540
   cccacgtttg ttcgcttcct gagtcttttc attgctacct ctaatagtcc tgtctcccac 3600
   ttcccactcg ttcccctcct cttccgagct gctttgtggg ctccaggcct gtactcatcg 3660
   gcaggtgcat gagtatctgt gggagtcctc tagagagatg agaagccagg aggcctgcac 3720
   caaatgtcag aagcttggca tgacctcatt ccggccacat cattctgtgt ctctgcatcc 3780
   atttgaacac attattaagc accgataata ggtagcctgc tgtggggtat acagcattga 3840
   ctcagatata gatcctgagc tcacagagtt tatagttaaa aaaacaaaca gaaacacaaa 3900
   caatttggat caaaaggaga aatgataagt gacaaaagca gcacaaggaa tttccctgtg 3960
   tggatgctga gctgtgatgg cgggcactgg gtacccaagt gaaggttccc gaggacatga 4020
   gtctgtagga gcaagggcac aaactgcagc tgtgagtgcg tgtgtgtgat ttggtgtagg 4080
   taggtctgtt tgccacttga tggggcctgg gtttgttcct ggggctggaa tgctgggtat 4140
   gctctgtgac aaggctacgc tgacaatcag ttaaacacac cggagaagaa ccatttacat 4200
   gcaccttata tttctgtgta cacatctatt ctcaaagcta aagggtatga aagtgcctgc 4260
   cttgtttata gccacttgtg agtaaaaatt tttttgcatt ttcacaaatt atactttata 4320
   taaggcattc cacacctaag aactagtttt gggaaatgta gccctgggtt taatgtcaaa 4380
   tcaaggcaaa aggaattaaa taatgtactt ttggctaaaa aaaaaaaaaa aaaaaaaaaa 4440
   aaaaaa 4446
<210> 56
   <211> 1276
   <212> DNA
   <213> human
<400> 56
   tgagatcact tcccttgcac agtttggaag ggagagcact ttattacaga ccttggaagc 60
   aagaggattg cattcagcct agttcctggt tgctggccaa agggatcatg gacattgaag 120
   catattttga aagaattggc tataagaact ctaggaacaa attggacttg gaaacattaa 180
   ctgacattct tgagcaccag atccgggctg ttccctttga gaaccttaac atgcattgtg 240
   ggcaagccat ggagttgggc ttagaggcta tttttgatca cattgtaaga agaaaccggg 300
   gtgggtggtg tctccaggtc aatcaacttc tgtactgggc tctgaccaca atcggttttc 360
   agaccacaat gttaggaggg tatttttaca tccctccagt taacaaatac agcactggca 420
   tggttcacct tctcctgcag gtgaccattg acggcaggaa ttacattgtc gatgctgggt 480
   ctggaagctc ctcccagatg tggcagcctc tagaattaat ttctgggaag gatcagcctc 540
   aggtgccttg cattttctgc ttgacagaag agagaggaat ctggtacctg gaccaaatca 600
   ggagagagca gtatattaca aacaaagaat ttcttaattc tcatctcctg ccaaagaaga 660
   aacaccaaaa aatatactta tttacgcttg aacctcgaac aattgaagat tttgagtcta 720
   tgaatacata cctgcagacg tctccaacat cttcatttat aaccacatca ttttgttcct 780
   tgcagacccc agaaggggtt tactgtttgg tgggcttcat cctcacctat agaaaattca 840
   attataaaga caatacagat ctggtcgagt ttaaaactct cactgaggaa gaggttgaag 900
   aagtgctgaa aaatatattt aagatttcct tggggagaaa tctcgtgccc aaacctggtg 960
   atggatccct tactatttag aataaggaac aaaataaacc cttgtgtatg tatcacccaa 1020
   ctcactaatt atcaacttat gtgctatcag atatcctctc taccctcacg ttattttgaa 1080
   gaaaatccta aacatcaaat actttcatcc ataaaaatgt cagcatttat taaaaaacaa 1140
   taacttttta aagaaacata aggacacatt ttcaaattaa taaaaataaa ggcattttaa 1200
   ggatggcctg tgattatctt gggaagcaga gtgattcatg ctagaaaaca tttaatattg 1260
   atttattgtt gaattc 1276
<210> 57
   <211> 4999
   <212> DNA
   <213> human
<400> 57
   gaggaggatt cgcagttcaa catcaaggtc cctgtgcgtt ttattgcgac ctgccggtgg 60
   gaactttgtc tccgagtcgg agcagcatgg agcggcggag cgagagcccg tgtctgcggg 120
   acagccccga ccggcggagc ggcagcccgg acgtcaaggg gcctccccca gtgaaggtgg 180
   cccggctgga gcagaacggc agccccatgg gagcccgcgg gaggcccaac ggcgccgtgg 240
   ccaaggccgt gggaggtttg atgattcctg tcttttgtgt cgtggagcag ttggacggct 300
   ctcttgaata tgacaacaga gaagaacacg ccgagtttgt cctggtgcgg aaagatgtgc 360
   tttttagcca gctggtggag actgcgctcc tggccctggg gtattctcac agctctgcgg 420
   cccaggccca aggaataatc aagctgggaa ggtggaaccc tctccccctc agttatgtga 480
   cagatgcacc cgacgcgaca gtggccgaca tgctacaaga tgtctatcat gttgtgacgt 540
   tgaaaatcca attacaaagt tgttcaaagt tggaagactt gcctgcggag cagtggaacc 600
   atgccacagt ccgcaatgcc ttaaaggaac tgctcaaaga gatgaaccag agcacattag 660
   ccaaagaatg ccctctctcc cagagtatga tttcatccat tgtaaatagc acatattatg 720
   ccaatgtgtc agcaaccaag tgccaggagt ttgggagatg gtataaaaag tacaagaaga 780
   ttaaagtgga aagagtggaa cgagaaaacc tttcagacta ttgtgttctg ggccagcgtc 840
   caatgcattt accaaatatg aaccagctgg catccctggg gaaaaccaac gaacagtctc 900
   ctcacagcca aattcaccac agtactccaa tccgaaacca agtgcccgca ttacagccca 960
   tcatgagccc tggtcttctt tctccccagc ttagtccaca acttgtaagg caacaaatag 1020
   ccatggccca tctgataaac caacagattg ccgttagccg gctcctggct caccagcatc 1080
   ctcaagccat caaccagcag ttcctgaacc atccacccat ccccagagca gttaagccag 1140
   agccaaccaa ctcttccgtg gaagtctctc cagatatcta ccagcaagtc agagatgagc 1200
   tgaagagggc cagtgtgtcc caagctgtct ttgcaagagt ggcattcaac cgcacacagg 1260
   gattgttgtc tgagattctg cgtaaggaag aagaccctcg gacagcctct cagtctcttc 1320
   tagtaaacct gagggccatg cagaatttcc tcaatctgcc agaagtggag cgagatcgca 1380
   tctaccagga tgagagggag cggagcatga atcccaatgt gagcatggtc tcctcggcct 1440
   ccagcagtcc cagctcctcc cgaacccctc aggccaaaac ctcgacaccg acaacagacc 1500
   tccctattaa ggtggacggc gccaacatca acatcacagc tgccatttat gacgagatcc 1560
   aacaggagat gaaaagggcc aaggtgtctc aagccctgtt tgccaaagtg gctgcaaata 1620
   aaagtcaggg ctggctgtgt gaactgctcc gctggaagga gaacccaagc ccagaaaacc 1680
   gcaccctctg ggaaaacctc tgtaccatcc gtcgcttcct gaaccttccc cagcatgaga 1740
   gggatgtcat ctatgaggag gagtcaaggc atcaccacag cgaacgcatg caacacgtgg 1800
   tccagcttcc ccctgagccg gtgcaggtac ttcatagaca gcagtctcag ccagccaagg   1860
   agagttcccc tcccagagaa gaagcgcctc ccccacctcc tccgactgaa gacagttgtg   1920
   ccaaaaagcc ccggtctcgc acaaagatct ccttagaagc cctggggatc ctccaaagct   1980
   ttattcatga tgtaggcctg tacccagacc aggaagccat ccacactctt tcggctcagc   2040
   tggatctccc caaacacacc atcatcaagt tcttccagaa ccagcggtac cacgtgaagc   2100
   accacgggaa gctgaaagag cacctgggct ccgcggtgga cgtggctgaa tataaggacg   2160
   aggagctgct gaccgagtca gaggagaacg acagcgagga aggctccgag gagatgtaca   2220
   aagtggaggc tgaggaggaa aatgctgaca aaagcaaggc agcacctgcc gaaattgacc   2280
   agagataatg tgaacttcta ctaggcaaag caatacatcg gtccaaggat tttctgcttt   2340
   catttcttta aaagtttttt gttagtttgt tttttgtttt tgtttttggg tttttttggc   2400
   tttatttttg tctttttatg tctgttttgt ttttcttacc cttttggaca tttctttgtt   2460
   gcacaggata cacctataga ctgaataagt tcagtatttc cgaatcagac atcgccttgg   2520
   caaagacact aaagcgttac actttatccc gtctctatga ctggatcata gtcattataa   2580
   tcacaggaga ctctgccttc attatccttg cacttaacgg aagttacatc aggcaagttc   2640
   caggatgaaa agaactatga aataaatgaa ggaagctaca agtgtgtgtg tatatgtata   2700
   tgtatatatc tctatattta catatatata ttaaaattgc atgggacaga gactttgcaa   2760
   tccgaaagaa tagactgtga aatgagttct taaagaaaag acttgtttat gtattaaaaa   2820
   aaccacttca cagtgagtcg ctttggcttt ttgataaact gcggcctgct ctcagggtgg   2880
   ggtgactatt tttgaattcc tatttatttt ttgtgtttgt ccctgatttt tttttttaat   2940
   tctatggctt cctatctggc agcttaatgg gtaatttttg aggtatgtat ttaacaaaat   3000
   aaacgacact gccgaaaaaa aaaaaagtga agtgaaaaca atcagggcac attaaaatga   3060
   tacaagtcaa ataaatctta aagacacaat gcacacttaa aatgactcaa taaaatgact   3120
   tgctacgttc cgttattcaa tttgtcatta ctgtagtgaa cagatgcatt tctgtggaat   3180
   tccaaataag taaaactgaa attcagtgca gagaaaactt tgtccactag tgcaagtctt   3240
   gatcaaatga cattttgaca ttggacatat ggaattcata gtatgagcca cattttgttg   3300
   tgaaatttat ttacctgctt gtggcttcaa atctgaaaat taataagcct gctcgtttaa   3360
   aagttgtttg ttgttgctgt ttttttgtct ttttgttttt tactagaaaa tagttcagtg   3420
   taatattaag ttagaaaaga agttgctgcc cagttaaagg ggctccctct caaataaatc   3480
   tccatccttc cctctcccaa aagacatttc tgatttctgc ttcactttgg gcttcctctt   3540
   cttcgtacac attccatcta cctaatcaaa cattttcagt ccctgatctc tcctgtccct   3600
   tttcctggga tgacagccct aacaagaact gtttttgaat cgttgtgcag ctccaggcaa 3660
   tagagtatgt gaagcgattt cagtagaatc acttactcat cctaaaagaa aacattatcc 3720
   cagttaccta catcgcaatt accttatgta aagcagaact aatgctgact ggatgtttaa 3780
   tgggatgagc attaaagctg caatctacta tagtactcca gatctctttc ggcttcctat 3840
   gagaaacacc agaagcatta ctttccactt ctacttacag taattgcaag aggagacctc 3900
   acattcagga ctggcctagt gaacgtaatc catgctttaa actggccatt aaacagtccc 3960
   acatggttgg attttttttt tttttttgag ttgtgctttc acaaaacctt gtcaaagacc 4020
   tcatgcaata tcactttgaa agttattttc tgtttactac acaaacattg taatataact 4080
   gttaatacta tttatatatt tgaaaggtat aaaaggtagg agttaaaaaa aaaacctcta 4140
   tgtgtagata ttaactcaga acttacaata tacagggaga agacatgttg caatacaagc 4200
   taattctagc tgctcagtaa cctctggagt ttttaaaggg acattttcct gtactttttc 4260
   aaataatgat gtttaaaaat tatcttgaca taagcgtcat atacctttgc aaaaggatgg 4320
   ttgtttgcag ttagccctgg ccccatcctt cctatttctg tagtatgctg cagctttaat 4380
   cagaaagtcc atggttgctg cttcctgatc tccgagttac tctttccaaa ttgtcttctt 4440
   acactgttgc tgaaggtcac tctgtacacg taatggaaac tgattttgcc aagctcttac 4500
   aaggtggttc atctatcgat ggcatccgca tttggtatct tttacacttc aaccaaaaat 4560
   ttattaggta tttttcaatg ctaagtcttg ccttttattt tttaatttca ctgccaagtt 4620
   tgcagtggtt ctaagtgaat ctgtgggcat tttagcctgt ggtcttgcca gatctttgcg 4680
   aattacaatg catatatgtc tatttattca atatctgtca tataatatct atttggaaga 4740
   agaaactttc tcttgtagtg cctcttgaca aagcacaatt tcccgccttt tttttttttt 4800
   ttgtgaaatg aaaaaaacaa attgtgtttt attgcggtat caacaatgtg aataaggatt 4860
   aacatattgt aaatgttctt ttttccatgt aaatcaacta tctttgttat cactaagtga 4920
   taattaattt ttaacttatg tgcattgtta ggctgttaga attttttggt tgttaaaata 4980
   aacgcattca ataaatatg 4999
<210> 58
   <211> 1117
   <212> DNA
   <213> human
<400> 58
   atctcccact cctgcagctc ttctcacagg accagccact agcgcagcct cgagcgatgg     60
   cctatgtccc cgcaccgggc taccagccca cctacaaccc gacgctgcct tactaccagc    120
   ccatcccggg cgggctcaac gtgggaatgt ctgtttacat ccaaggagtg gccagcgagc    180
   acatgaagcg gttcttcgtg aactttgtgg ttgggcagga tccgggctca gacgtcgcct    240
   tccacttcaa tccgcggttt gacggctggg acaaggtggt cttcaacacg ttgcagggcg    300
   ggaagtgggg cagcgaggag aggaagagga gcatgccctt caaaaagggt gccgcctttg    360
   agctggtctt catagtcctg gctgagcact acaaggtggt ggtaaatgga aatcccttct    420
   atgagtacgg gcaccggctt cccctacaga tggtcaccca cctgcaagtg gatggggatc    480
   tgcaacttca atcaatcaac ttcatcggag gccagcccct ccggccccag ggacccccga     540
   tgatgccacc ttaccctggt cccggacatt gccatcaaca gctgaacagc ctgcccacca    600
   tggaaggacc cccaaccttc aacccgcctg tgccatattt cgggaggctg caaggagggc    660
   tcacagctcg aagaaccatc atcatcaagg gctatgtgcc tcccacaggc aagagctttg    720
   ctatcaactt caaggtgggc tcctcagggg acatagctct gcacattaat ccccgcatgg    780
   gcaacggtac cgtggtccgg aacagccttc tgaatggctc gtggggatcc gaggagaaga    840
   agatcaccca caacccattt ggtcccggac agttctttga tctgtccatt cgctgtggct    900
   tggatcgctt caaggtttac gccaatggcc agcacctctt tgactttgcc catcgcctct    960
   cggccttcca gagggtggac acattggaaa tccagggtga tgtcaccttg tcctatgtcc   1020
   agatctaatc tattcctggg gccataactc atgggaaaac agaattatcc cctaggactc   1080
   ctttctaagc ccctaataaa atgtctgagg gtgtctc                            1117
<210> 59
   <211> 2246
   <212> DNA
   <213> human
<400> 59
   gatccagcta tggagaaagc cgcagatctg caggacacag cctcgttaac tctgaagttt    60
   aagtttaacc caaagctggg cattgataat cctgtcctct ccctggccga agaccacgac   120
   ccctatgatc cctggagcct ggagcggcct cgcttctgtt tactgagcaa agaggagggc   180
   aagagttttg gcttccacct gcagcaggag ctgggcaggg ctgggcatgt ggtgtgcagg   240
   gtggacccag gcacctctgc ccagcgccag ggtcttcagg aaggagacag gatcctggcg   300
   gtgaacaatg atgttgtgga acacgaagac tatgcggtgg tggtacgccg catccgggcc   360
   agcagccctc gggtgttgct gacagtattg gcacggcatg cacatgacgt ggcccgagct   420
   cagctgggag aagatgccca cctctgtccc accctaggcc caggggtccg gccccggctg   480
   tgccacatag tgaaagatga gggtggtttt ggcttcagtg tcacccatgg caatcagggt   540
   cctttctggt tggtgctaag tactggagga gcagctgagc gggcaggggt gccccccggg  600
   gcccggctgc tggaagtgaa tgggctttgg cagagtggac agcaggtgac cttgctggtg   660
   gcagggccag aggtggaaga acagtgtcgc cagctgggat tgcccctggc tgcacccctg 720
   gcagagggct gggcactgcc caccaagccc cgctgcctgc acctggagaa agggccccag 780
   ggttttgggt tcctgctccg ggaggaaaag ggccttgacg gtcgccctgg acagttcctg 840
   tgggaggtgg acccgggact gccagccaag aaggctggga tgcaggctgg ggaccggctg 900
   gtggctgtgg ctggggagag cgtggagggg ctgggccatg aggagacagt gtccaggatc 960
   caggggcagg gctcctgtgt ctccctcact gtcgtcgacc ctgaggcgga ccgcttcttc 1020
   agcatggttc gcctgtcccc actcctcttc ttggagaaca cagaggctcc cgcctcgccc 1080
   cagggcagca gctcagcctc actggttgag acagaggacc cttcacttga agacacaagc 1140
   gtgccttctg tccctcttgg ctcccgacag tgcttcctgt accctgggcc tggtggcagc 1200
   tatggcttcc gactcagttg tgtggccagt gggcctcgtc tcttcatctc ccaggtgact 1260
   ccaggaggct cagctgcccg ggctgggctg caagtgggag acgtgattct ggaagtgaac 1320
   gggtatcctg ttgggggaca gaatgacctg gagaggcttc agcagctgcc tgaggctgag 1380
   ccacccctct gcctgaagct ggcagccagg tctctgcggg gcttggaagc ctggattccc 1440
   cctggggctg cagaggactg ggctctggcc tcggatctac tgtagagcac ccctgcttgg 1500
   tacagacata ctcaggggct accgtgtctt cactctccag cctgaggtgg tgaaggcagg 1560
   atgctctctc taagccagac cagagggact cagacaccac cgatcacagg ctggcccagg 1620
   tgctccctcc cttcctgcag gcccacctgc cagcagaggg tgtggttgga ggcctcagac 1680
   aggtccctga aggagtctga ggctccagag gatgtcatat gggagtttta gagagctgtg 1740
   tcccaaggat gaaggtgtgg ctgtgggtct ggctaggatt gaagccatct ggaccttttc 1800
   tagatatgac tccaggaccc ttgagtgtaa tgcaaaaatt tggagaccag ctatgcctgc 1860
   cctctgtggg tgccttagca ttgcgggagg gtggtgcttg gtcaccgttg catttgttat 1920
   agaaatggcc attcgccata aatctgactg cctgtgtttg tgttggtggg ggtaaggggc 1980
   agtggtgtga agggaccaaa agggcctcag gctcaagggg tgggatgcgg ctcctgcagg 2040
   agagaggttg agacctggtc aaatttattt cctatcaatc actgaatctc agggataatg 2100
   ggtcaaccca gaactgagat gtctgtatga cagccactcc taaaaataaa caacaacaaa 2160
   aacaaaaaaa gaagaaaact aaataaaaat aaaaataaaa ataaaaaaaa aaaaaaaaaa 2220
   aaaaaaaaaa aaaaaaaaaa aaaaaa 2246
<210> 60
   <211> 2418
   <212> DNA
   <213> human
<400> 60
   agtccagctt gggtccctga gagctgtgag aaggagatgc ggctgctgct ggccctgttg 60
   ggggtcctgc tgagtgtgcc tgggcctcca gtcttgtccc tggaggcctc tgaggaagtg 120
   gagcttgagc cctgcctggc tcccagcctg gagcagcaag agcaggagct gacagtagcc 180
   cttgggcagc ctgtgcggct gtgctgtggg cgggctgagc gtggtggcca ctggtacaag 240
   gagggcagtc gcctggcacc tgctggccgt gtacggggct ggaggggccg cctagagatt 300
   gccagcttcc tacctgagga tgctggccgc tacctctgcc tggcacgagg ctccatgatc 360
   gtcctgcaga atctcacctt gattacaggt gactcctcga cctccagcaa cgatgatgag 420
   gaccccaagt cccataggga cctctcgaat aggcacagtt acccccagca agcaccctac 480
   tggacacacc cccagcgcat ggagaagaaa ctgcatgcag tacctgcggg gaacaccgtc 540
   aagttccgct gtccagctgc aggcaacccc acgcccacca tccgctggct taaggatgga 600
   caggcctttc atggggggaa ccgcattgga ggcattcggc tgcgccatca gcactggagt 660
   ctcgtgatgg agagcgtggt gccctcggac cgcggcacat acacctgcct ggtagagaac 720
   gctgtgggca gcatccgtta taactacctg ctagatgtgc tggagcggtc cccgcaccgg 780
   cccatcctgc aggccgggct cccggccaac accacagccg tggtgggcag cgacgtggag 840
   ctgctgtgca aggtgtacag cgatgcccag ccccacatcc agtggctgaa gcacatcgtc 900
   atcaacggca gcagcttcgg agccgacggt ttcccctatg tgcaagtcct aaagactgca 960
   gacatcaata gctcagaggt ggaggtcctg tacctgcgga acgtgtcagc cgaggacgca 1020
   ggcgagtaca cctgcctcgc aggcaattcc atcggcctct cctaccagtc tgcctggctc 1080
   acggtgctgc caggtactgg gcgcatcccc cacctcacat gtgacagcct gactccagca 1140
   ggcagaacca agtctcccac tttgcagttc tccctggagt caggctcctc cggcaagtca 1200
   agctcatccc tggtacgagg cgtgcgtctc tcctccagcg gccccgcctt gctcgccggc 1260
   ctcgtgagtc tagatctacc tctcgaccca ctatgggagt tcccccggga caggctggtg 1320
   cttgggaagc ccctaggcga gggctgcttt ggccaggtag tacgtgcaga ggcctttggc 1380
   atggaccctg cccggcctga ccaagccagc actgtggccg tcaagatgct caaagacaac 1440
   gcctctgaca aggacctggc cgacctggtc tcggagatgg aggtgatgaa gctgatcggc 1500
   cgacacaaga acatcatcaa cctgcttggt gtctgcaccc aggaagggcc cctgtacgtg 1560
   atcgtggagt gcgccgccaa gggaaacctg cgggagttcc tgcgggcccg gcgcccccca 1620
   ggccccgacc tcagccccga cggtcctcgg agcagtgagg ggccgctctc cttcccagtc 1680
   ctggtctcct gcgcctacca ggtggcccga ggcatgcagt atctggagtc ccggaagtgt 1740
   atccaccggg acctggctgc ccgcaatgtg ctggtgactg aggacaatgt gatgaagatt 1800
   gctgactttg ggctggcccg cggcgtccac cacattgact actataagaa aaccagcaac 1860
   ggccgcctgc ctgtgaagtg gatggcgccc gaggccttgt ttgaccgggt gtacacacac 1920
   cagagtgacg tgtggtcttt tgggatcctg ctatgggaga tcttcaccct cgggggctcc 1980
   ccgtatcctg gcatcccggt ggaggagctg ttctcgctgc tgcgggaggg acatcggatg 2040
   gaccgacccc cacactgccc cccagagctg tacgggctga tgcgtgagtg ctggcacgca 2100
   gcgccctccc agaggcctac cttcaagcag ctggtggagg cgctggacaa ggtcctgctg 2160
   gccgtctctg aggagtacct cgacctccgc ctgaccttcg gaccctattc cccctctggt 2220
   ggggacgcca gcagcacctg ctcctccagc gattctgtct tcagccacga ccccctgcca 2280
   ttgggatcca gctccttccc cttcgggtct ggggtgcaga catgagcaag gctcaaggct 2340
   gtgcaggcac ataggctggt ggccttgggc cttggggctc agccacagcc tgacacagtg 2400
   ctcgaccttg atagcatg 2418
<210> 61
   <211> 1944
   <212> DNA
   <213> human
<400> 61
   ccctctcgcg ccccaggccg gtgtaccccc gcactccgcg ccccggccta gaagctctct      60
   ctccccgctc cccggcccgg cccccgcccc gccccgcccc agcccgctgg gccgccatgg     120
   agcgctggcc ttggccgtcg ggcggcgcct ggctgctcgt ggctgcccgc gcgctgctgc     180
   agctgctgcg ctcagacctg cgtctgggcc gcccgctgct ggcggcgctg gcgctgctgg     240
   ccgcgctcga ctggctgtgc cagcgcctgc tgcccccgcc ggccgcactc gccgtgctgg     300
   ccgccgccgg ctggatcgcg ttgtcccgcc tggcgcgccc gcagcgcctg ccggtggcca     360
   ctcgcgcggt gctcatcacc ggctgtgact ctggttttgg caaggagacg gccaagaaac     420
   tggactccat gggcttcacg gtgctggcca ccgtattgga gttgaacagc cccggtgcca     480
   tcgagctgcg tacctgctgc tcccctcgcc taaggctgct gcagatggac ctgaccaaac     540
   caggagacat tagccgcgtg ctagagttca ccaaggccca caccaccagc accggcctgt     600
   ggggcctcgt caacaacgca ggccacaatg aagtagttgc tgatgcggag ctgtctccag     660
   tggccacttt ccgtagctgc atggaggtga atttctttgg cgcgctcgag ctgaccaagg     720
   gcctcctgcc cctgctgcgc agctcaaggg gccgcatcgt gactgtgggg agcccagcgg     780
   gggacatgcc atatccgtgc ttgggggcct atggaacctc caaagcggcc gtggcgctac     840
   tcatggacac attcagctgt gaactccttc cctggggggt caaggtcagc atcatccagc     900
   ctggctgctt caagacagag tcagtgagaa acgtgggtca gtgggaaaag cgcaagcaat     960
   tgctgctggc caacctgcct caagagctgc tgcaggccta cggcaaggac tacatcgagc    1020
   acttgcatgg gcagttcctg cactcgctac gcctggccat gtccgacctc accccagttg   1080
   tagatgccat cacagatgcg ctgctggcag ctcggccccg ccgccgctat taccccggcc   1140
   agggcctggg gctcatgtac ttcatccact actacctgcc tgaaggcctg cggcgccgct   1200
   tcctgcaggc cttcttcatc agtcactgtc tgcctcgagc actgcagcct ggccagcctg   1260
   gcactacccc accacaggac gcagcccagg gcccaaacct gagccccggc ccttccccag   1320
   cagtggctcg gtgagccatg tgcacctatg gcccagccac tgcagcacag gaggctccgt   1380
   gagcccttgg ttcctccccg aaaaccccca gcattacgat cccccaagtg tcctggaccc   1440
   tggcctaaag aatcccaccc ccacttcatg cccactgccg atgcccaatc caggcccggt   1500
   gaggccaagg tttcccagtg agcctctgcg cctctccact gtttcatgag cccaaacacc   1560
   ctcctggcac aacgctctac cctgcagctt ggagaactcc gctggatggg gagtctcatg   1620
   caagacttca ctgcagcctt tcacaggact ctgcagatag tgcctctgca aactaaggag   1680
   tgactaggtg ggttggggac cccctcagga ttgtttctcg gcaccagtgc ctcagtgctg   1740
   caattgaggg ctaaatccca agtgtctctt gactggctca agaattaggg ccccaactac   1800
   acacccccaa gccacaggga agcatgtact gtacttccca attgccacat tttaaataaa   1860
   gacaaatttt tatttcttct aaaaaaaaaa aaaaaaaaaa aaaaaaaaaa aaaaaaaaaa   1920
   aaaaaaaaaa aaaaaaaaaa aaaa                                          1944
<210> 62
   <211> 661
   <212> DNA
   <213> human
<400> 62
   tttttttttt tttaaaatca atacaaatct tttattaaag atctactcat accatggctg 60
   aaatcatcta ttattgttgc tagttagcct ctcttctata gttgggtaat gttgtcttgc 120
   cactgtgttt gccatctctc ccaagtgaaa agaacacttt ttaaaaaaaa ttaattgctc 180
   caagttttca ggcccagggg aggctctccc attctcctcc ttcaataagt cccgtccagg 240
   aaagggtgat cttgtggata aattcatcat acttcacttt gccattgggt tcgatatctg 300
   cttccctgaa gagatcatcc acttccttgt gggtgagctt ctcccccaga ctcgtgagtt 360
   ttgaccgcag gtcggacgcc atgacgtaac ctttcttctc cttgtccacc atcaacatgg 420
   ctagaagaat ttctttcttt gggtcttctt gttttatttg catgtgcata atggtcagaa 480
   aagtggagaa atccagctct ccatttccgt ctatcccgtg ggtctgcagg tgccgctgca 540
   cctcccctgg cgttgggctg gcccccaggc acctcatggc caccatgagg tcgggggctt 600
   tatcttcccc ctctgctgct gtcatacaag gagaagcatt tcttgtctca ttaatttggt 660
   c 661
<210> 63
   <211> 532
   <212> DNA
   <213> human
<220>
   <221> misc_feature
   <222> (519)..(519)
   <223> N EQUALS ANY KIND OF BASE
<400> 63
   taactatgga aaaccatgtt tatttttaat aaaggatgac atttccaatc agtaaaatat 60
   cataaaagta taaaaatgta ctaagtacaa tcattagcat tatgttatag gggaatagtg 120
   gttataactt ttccctgtaa gatggcacat tggatggtca cagttggctt gatttacaga 180
   ggggcaagag taggtgacca gttgtaccag ttgctccagt ttcctaggat ttgggactct 240
   tgtaaaatga gaaagtccca ggcaaactgg gacggttggt cctacaagaa aaagagcagc 300
   atcagagtgt tggctatagt ttggaactta ggaacaggat cagacattat tttttaactt 360
   ctccacctat tttcccttta gctgtgaaat aaaaatccct tttgttatta ctgagggtgt 420
   tacagctttc agaggctttt ttaccactgg gtttcatgta attttgactt aatacctatg 480
   tcaagcctgg gaagaaaggc agttctaatc aacttgcang tgtggcattc tg 532
<210> 64
   <211> 1013
   <212> DNA
   <213> human
<400> 64
   atcattccta gaactgaagt tgaaaaggcc atcaggatgt cccggagccg tatcaatgat      60
   gctttccgtc tgaatgacaa cagcctagag tttctgggga tacagccaac acttggacct     120
   cctaaccagc cccctgtttc catatggctg attgtttttg gagttgtgat gggagtgata     180
   gtggttggca ttgtcatcct gatcttcact gggatcagag atcggaagaa gaaaaataaa     240
   gcaagaagtg gagaaaatcc ttatgcctcc atcgatatta gcaaaggaga aaataatcca     300
   ggattccaaa acactgatga tgttcagacc tccttttaga aaaatctatg tttttcctct     360
   tgaggtgatt ttgttgtatg taaatgttaa tttcatggta tagaaaatat aagatgataa     420
   agatatcatt aaatgtcaaa actatgactc tgttcagaaa aaaaattgtc caaagacaac     480
   atggccaagg agagagcatc ttcattgaca ttgctttcag tatttatttc tgtctctgga     540
   tttgacttct gttctgtttc ttaataagga ttttgtatta gagtatatta gggaaagtgt     600
   gtatttggtc tcacaggctg ttcagggata atctaaatgt aaatgtctgt tgaatttctg     660
   aagttgaaaa caaggatata tcattggagc aagtgttgga tcttgtatgg aatatggatg     720
   gatcacttgt agaggacatt gctttttcac ttccaaggtg cttgatcaac atctccctga     780
   caacacaaaa ctagagccag gggcctccgt gaactcccag agcatgcctg atagaaactc     840
   atttctactg ttctctaact gtggagtgaa tggaaattcc aactgtatgt tcaccctctg     900
   aagtgggtac ccagtctctt aaatcttttg tatttgctca cagtgtttga gcagtgctga     960
   gcacaaagca gacactcaat aaatgctaga tttacacaaa aaaaaaaaaa aaa           1013
<210> 65
   <211> 2060
   <212> DNA
   <213> human
<400> 65
   tgttcccagc actcaagcct tgccaccgcc gagccgggct tcctgggtgt ttcaggcaag 60
   gaagtctagg tccctggggg gtgaccccca aggaaaaggc agcctccctg cgcacccggt 120
   tgcccggagc cctctccagg gccggctggg ctgggggttg ccctggccag caggggcccg 180
   ggggcgatgc cacccggtgc cgactgaggc caccgcacca tggcccgctc gctgacctgg 240
   cgctgctgcc cctggtgcct gacggaggat gagaaggccg ccgcccgggt ggaccaggag 300
   atcaacagga tcctcttgga gcagaagaag caggaccgcg gggagctgaa gctgctgctt 360
   ttgggcccag gcgagagcgg gaagagcacc ttcatcaagc agatgcggat catccacggc 420
   gccggctact cggaggagga gcgcaagggc ttccggcccc tggtctacca gaacatcttc 480
   gtgtccatgc gggccatgat cgaggccatg gagcggctgc agattccatt cagcaggccc 540
   gagagcaagc accacgctag cctggtcatg agccaggacc cctataaagt gaccacgttt 600
   gagaagcgct acgctgcggc catgcagtgg ctgtggaggg atgccggcat ccgggcctgc 660
   tatgagcgtc ggcgggaatt ccacctgctc gattcagccg tgtactacct gtcccacctg 720
   gagcgcatca ccgaggaggg ctacgtcccc acagctcagg acgtgctccg cagccgcatg 780
   cccaccactg gcatcaacga gtactgcttc tccgtgcaga aaaccaacct gcggatcgtg 840
   gacgtcgggg gccagaagtc agagcgtaag aaatggatcc attgtttcga gaacgtgatc 900
   gccctcatct acctggcctc actgagtgaa tacgaccagt gcctggagga gaacaaccag 960
   gagaaccgca tgaaggagag cctcgcattg tttgggacta tcctggaact accctggttc 1020
   aaaagcacat ccgtcatcct ctttctcaac aaaaccgaca tcctggagga gaaaatcccc 1080
   acctcccacc tggctaccta tttccccagt ttccagggcc ctaagcagga tgctgaggca 1140
   gccaagaggt tcatcctgga catgtacacg aggatgtaca ccgggtgcgt ggacggcccc 1200
   gagggcagca agaagggcgc acgatcccga cgccttttca gccactacac atgtgccaca 1260
   gacacacaga acatccgcaa ggtcttcaag gacgtgcggg actcggtgct cgcccgctac 1320
   ctggacgaga tcaacctgct gtgacccagg ccccacctgg ggcaggcggc accggcgggc 1380
   gggtgggagg tgggagtggc tgcagggacc ctagtgtcct ggtctatctc tccagcctcg 1440
   gcccacacgc aagggagtcg ggggacggcc cgctgctggc cgctctcttc tctgcctctc 1500
   accaggacag ccgcccccca gggtactcct gcccttgctt gactcagttt ccctcctttg 1560
   aaagggaagg agcaaaacgg ccatttggga tgccagggtg gatgaaaagg tgaagaaatc 1620
   aggggattga gacttgggtg ggtgggcatc tctcaggagc cccatctccg ggcgtgtcac 1680
   ctcctgggca gggttctggg accctctgtg ggtgacgcac accctgggat ggggctagta 1740
   gagccttcag gcgccttcgg gcgtggactc tggcgcactc tagtggacag gagaaggaac 1800
   gccttccagg aacctgtgga ctaggggtgc agggacttcc ctttgcaagg ggtaacagac 1860
   cgctggaaaa cactgtcact ttcagagctc ggtggctcac agcgtgtcct gccccggttt 1920
   gcggacgaga gaaatcgcgg cccacaagca tcccccatcc cttgcaggct gggggctggg 1980
   catgctgcat cttaaccttt tgtatttatt ccctcacctt ctgcagggct ccgtgcgggc 2040
   tgaaattaaa gatttcttag 2060
<210> 66
   <211> 7265
   <212> DNA
   <213> human
<400> 66
   catagagcca gcgggcgcgg gcgggacggg cgccccgcgg ccggacccag ccagggcacc    60
   acgctgcccg gccctgcgcc gccaggcact tctttccggg gctcctaggg acgccagaag   120
   gaagtcaacc tctgctgctt ctccttggcc tgcgttggac cttccttttt ttgttgtttt   180
   tttttgtttt tcccctttct tccttttgaa ttaactggct tcttggctgg atgttttcaa   240
   cttctttcct ggctgcgaac ttttccccaa ttgttttcct tttacaacag ggggagaaag   300
   tgctctgtgg tccgaggcga gccgtgaagt tgcgtgtgcg tggcagtgtg cgtggcagga   360
   tgtgcgtgcg tgtgtaaccc gagccgcccg atctgtttcg atctgcgccg cggagccctc   420
   cctcaaggcc cgctccacct gctgcggtta cgcggcgctc gtgggtgttc gtgcctcgga   480
   gcagctaacc ggcgggtgct gggcgacggt ggaggagtat cgtctcgctg ctgcccgagt   540
   cagggctgag tcacccagct gatgtagaca gtggctgcct tccgaagagt gcgtgtttgc   600
   atgtgtgtga ctctgcggct gctcaactcc caacaaacca gaggaccagc cacaaactta   660
   accaacatcc ccaaacccga gttcacagat gtgggagagc tgtagaaccc tgagtgtcat   720
   cgactgggcc ttcttatgat tgttgtttta agattagctg aagatctctg aaacgctgaa 780
   ttttctgcac tgagcgtttt gacagaattc attgagagaa cagagaacat gacaagtact 840
   tctagctcag cactgctcca actactgaag ctgattttca aggctactta aaaaaatctg 900
   cagcgtacat taatggattt ctgttgtgtt taaattctcc acagattgta ttgtaaatat 960
   tttatgaagt agagcatatg tatatattta tatatacgtg cacatacatt agtagcacta 1020
   cctttggaag tctcagctct tgcttttcgg gactgaagcc agttttgcat gataaaagtg 1080
   gccttgttac gggagataat tgtgttctgt tgggacttta gacaaaactc acctgcaaaa 1140
   aactgacagg cattaactac tggaacttcc aaataatgtg tttgctgatc gttttactct 1200
   tcgcataaat attttaggaa gtgtatgaga attttgcctt caggaacttt tctaacagcc 1260
   aaagacagaa cttaacctct gcaagcaaga ttcgtggaag atagtctcca ctttttaatg 1320
   cactaagcaa tcggttgcta ggagcccatc ctgggtcaga ggccgatccg cagaaccaga 1380
   acgttttccc ctcctggact gttagtaact tagtctccct cctcccctaa ccacccccgc 1440
   ccccccccac cccccgcagt aataaaggcc cctgaacgtg tatgttggtc tcccgggagc 1500
   tgcttgctga agatccgcgc ccctgtcgcc gtctggtagg agctgtttgc agggtcctaa 1560
   ctcaatcggc ttgttgtgat gcgtatcccc gtagatgcca gcacgagccg ccgcttcacg 1620
   ccgccttcca ccgcgctgag cccaggcaag atgagcgagg cgttgccgct gggcgccccg 1680
   gacgccggcg ctgccctggc cggcaagctg aggagcggcg accgcagcat ggtggaggtg 1740
   ctggccgacc acccgggcga gctggtgcgc accgacagcc ccaacttcct ctgctccgtg 1800
   ctgcctacgc actggcgctg caacaagacc ctgcccatcg ctttcaaggt ggtggcccta 1860
   ggggatgttc cagatggcac tctggtcact gtgatggctg gcaatgatga aaactactcg 1920
   gctgagctga gaaatgctac cgcagccatg aagaaccagg ttgcaagatt taatgacctc 1980
   aggtttgtcg gtcgaagtgg aagagggaaa agcttcactc tgaccatcac tgtcttcaca 2040
   aacccaccgc aagtcgccac ctaccacaga gccatcaaaa tcacagtgga tgggccccga 2100
   gaacctcgaa gacatcggca gaaactagat gatcagacca agcccgggag cttgtccttt 2160
   tccgagcggc tcagtgaact ggagcagctg cggcgcacag ccatgagggt cagcccacac 2220
   cacccagccc ccacgcccaa ccctcgtgcc tccctgaacc actccactgc ctttaaccct 2280
   cagcctcaga gtcagatgca ggatacaagg cagatccaac catccccacc gtggtcctac 2340
   gatcagtcct accaatacct gggatccatt gcctctcctt ctgtgcaccc agcaacgccc 2400
   atttcacctg gacgtgccag cggcatgaca accctctctg cagaactttc cagtcgactc 2460
   tcaacggcac ccgacctgac agcgttcagc gacccgcgcc agttccccgc gctgccctcc 2520
   atctccgacc cccgcatgca ctatccaggc gccttcacct actccccgac gccggtcacc 2580
   tcgggcatcg gcatcggcat gtcggccatg ggctcggcca cgcgctacca cacctacctg   2640
   ccgccgccct accccggctc gtcgcaagcg cagggaggcc cgttccaagc cagctcgccc   2700
   tcctaccacc tgtactacgg cgcctcggcc ggctcctacc agttctccat ggtgggcggc   2760
   gagcgctcgc cgccgcgcat cctgccgccc tgcaccaacg cctccaccgg ctccgcgctg   2820
   ctcaacccca gcctcccgaa ccagagcgac gtggtggagg ccgagggcag ccacagcaac   2880
   tcccccacca acatggcgcc ctccgcgcgc ctggaggagg ccgtgtggag gccctactga   2940
   ggcgccaggc ctggcccggc tgggccacgc gggccgccgc cttcgcctcc gggcgcgcgg    3000
   gcctcctgtt cgcgacaagc ccgccgggat cccgggccct gggcccggcc accgtcctgg   3060
   ggccgagggc gcccgacggc caggatctcg ctgtaggtca ggcccgcgca gcctcctgcg   3120
   cccagaagcc cacgccgccg ccgtctgctg gcgccccggc cctcgcggag gtgtccgagg   3180
   cgacgcacct cgagggtgtc cgccggcccc agcacccagg ggacgcgctg gaaagcaaac   3240
   aggaagattc ccggagggaa actgtgaatg cttctgattt agcaatgctg tgaataaaaa   3300
   gaaagatttt atacccttga cttaactttt taaccaagtt gtttattcca aagagtgtgg    3360
   aattttggtt ggggtggggg gagaggaggg atgcaactcg ccctgtttgg catctaattc   3420
   ttatttttaa tttttccgca ccttatcaat tgcaaaatgc gtatttgcat ttgggtggtt    3480
   tttattttta tatacgttta tataaatata tataaattga gcttgcttct ttcttgcttt   3540
   gaccatggaa agaaatatga ttcccttttc tttaagtttt atttaacttt tcttttggac    3600
   ttttgggtag ttgttttttt ttgttttgtt ttgttttttt gagaaacagc tacagctttg     3660
   ggtcattttt aactactgta ttcccacaag gaatccccag atatttatgt atcttgatgt   3720
   tcagacattt atgtgttgat aattttttaa ttatttaaat gtacttatat taagaaaaat   3780
   atcaagtact acattttctt ttgttcttga tagtagccaa agttaaatgt atcacattga   3840
   agaaggctag aaaaaaagaa tgagtaatgt gatcgcttgg ttatccagaa gtattgttta   3900
   cattaaactc cctttcatgt taatcaaaca agtgagtagc tcacgcagca acgtttttaa   3960
   taggattttt agacactgag ggtcactcca aggatcagaa gtatggaatt ttctgccagg   4020
   ctcaacaagg gtctcatatc taacttcctc cttaaaacag agaaggtcaa tctagttcca   4080
   gagggttgag gcgggtgcca ataattacat ctttggagag gatttgattt ctgcccaggg   4140
   atttgctcac cccaaggtca tctgataatt tcacagatgc tgtgtaacag aacacagcca   4200
   aagtaaactg tgtaggggag ccacatttac ataggaacca aatcaatgaa tttaggggtt   4260
   acgattatag caatttaagg gccaccagaa gcaggcctcg aggagtcaat ttgcctctgt   4320
   gtgcctcagt ggagacaagt gggaaaacat ggtcccacct gtgcgagacc ccctgtcctg   4380
   tgctgctcac tcaacaacat ctttgtgttg ctttcaccag gctgagaccc taccctatgg 4440
   ggtatatggg cttttacctg tgcaccagtg tgacaggaaa gattcatgtc actactgtcc 4500
   gtggctacaa ttcaaaggta tccaatgtcg ctgtaaattt tatggcacta tttttattgg 4560
   aggatttggt cagaatgcag ttgttgtaca actcataaat actaactgct gattttgaca 4620
   catgtgtgct ccaaatgatc tggtggttat ttaacgtacc tcttaaaatt cgttgaaacg 4680
   atttcaggtc aactctgaag agtatttgaa agcaggactt cagaacagtg tttgattttt 4740
   attttataaa tttaagcatt caaattaggc aaatctttgg ctgcaggcag caaaaacagc 4800
   tggacttatt taaaacaact tgtttttgag ttttcttata tatatattga ttatttgttt 4860
   tacacacatg cagtagcact ttggtaagag ttaaagagta aagcagctta tgttgtcagg 4920
   tcgttcttat ctagagaaga gctatagcag atctcggaca aactcagaat atattcactt 4980
   tcatttttga caggattccc tccacaactc agtttcatat attattccgt attacatttt 5040
   tgcagctaaa ttaccataaa atgtcagcaa atgtaaaaat ttaatttctg aaaagcacca 5100
   ttagcccatt tcccccaaat taaacgtaaa tgtttttttt cagcacatgt taccatgtct 5160
   gacctgcaaa aatgctggag aaaaatgaag gaaaaaatta tgtttttcag tttaattctg 5220
   ttaactgaag atattccaac tcaaaaccag cctcatgctc tgattagata atcttttaca 5280
   ttgaaccttt actctcaaag ccatgtgtgg agggggcttg tcactattgt aggctcactg 5340
   gattggtcat ttagagtttc acagactctt accagcatat atagtattta attgtttcaa 5400
   aaaaaatcaa actgtagttg ttttggcgat aggtctcacg caacacattt ttgtatgtgt 5460
   gtgtgtgtgc gtgtgtgtgt gtgtgtgtga aaaattgcat tcattgactt caggtagatt 5520
   aaggtatctt tttattcatt gccctcagga aagttaaggt atcaatgaga cccttaagcc 5580
   aatcatgtaa taactgcatg tgtctggtcc aggagaagta ttgaataagc catttctact 5640
   gcttactcat gtccctattt atgatttcaa catggataca tatttcagtt ctttcttttt 5700
   ctcactatct gaaaatacat ttccctccct ctcttccccc caatatctcc cttttttcct 5760
   ctcttcctct atcttccaaa ccccactttc tccctcctcc ttttcctgtg ttctcttaag 5820
   cagatagcac atacccccac ccagtaccaa atttcagaac acaagaaggt ccagttcttc 5880
   ccccttcaca taaaggaaca tggtttgtca gcctttctcc tgtttatggg tttcttccag 5940
   cagaacagag acattgccaa ccatattgga tctgcttgct gtccaaacca gcaaacttcc 6000
   tgggcaaatc acaatcagtg agtaaataga cagcctttct gctgccttgg gtttctgtgc 6060
   agataaacag aaatgctctg attagaaagg aaatgaatgg ttccactcaa atgtcctgca 6120
   atttaggatt gcagatttct gccttgaaat acctgtttct ttgggacatt ccgtcctgat 6180
   gatttttatt tttgttggtt tttatttttg gggggaatga catgtttggg tcttttatac 6240
   atgaaaattt gtttgacaat aatctcacaa aacatatttt acatctgaac aaaatgcctt 6300
   tttgtttacc gtagcgtata catttgtttt gggatttttg tgtgtttgtt gggaattttg 6360
   tttttagcca ggtcagtatt gatgaggctg atcatttggc tctttttttc cttccagaag 6420
   agttgcatca acaaagttaa ttgtatttat gtatgtaaat agattttaag cttcattata 6480
   aaatattgtt aatgcctata actttttttc aatttttttg tgtgtgtttc taaggacttt 6540
   ttcttaggtt tgctaaatac tgtagggaaa aaatgcttct ttctaacttt gtttatttta 6600
   gactttaaaa tgagctactt cttattcact tttgtaaaca gctaatagca tggttccaat 6660
   tttttttaag ttcacttttt ttgttctagg ggaaatgaat gtgcaaaaaa agaaaaagaa 6720
   ctgttggtta tttgtgttat tctggatgta taaaaatcaa tggaaaaaaa taaactttca 6780
   aattgaaatg acggtataac acatctactg aaaaagcaac gggaaatgtg gtcctattta 6840
   agccagcccc cacctagggt ctatttgtgt ggcagttatt gggtttggtc acaaaacatc 6900
   ctgaaaattc gtgcgtgggc ttctttctcc ctggtacaaa cgtatggaat gcttcttaaa 6960
   ggggaactgt caagctggtg tcttcagcca gatgacatga gagaatatcc cagaaccctc 7020
   tctccaaggt gtttctagat agcacaggag agcaggcact gcactgtcca cagtccacgg 7080
   tacacagtcg ggtgggccgc ctcccctctc ctgggagcat tcgtcgtgcc cagcctgagc 7140
   agggcagctg gactgctgct gttcaggagc caccagagcc ttcctctctt tgtaccacag 7200
   tttcttctgt aaatccagtg ttacaatcag tgtgaatggc aaataaacag tttgacaagt 7260
   acata 7265
<210> 67
   <211> 4221
   <212> DNA
   <213> human
<400> 67
   gtcggccgtc ccctttaatt tttaaataca cggtcccctc ttttctctgg ggggggcaag      60
   caagaaatca aagaaggagg agacaagccg tcaattttct ccaaaacaaa ccccaccggg    120
   caatttggtc tcggggtagg gggagacggg gtgattgcaa attattccag gacgagatcc    180
   agttctccag cgggaaaggg gcaaaggaac gccgcgcgtt ggaagggcca gggtacgcag    240
   ctccccttgc agcgcccgca ggacccccgc aagctcgtgc cggcgaaatc ggagaccgcc    300
   gatctgtcct cgttctctcc tgcacgtctg gctgcattcg gaggaagacc tggggcgcga    360
   gcgagcggcg acagcatgag cctgtgctga cctccgcgcg gcgggccgag cccagggctt    420
   tgtcgcggta cctgcgccca gcccgcgccg caactctgtg cccagctttt gcaatctttt    480
   gttgcagcgc tgaccgcacc aagttaaatg ctcccttgca atttttcttt tttttgtttg    540
   tttgtttaat ttttggagag ctcgcgatct tggaaaagcc tcagacgcca tctacagtta    600
   aaacgtaggt aactgccctc tcccgcaccc cccccttaca cgccccccac cctttccacc    660
   aaaaaaaggg ggtgcagcgc ggattctggc tgccgtgcgt cgccagccgg tagacccgtg    720
   cttgtttcct ttctcttttt gtttggcttc taacgcgttg ggactgagtc gccgccgtga    780
   gctccccgaa gactgcacaa actaccgcgg gctcctccgc cccgtctgcg attcggaagc    840
   cggcctgggg gtcgcgtcgg gagccctgcg ctgcagctcc gcaccttagc agcccgggta    900
   ctcatccaga tccacgccgg ggacacacac acagagtaac taaaagtgcg gcgattctgc    960
   acatcgccga ctgctttggg gtaacaaaaa gacccgagtt gcctgccgac cgaggacccc   1020
   cgggagccgg gctcggagca gacgaggtat ccggcggcgc ccatttgggg gcttctaact   1080
   ctttctccac gcagcccctc ttctgtcccc tcccctctcg ctccctttta aaatcagtgg   1140
   caccgaggcg cctgcagccg cactcgccag cgactcatct ctccagcggg tttttttttg   1200
   tttgtcgtgt gcgatcctca cactcatgaa catacacagg tctaccccca tcacaatagc   1260
   gagatatggg agatcgcgga acaaaaccca ggatttcgaa gagttgtcgt ctataaggtc   1320
   cgcggagccc agccagagtt tcagcccgaa cctcggctcc ccgagcccgc ccgagactcc   1380
   gaacttgtcg cattgcgttt cttgtatcgg gaaatactta ttgttggaac ctctggaggg   1440
   agaccacgtt tttcgtgccg tgcatctgca cagcggagag gagctggtgt gcaaggtgtt   1500
   tgatatcagc tgctaccagg aatccctggc accgtgcttt tgcctgtctg ctcatagtaa   1560
   catcaaccaa atcactgaaa ttatcctggg tgagaccaaa gcctatgtgt tctttgagcg   1620
   aagctatggg gacatgcatt ccttcgtccg cacctgcaag aagctgagag aggaggaggc   1680
   agccagactg ttctaccaga ttgcctcggc agtggcccac tgccatgacg gggggctggt   1740
   gctgcgggac ctcaagctgc ggaaattcat ctttaaggac gaagagagga ctcgggtcaa   1800
   gctggaaagc ctggaagacg cctacattct gcggggagat gatgattccc tctccgacaa   1860
   gcatggctgc ccggcttacg taagcccaga gatcttgaac accagtggca gctactcggg   1920
   caaagcagcc gacgtgtgga gcctgggggt gatgctgtac accatgttgg tggggcggta   1980
   ccctttccat gacattgaac ccagctccct cttcagcaag atccggcgtg gccagttcaa   2040
   cattccagag actctgtcgc ccaaggccaa gtgcctcatc cgaagcattc tgcgtcggga   2100
   gccctcagag cggctgacct cgcaggaaat tctggaccat ccttggtttt ctacagattt   2160
   tagcgtctcg aattcagcat atggtgctaa ggaagtgtct gaccagctgg tgccggacgt   2220
   caacatggaa gagaacttgg accctttctt taactgagct catgccccac ggagacttag   2280
   caggttccag gagtgagcga gggcagcgga aaggagttct tccgggggac acgaattgcc   2340
   tggctgagta gcaagaaaga cacactctta agtttcttgg ttcagagcag gaaaaccttc 2400
   aaggagctga ctgaccacgt agcatggggg caagaggcgt gggatgggga ttggggtgag   2460
   atggatggga gcccgctgga gcttgtcttc cctaacatag cctgggagac caccccttgc   2520
   cacttgggcc acttccgcct accccacttt tcattttgtt ccaaaatagt tgcagatcct 2580
   gacagaatca aaactctctg cctcaaacac acatcctggc atcgcactgt tagcatttaa 2640
   cttcttgtta ggattcaggg aaggaacagt tggccaagaa ttttttttct tttaaacaag 2700
   ccaaccacct agctggtaat taatgaggtt cacttaaaaa aaaaattcgg tgcacacaga 2760
   ctgacatgaa acctgggtgc tacagtaaaa gaaaacaaaa gtccagtttg tgtctcttaa 2820
   tcgctcactt caactcattt cttctaaata aactatttaa tatcctggtc aggaaatgac 2880
   atgttaatgc tttgctccct gaagggggaa aaaatctgtc ctttaacaag ctattctgtt 2940
   ttgtgtcaat tgggtccgtg gcaaggaagc tattaggaag tcaaacggtc caggatgcat 3000
   tacctgctaa tccttaggtt taaaggggga aagaaaaggg aagaagaaag gaaaagagaa 3060
   atccaactcc tttttcatgt tttgcttttg aacaatgagg gtttgtgtga caggcattcc 3120
   tctttgctga gatgatagca atggcctgag attttagcaa gctcctggag tctgatgctt 3180
   ttgcagtact ctgatcgcaa ctaaacattt gtctttgttt tattagaaac tagtgaaaca 3240
   aagcaggttg tcccacatgt ataaaataca gggcagctat ttagttttct ttacagagaa 3300
   tgatcctttt aaggcttgta aggccctctg gtttggacaa aaaccctcag tagagacaag 3360
   cgggaaggat aattagctga aagctatgat gatataaata aaaacagctc tctatcccaa 3420
   tacgcacctt tgtattttca agaactcttc tatttattaa ggaaaatgtc acattgtgat 3480
   gtattaagcc agtacttcaa ttacgggttg acttgggatg acatattaca tgctgtagtt 3540
   aacatttata attctttttc cttgtttgag tatttctgtc tctgaaataa ccttttactt 3600
   ggcttttcta gatagcttta tttgatttcg agtggcaaaa tgttttttat tacggctttt 3660
   ctattgctgt atgatacaga actcttttgg cataaatatt tgtgttccca gtacctcact 3720
   tgttcggatt tgactgcctg tatatgtttt gtgaaatggt cctgtttttg ggtaggtgac 3780
   acgtggactc tagtatgtaa atgttacttg aatctgtgct tcataatagt gtgtggcatg 3840
   tatgtgcaga ctcttggatg ctttatgcct gcgcaccagg agccctgtcc tcacgttccc 3900
   aggagggcgg cttcaccctt cgtaaccagg agacaaggcg gccatggatt tgcccttgat 3960
   tctattttgc taatggaaga tagaaaggag agaaggtttt tttttttttt taacattctg 4020
   aagatggtgc tgtgtcaaga aggacctttt ttttcccctc tcccctattt tttaagtacc 4080
   ttggaggagg agaggttggt gacatgcatg gtggggatct atggcctctg gtgctttgtc 4140
   ctgtatttgg tttaatgttt ttgtcctaat ctcttcaatc aataaaattg tgcgtattta 4200
   actaaaaaaa aaaaaaaaaa a 4221
<210> 68
   <211> 524
   <212> DNA
   <213> human
<400> 68
   ccctgccttc caccttggaa gaggaggctg gacgcatcag cagtggccag gcaggtcgca 60
   aaatctccca gcctagagac cacacctgaa acggctgaag ccagcttgca caagggctgc 120
   tgtccctctg cggcaggcag agctggtggg ggcaggggtc acagagcagt catagacacc 180
   atggaccagg caggagaagg gcagatggca catgggcaca acagggcctt gtccttagag 240
   cactgggggg tcatggctgg gaggggcatg gcaggggctg gcatccctgt agagccagag 300
   gggccaccca ggcagtgaca ttccagatat gttgggctca cctcatcctt gctgtgagac 360
   tggagttcca tggggacatg aagtcagtac accgcagagc tgctcagctg ctctacctct 420
   cgctgacttt tttgttgcac atatacattt tctttcaatt agcatttatt tcagctttta 480
   tttaagcttt ttgacagtac atgtaaatat atgattataa ccat 524
<210> 69
   <211> 4151
   <212> DNA
   <213> human
<400> 69
   gggaatagca gaataggagc aagccagcac tagtcagcta actaagtgac tcaaccaagg 60
   ccttttttcc ttgttatctt tgcagatact tcattttctt agcgtttctg gagattacaa 120
   catcctgcgg ttccgtttct gggaacttta ctgatttatc tcccccctca cacaaataag 180
   cattgattcc tgcatttctg aagatctcaa gatctggact actgttgaaa aaatttccag 240
   tgaggctcac ttatgtctgt aaagatggga aaaaaataca agaacattgt tctactaaaa 300
   ggattagagg tcatcaatga ttatcatttt agaatggtta agtccttact gagcaacgat 360
   ttaaaactta atttaaaaat gagagaagag tatgacaaaa ttcagattgc tgacttgatg 420
   gaagaaaagt tccgaggtga tgctggtttg ggcaaactaa taaaaatttt cgaagatata 480
   ccaacgcttg aagacctggc tgaaactctt aaaaaagaaa agttaaaagt aaaaggacca 540
   gccctatcaa gaaagaggaa gaaggaagtg catgctactt cacctgcacc ctccacaagc 600
   agcactgtca aaactgaagg agcagaggca actcctggag ctcagaaaag aaaaaaatca 660
   accaaagaaa aggctggacc caaagggagt aaggtgtccg aggaacagac tcagcctccc 720
   tctcctgcag gagccggcat gtccacagcc atgggccgtt ccccatctcc caagacctca 780
   ttgtcagctc cacccaacag ttcttcaact gagaacccga aaacagtggc caaatgtcag 840
   gtaactccca gaagaaatgt tctccaaaaa cgcccagtga tagtgaaggt actgagtaca 900
   acaaagccat ttgaatatga gaccccagaa atggagaaaa aaataatgtt tcatgctaca 960
   gtggctacac agacacagtt cttccatgtg aaggttttaa acaccagctt gaaggagaaa 1020
   ttcaatggaa agaaaatcat catcatatca gattatttgg aatatgatag tctcctagag 1080
   gtcaatgaag aatctactgt atctgaagct ggtcctaacc aaacgtttga ggttccaaat 1140
   aaaatcatca acagagcaaa ggaaactctg aagattgata ttcttcacaa acaagcttca 1200
   ggaaatattg tatatggggt atttatgcta cataagaaaa cagtaaatca gaagaccaca 1260
   atctacgaaa ttcaggatga tagaggaaaa atggatgtag tggggacagg acaatgtcac 1320
   aatatcccct gtgaagaagg agataagctc cagcttttct gctttcgact tagaaaaaag 1380
   aaccagatgt caaaactgat ttcagaaatg catagtttta tccagataaa gaaaaaaaca 1440
   aacccgagaa acaatgaccc caagagcatg aagctacccc aggaacagcg tcagcttcca 1500
   tatccttcag aggccagcac aaccttccct gagagccatc ttcggactcc tcagatgcca 1560
   ccaacaactc catccagcag tttcttcacc aagaaaagtg aagacacaat ctccaaaatg 1620
   aatgacttca tgaggatgca gatactgaag gaagggagtc attttccagg accgttcatg 1680
   accagcatag gcccagctga gagccatccc cacactcctc agatgcctcc atcaacacca 1740
   agcagcagtt tcttaaccac gttgaaacca agactgaaga ctgaacctga agaagtttcc 1800
   atagaagaca gtgcccagag tgacctcaaa gaagtgatgg tgctgaacgc aacagaatca 1860
   tttgtatatg agcccaaaga gcagaagaaa atgtttcatg ccacagtggc aactgagaat 1920
   gaagtcttcc gagtgaaggt ttttaatatt gacctaaagg agaagttcac cccaaagaag 1980
   atcattgcca tagcaaatta tgtttgccgc aatgggttcc tggaggtata tcctttcaca 2040
   cttgtggctg atgtgaatgc tgaccgaaac atggagatcc caaaaggatt gattagaagt 2100
   gccagcgtaa ctcctaaaat caatcagctt tgctcacaaa ctaaaggaag ttttgtgaat 2160
   ggggtgtttg aggtacataa ggtaagccca caccattgtt ttataaaatt tctcctgcaa 2220
   cctccaattt ttaaagtctt aacttgtcaa ctggagtttg gtcaacttac tcaacacaga 2280
   aaatcaaccc cttcaccctt cccccagcac tagagataat tgaatagagt tcatttcagg 2340
   atatggggta cgttatattg taacattcct cttcttaagg tatcatcatg caagttattt 2400
   agacagtcac taggaaactt ggcattttat tagttttgat gatctattca gagccaccct 2460
   tgtccaggac agtgcagagt ttatatcaac acacatatcc ttaggatttt gtttctttga 2520
   gttcttctcc atctgtatca atgacaactt aatttaattg tgaataaaag agttgctctc 2580
   ccaagcctga atcctgattg tgacaaccag agtaagaaat aaaatagact actctgcttt 2640
   agaatgcagc tatgtctaac agttagctag aattctgatc atttggactc caaagtttct 2700
   tgcctcttct cattcattaa ttcatcagga gactgtagag caactaactt ctgcattaaa 2760
   taataagaga aatacgaagc aaaaagacta aaaaagtcac gtagcttaac tgctcaattt 2820
   ataaatgggg caataaaatg caaaaaaaaa gaaaaaaagc ttggtgaatt cttaggctta 2880
   cagtgtgcct ttcagtctct acacatcatg taaatattat gcttagctga tttaacttct 2940
   tgtttgaagt actgtttcat actccattat acatgtcttc tagggtggct tacttttaat 3000
   tgtgctgttt tctctacact cagtttaaat gactgtacat atatatgtgg ttggagagtt 3060
   aatgaataat gagctacaaa ccagaacaat gtgactagat agataggatg atctagaatt 3120
   gagaactggc agattgggaa aagagtggct atatggagaa agaaagaaag tagttccata 3180
   ttgaaataac agtctactta atgaggaccg ttgcaacatt ctttctcaaa cttacaaagt 3240
   gccataaaaa gcctctattc tctgctcttg ggcaggtgtg aaagaaacct accaaattaa 3300
   tcagattttt ctgtatccag gctccttaaa aaatcccagc tgtgctgatg tggaaacagg 3360
   aagaattagg aaagtaatca attttttttc ctagaaaaaa tccagcagac aaagaacttc 3420
   aacaaaagag gctcaaggga ggagttgaaa ggcaggattc aaagaccaag tatcttaagc 3480
   tatttggtac ctgttattca ggacctacag ctctgtttac tctatcaaag accaaaagtt 3540
   tccagaaaca ccctgtattt ctcatagatt tgaaaattat tgatccagtt tcagaagata 3600
   agtgttaatt ttcttttgca gaaaaatgta aggggtgaat tcacttatta tgaaatacaa 3660
   gataatacag ggaagatgga agtggtggtg catggacgac tgaccacaat caactgtgag 3720
   gaaggagata aactgaaact cacctgcttt gaattggcac cgaaaagtgg gaataccggg 3780
   gagttgagat ctgtaattca tagtcacatc aaggtcatca agaccaggaa aaacaagaaa 3840
   gacatactca atcctgattc caagtatgga aacttcacca gactttttct tctaaaatct 3900
   ggatgtcatt gacgataatg tttatggaga taaggtctaa gtgcctaaaa aaatgtacat 3960
   atacctggtt gaaatacaac actatacata cacaccacca tatatactag ctgttaatcc 4020
   tatggaatgg ggtattggga gtgctttttt aatttttcat agtttttttt taataaaatg 4080
   gcatattttg catctacaac ttctataatt tgaaaaaata aataaacatt atcttttttg 4140
   tgaaaaaaaa a 4151
<210> 70
   <211> 741
   <212> DNA
   <213> human
<220>
   <221> misc_feature
   <222> (492)..(492)
   <223> N EQUALS ANY KIND BASE
<220>
   <221> misc_feature
   <222> (652) ... (652)
   <223> N EQUALS ANY KIND BASE
<220>
   <221> misc_feature
   <222> (696)..(696)
   <223> N EQUALS ANY KIND BASE
<220>
   <221> misc_feature
   <222> (707)..(707)
   <223> N EQUALS ANY KIND BASE
<400> 70
   tttgcttttg tttcttctgg tcccatcact gggacctaaa agaagctcac atgtggtctc     60
   tggaaatgct gaagctgtgg actgtagaca ttattttcag tccttgtcct ggtggctgca    120
   taccagatgc tgctccttcc ctgtgtgtgg ccagctgtac acagtgacat gctcccaagg    180
   ccgcggcaca ggcggtgatg ggaactcctc cccgggccag cctctcaggc tgcagcccca    240
   cggcaccctg aggccctcat ctctgctcgg cagctaaaac atctccttct tcgatgctct    300
   gcaactgcag cctctggctc acaagagttc tgctgcctcg gcggcccccg aagccgcccc    360
   ccgggacagt ccgtgctgta accaagaccc ctggcaaagc ctcctcccca aataagttga    420
   ttttggcttc ggcctcaatg gctttggcca agcttgctgc gtaactgtcc aaggacttgt    480
   gtacttcagc tntcacatga agaatcgagt tcttggcagc ttctttgatt tgttccactt    540
   catcctcaaa tgtcaccgag ctccttctct ttggtgggca agtgcagtga gaagggaggg    600
   tgtcatcaga cgtgtgtcct caagcagcat catatctgtc cctgagtcct gngagcagct    660
   gaaaccagtg tcccccgtgc tgctgctgtg ccccangggc tgtccgnctg ccatgagcgc    720
   aatgagtgtg ggacaggccc c                                              741
<210> 71
   <211> 755
   <212> DNA
   <213> human
<220>
   <221> misc_feature
   <222> (643)..(643)
   <223> N EQUALS ANY KIND BASE
<220>
   <221> misc_feature
   <222> (741)..(741)
   <223> N EQUALS ANY KIND BASE
<400> 71
   tggtagtgaa tactttattt tgttgtaaac aagttagttt tgagggtatt tcctcgtggt      60
   cctcctgccg tcactcgtcc ccatgttcca atgatgctga tcaactgctt tattcagttt     120
   cccatctttc ttcttgccca ggcatcgtag cctttctttt tttaaacaca tgatccctag     180
   tactcatctt tggaggacaa aaggctttcc atatgttaga aaaatttgaa tctcatagta     240
   ctcacaacaa tgagcagcat tgtaagttgt gatgcattca tttggattgg aacattctca     300
   atcagtcctt ccactctaag taaatatttg tttctcacag aacacaaggc agttcaaaag     360
   gcctcttggt taaggaatta tagggtgttg aatgggaaac atcatacaag cagtgaaaac     420
   aaaaatcttt ccaggttgtc ggattttctc cttcttggtc ttataaaaag caactagaca     480
   tctctaattt aaaaaataca tgcacatata tacaatagtg attggaatgt attcttatcc     540
   aaaacattat agagtttatc tcagatatac tgagtactgt cactcagtct gtaaattacc     600
   cccaagaagg tgggttgttt cctcattcct taaataaaaa cangatcgaa taacagacca     660
   aaaagaagtt actaaatttt aacactgaca tccttgtgaa gagccagtct ttacaggcgt     720
   ttgtaaagta gactgtgggg nagtgtacac taata                                755
<210> 72
   <211> 1894
   <212> DNA
   <213> human
<400> 72
   aggactcggg ccggagcgtg gccggacccc cacccgccga ggggcccagg gaggacgcgg      60
   cagagtcacg gtggcagcat tgagagttgg acacccgggt ccttgaagtg atctctaggc     120
   cccagcccca aatccgccac cattccgtgc tgcggggaca ccatggctcc agaagaggac     180
   gctggagggg aggccttagg gggcagtttc tgggaggctg gcaactacag gcgcacggta     240
   cagcgggtgg aggacgggca ccggctgtgc ggggacctgg tcagctgctt ccaggagcgc     300
   gcccgcatcg agaaggctta tgcccagcag ttggctgact gggcccgaaa gtggaggggg     360
   accgtggaga agggccccca gtatggcaca ctggagaagg cctggcatgc ctttttcacg     420
   gcggctgagc ggctgagcgc gctgcacctg gaggtgcggg agaagctgca agggcaggac     480
   agtgagcggg tgcgcgcctg gcagcggggg gctttccacc ggcctgtgct gggcggcttc     540
   cgcgagagcc gggcggccga ggacggcttc cgcaaggccc agaagccctg gctgaagagg     600
   ctgaaggagg ttgaggcttc caagaaaagc taccacgcag cccggaagga tgagaagacc     660
   gcccagacga gggagagcca cgcaaaggca gacagcgccg tctcccagga gcagctgcgc 720
   aaactgcagg aacgggtgga acgctgtgcc aaggaggccg agaagacaaa agctcagtat 780
   gagcagacgc tggcagagct gcatcgctac actccacgct acatggagga catggaacag 840
   gcctttgaga cctgccaggc cgccgagcgc cagcggcttc ttttcttcaa ggatatgctg 900
   ctcaccttac accagcacct ggacctttcc agcagtgaga agttccatga actccaccgt 960
   gacttgcacc agggcattga ggcagccagt gacgaagagg atctgcgctg gtggcgcagc 1020
   acccacgggc caggcatggc catgaactgg ccacagttcg aggagtggtc cttggacaca 1080
   cagaggacaa tcagccggaa agagaagggt ggccggagcc ctgatgaggt taccctgacc 1140
   agcattgtgc ctacaagaga tggcaccgca cccccacccc agtccccggg gtccccaggc 1200
   acggggcagg atgaggagtg gtcagatgaa gagagtcccc ggaaggctgc caccggggtt 1260
   cgggtgaggg cactctatga ctacgctggc caggaagctg atgagctgag cttccgagca 1320
   ggggaggagc tgctgaagat gagtgaggag gacgagcagg gctggtgcca aggccagttg 1380
   cagagtggcc gcattggcct gtaccctgcc aactacgtgg agtgtgtggg cgcctgagtg 1440
   tcctgacagc ccttctgcaa cgtttaccca ccctggttca gagcccagct tctcctggag 1500
   agccggaccc tcagggccct gaaccgtcgc tctctggctg ctcctctgtc ccttgaggga 1560
   ggaagtcctg ggacccaggg aggggagggg cctttgtcta gggaagggac tggtagggaa 1620
   gggacgagtc taggctgagg gcaagatggg aggtcagagg tgacagaagc gttcaggggt 1680
   gcctgggcct ccccaggagc tgtggactca gttcctgacc tctgctttgg ggttcctggg 1740
   gtgggcttgg ggtgagtgta gttctggcct agcagcaccc tcttgtggct tgttctagcg 1800
   tgtattaaaa cttgacacac acccacacac aaaaccaaaa aaaaaaaaaa aaaaaaaaaa 1860
   aaaaaaaaaa aaaaaaaaaa aaaaaaaaaa aaaa 1894
<210> 73
   <211> 649
   <212> DNA
   <213> human
<400> 73
   ggcgaggcgt ctcggagtct cagagacacc aaggcccctg cgacaaggtg gctgcagcta 60
   ggccgggggc gtcaggacga cggagcgggt tcgggtcggt gacacgcaga cctgagggag 120
   ctgggcccgc cttttccgcc cgcgccccag gcccttgcag atcgagattt gcgtcctaga 180
   gtgggaaaaa agcagaggcc agggcgccga ttttatttgg agagaagcaa gcatctttgc 240
   ctctttggag taggaaattc agacttgaaa aagtggtgtg tggttgactc tgtttctcgc 300
   catgtcttct cacaagactt tcaccattaa gcgattcctg gccaagaaac aaaagcaaaa 360
   tcgtcccatc ccccagtgga ttcagatgaa acctggtagt aaaatcaggt acaactccaa     420
   aaggaggcat tggagaagaa ccaagctggg tctataagga attgcacatg agatggcaca     480
   catatttatg ctgtatcaag ttcacgatca tcttacgata tcaagctgaa aatgtcacca     540
   ctacctggac agttgcacat gttttactgg gaatattttt ttctgttttt ctgtatgctc     600
   tgtgctagta gggtggattc agtaataaat atgtgaaagc ttttgtttc                 649
<210> 74
   <211> 1561
   <212> DNA
   <213> human
<400> 74
   gcggcgcgga gggcgcgggc ccgggagcca gggagcgagc ggggcgcccg gcagcgcgga       60
   gtcagcgccg cgggggccgc acccgactcg cgcctggaca ctcgcggggc gccgacctgg      120
   cagggggcca aaccagtgct cctgccacct ctctggctgc cccctagagc ctgcccatcc     180
   cagcctgacc aatgtccaca gccagggagc agccaatctt cagcacacgg gcgcacgtgt      240
   tccaaattga cccagccacc aagcgaaact ggatcccagc gggcaagcac gcactcactg      300
   tctcctattt ctacgatgcc acccgcaatg tgtaccgcat catcagcatc ggaggcgcca      360
   aggccatcat caacagcact gtcactccca acatgacctt caccaaaact tcccagaagt      420
   tcgggcagtg ggccgacagt cgcgccaaca cagtctacgg cttgggcttt gcctctgaac      480
   agcatctgac acagtttgcc gagaagttcc aggaagtgaa ggaagcagcc aggctggcca      540
   gggagaaatc tcaggatggc ggggagctca ccagtccagc cctggggctc gcctcccacc      600
   aggtgccccc gagccctctc gtcagtgcca acggccccgg cgaggaaaaa ctgttccgca      660
   gccagagcgc tgatgccccc ggccccacag agcgcgagcg gctaaagaag atgttgtctg      720
   agggctccgt gggcgaggta cagtgggagg ccgagttttt cgcactgcag gacagcaaca      780
   acaagctggc aggcgccctg cgagaggcca acgccgccgc agcccagtgg aggcagcagc      840
   tggaggctca gcgtgcagag gccgagcggc tgcggcagcg ggtggctgag ctggaggctc      900
   aggcagcttc agaggtgacc cccaccggtg agaaggaggg gctgggccag ggccagtcgc      960
   tggaacagct ggaagctctg gtgcaaacca aggaccagga gattcagacc ctgaagagtc     1020
   agactggggg gccccgcgag gccctggagg ctgccgagcg tgaggagact cagcagaagg    1080
   tgcaggacct ggagacccgc aatgcggagt tggagcacca gctgcgggcg atggagcgca     1140
   gcctggagga ggcacgggca gagcgggagc gggcgcgggc tgaggtgggc cgggcagcgc     1200
   agctgctgga cgtcaggctg tttgagctga gtgagctgcg tgagggcctg gcccgcctgg     1260
   ctgaggctgc gccctgagcc ggggctggtt ttctatgaac gattccggcc tgggatgcgg     1320
   gccaggctgc aggcggcata gttgggccca ttcgtcctgg aaagggactg gggggtccca   1380
   acttagccct gggtgggccg ggccgggctg ggctggggtg ggccccagtc ggctctggtt   1440
   gttggcagct ttggggctgt ttttgagctt ctcattgtgt agaatttcta gatcccccga   1500
   ttacatttct aagcgtgaaa aaaaaaaaaa aaaaaaaaaa aaaaaaaaaa aaaaaaaaaa   1560
   a                                                                   1561
<210> 75
   <211> 1188
   <212> DNA
   <213> human
<400> 75
   tcgagatcca ttgtgctcta aaggctcgcc ctcctgtgca tcgcggctaa tttggggtat     60
   cactgagctg aagacaaaga gaagggggag aaaacctagc agaccaccat gtgctatggg    120
   aagtgtgcac gatgcatcgg acattctctg gtggggctcg ccctcctgtg catcgcggct    180
   aatattttgc tttactttcc caatggggaa acaaagtatg cctccgaaaa ccacctcagc    240
   cgcttcgtgt ggttcttttc tggcatcgta ggaggtggcc tgctgatgct cctgccagca    300
   tttgtcttca ttgggctgga acaggatgac tgctgtggct gctgtggcca tgaaaactgt    360
   ggcaaacgat gtgcgatgct ttcttctgta ttggctgctc tcattggaat tgcaggatct    420
   ggctactgtg tcattgtggc agcccttggc ttagcagaag gaccactatg tcttgattcc    480
   ctcggccagt ggaactacac ctttgccagc accgagggcc agtaccttct ggatacctcc    540
   acatggtccg agtgcactga acccaagcac attgtggaat ggaatgtatc tctgttttct    600
   atcctcttgg ctcttggtgg aattgaattc atcttgtgtc ttattcaagt aataaatgga    660
   gtgcttggag gcatatgtgg cttttgctgc tctcaccaac agcaatatga ctgctaaaag    720
   aaccaaccca ggacagagcc acaatcttcc tctatttcat tgtaatttat atatttcact    780
   tgtattcatt tgtaaaactt tgtattagtg taacatactc cccacagtct acttttacaa    840
   acgcctgtaa agactggcat cttcacagga tgtcagtgtt taaatttagt aaacttcttt    900
   tttgtttgtt tatttgtgta acatactccc cacagtctac ttttacaaac gcctgtaaag    960
   actggcatct tcacaggatg tcagtgttta aatttagtaa acttcttttt tgtttgttta   1020
   tttgtttttg ttttttttta aggaatgagg aaacaaacca ccctctgggg gtagtttaca   1080
   gactgagtga cagtactcag tatatctgag ataaactcta taatgttttg gataaaaata   1140
   acattccatg gcacatatat acaatagtga ttggctttag agcacaat                1188
<210> 76
   <211> 1075
   <212> DNA
   <213> human
<400> 76
   cgcagcaaac acatccgtag aaggcagcgc ggccgccgag agccgcagcg ccgctcgccc      60
   gccgcccccc accccgccgc cccgcccggc gaattgcgcc ccgcgcccct cccctcgcgc     120
   ccccgagaca aagaggagag aaagtttgcg cggccgagcg gggcaggtga ggagggtgag     180
   ccgcgcggga ggggcccgcc tcggccccgg ctcagccccc gcccgcgccc ccagcccgcc     240
   gccgcgagca gcgcccggac cccccagcgg cggcccccgc ccgcccagcc ccccggcccg     300
   ccatgggcgc cgcggcccgc accctgcggc tggcgctcgg cctcctgctg ctggcgacgc     360
   tgcttcgccc ggccgacgcc tgcagctgct ccccggtgca cccgcaacag gcgttttgca     420
   atgcagatgt agtgatcagg gccaaagcgg tcagtgagaa ggaagtggac tctggaaacg     480
   acatttatgg caaccctatc aagaggatcc agtatgagat caagcagata aagatgttca     540
   aagggcctga gaaggatata gagtttatct acacggcccc ctcctcggca gtgtgtgggg     600
   tctcgctgga cgttggagga aagaaggaat atctcattgc aggaaaggcc gagggggacg    660
   gcaagatgca catcaccctc tgtgacttca tcgtgccctg ggacaccctg agcaccaccc     720
   agaagaagag cctgaaccac aggtaccaga tgggctgcga gtgcaagatc acgcgctgcc     780
   ccatgatccc gtgctacatc tcctccccgg acgagtgcct ctggatggac tgggtcacag     840
   agaagaacat caacgggcac caggccaagt tcttcgcctg catcaagaga agtgacggct     900
   cctgtgcgtg gtaccgcggc gcggcgcccc ccaagcagga gtttctcgac atcgaggacc     960
   cataagcagg cctccaacgc ccctgtggcc aactgcaaaa aaagcctcca agggtttcga    1020
   ctggtccagc tctgacatcc cttcctggaa acagcatgaa taaaacactc atccc         1075
<210> 77
   <211> 1358
   <212> DNA
   <213> human
<400> 77
   gcgacccggg gcgtttgcag cggtgccgag gaagaggacg ggaacggtgt tacgattgcc 60
   tgcgtttagg aggtggctgc gttgtgggaa aagctatcaa ggaagaaatt gccaaaccat 120
   gtcttttttt ctgttttcag agtagttcac aacagatctg agtgttttaa ttaagcatgg 180
   aatacagaaa acaacaaaaa acttaagctt taatttcatc tggaattcca cagttttctt 240
   agctccctgg acccggttga cctgttggct cttcccgctg gctgctctat cacgtggtgc 300
   tctccgacta ctcaccccga gtgtaaagaa ccttcggctc gcgtgcttct gagctgctgt 360
   ggatggcctc ggctctctgg actgtccttc cgagtaggat gtcactgaga tccctcaaat 420
   ggagcctcct gctgctgtca ctcctgagtt tctttgtgat gtggtacctc agccttcccc 480
   actacaatgt gatagaacgc gtgaactgga tgtacttcta tgagtatgag ccgatttaca 540
   gacaagactt tcacttcaca cttcgagagc attcaaactg ctctcatcaa aatccatttc 600
   tggtcattct ggtgacctcc cacccttcag atgtgaaagc caggcaggcc attagagtta 660
   cttggggtga aaaaaagtct tggtggggat atgaggttct tacatttttc ttattaggcc 720
   aagaggctga aaaggaagac aaaatgttgg cattgtcctt agaggatgaa caccttcttt 780
   atggtgacat aatccgacaa gattttttag acacatataa taacctgacc ttgaaaacca 840
   ttatggcatt caggtgggta actgagtttt gccccaatgc caagtacgta atgaagacag 900
   acactgatgt tttcatcaat actggcaatt tagtgaagta tcttttaaac ctaaaccact 960
   cagagaagtt tttcacaggt tatcctctaa ttgataatta ttcctataga ggattttacc 1020
   aaaaaaccca tatttcttac caggagtatc ctttcaaggt gttccctcca tactgcagtg 1080
   ggttgggtta tataatgtcc agagatttgg tgccaaggat ctatgaaatg atgggtcacg 1140
   taaaacccat caagtttgaa gatgtttatg tcgggatctg tttgaattta ttaaaagtga 1200
   acattcatat tccagaagac acaaatcttt tctttctata tagaatccat ttggatgtct 1260
   gtcaactgag acgtgtgatt gcagcccatg gcttttcttc caaggagatc atcacttttt 1320
   ggcaggtcat gctaaggaac accacatgcc attattaa 1358
<210> 78
   <211> 1246
   <212> DNA
   <213> human
<400> 78
   ggaaaggcct tggaaagcag tcgttgcgcc agacagccca gggaagagcg gcagcctgag      60
   gacctagggc cacctgctgt tccctgggat tcatgtcctt ctggggagga gggaggaccc     120
   aggacaatgg ctgctgttca tgatctggag atggagagca tgaatctgaa tatggggaga     180
   gagatgaaag aagagctgga ggaagaggag aaaatgagag aggatggggg aggtaaagat     240
   cgggccaaga gtaaaaaggt ccacaggatt gtctcaaaat ggatgctgcc cgaaaagtcc     300
   cgaggaacat acttggagag agctaactgc ttcccgcctc ccgtgttcat catctccatc     360
   agcctggccg agctggcagt gtttatttac tatgctgtgt ggaagcctca gaaacagtgg     420
   atcacgttgg acacaggcat cttggagagt ccctttatct acagtcctga gaagagggag     480
   gaagcctgga ggtttatctc atacatgctg gtacatgctg gagttcagca catcttgggg     540
   aatctttgta tgcagcttgt tttgggtatt cccttggaaa tggtccacaa aggcctccgt     600
   gtggggctgg tgtacctggc aggagtgatt gcagggtccc ttgccagctc catctttgac     660
   ccactcagat atcttgtggg agcttcagga ggagtctatg ctctgatggg aggctatttt     720
   atgaatgttc tggtgaattt tcaagaaatg attcctgcct ttggaatttt cagactgctg     780
   atcatcatcc tgataattgt gttggacatg ggatttgctc tctatagaag gttctttgtt     840
   cctgaagatg ggtctccggt gtcttttgca gctcacattg caggtggatt tgctggaatg     900
   tccattggct acacggtgtt tagctgcttt gataaagcac tgctgaaaga tccaaggttt     960
   tggatagcaa ttgctgcata tttagcttgt gtcttatttg ctgtgttttt caacattttc    1020
   ctatctccag caaactgacc tgcccctatt gtaagtcaat taataaaaag agccatctgg    1080
   aggaaataaa aaaaaaagga agactctatg aagaaacaga gaagtctcag aaaaggctaa    1140
   caattttata tagaggacaa aacagcatta aactcatcag ttgcaaagat tgcctataaa    1200
   aggaccttag gatttaagga aggggcttct taatgtagaa agggaa                   1246
<210> 79
   <211> 704
   <212> DNA
   <213> human
<220>
   <221> misc_feature
   <222> (23) .. (23)
   <223> N EQUALS ANY KIND BASE
<400> 79
   tttttttttt tttttttcag tantcagaat aatatatttt acttcttata atgtaaaaaa     60
   tataatcgtt tgagtggttt tcagcatgat ctgttaattt tgaatacaga gaatgaacaa    120
   agcaggtaaa tatatgtata tgctgaataa tgtaattcca tatacaattc acagttagat    180
   gcacttaatt gtggaaaata aaggaagaca ataacatcaa gatctttttc caaaacacgg    240
   taaaaataac gttcacatgc attaaacatt tcaagccatc tcagtatatg tctttcttga    300
   gtaagtagtg aaccaatgga ccagtggtta ttgttggaga aaacaattag gcaactcatc    360
   aatgcgctat ttatacaatc ttagtgacta tttaccactt cacctaagta gactttccca    420
   ctcatttgaa gctattgcta tctataaata aatggcaaca ggaaatgttt cacaagggcc    480
   tttgatttcc aaaactctca aattccacag caaagactca atttaaggca attatttatg    540
   cactgaatat ttgaatgaag atgtattatt ttccttaagt gaaaaaagct gatactattt    600
   tgtaatgata aaatttgtat accatagtag aaaatgattt gcaattatgt gttaggactt    660
   ttcatattcc atattgaaac atagtgattc tgtagctggg atca                     704
<210> 80
   <211> 1605
   <212> DNA
   <213> human
<400> 80
   atgggctgtg tgcaatgtaa ggataaagaa gcaacaaaac tgacggagga gagggacggc    60
   agcctgaacc agagctctgg gtaccgctat ggcacagacc ccacccctca gcactacccc   120
   agcttcggtg tgacctccat ccccaactac aacaacttcc acgcagccgg gggccaagga   180
   ctcaccgtct ttggaggtgt gaactcttcg tctcatacgg ggaccttgcg tacgagagga   240
   ggaacaggag tgacactctt tgtggccctt tatgactatg aagcacggac agaagatgac   300
   ctgagttttc acaaaggaga aaaatttcaa atattgaaca gctcggaagg agattggtgg   360
   gaagcccgct ccttgacaac tggagagaca ggttacattc ccagcaatta tgtggctcca   420
   gttgactcta tccaggcaga agagtggtac tttggaaaac ttggccgaaa agatgctgag   480
   cgacagctat tgtcctttgg aaacccaaga ggtacctttc ttatccgcga gagtgaaacc   540
   accaaaggtg cctattcact ttctatccgt gattgggatg atatgaaagg agaccatgtc   600
   aaacattata aaattcgcaa acttgacaat ggtggatact acattaccac ccgggcccag   660
   tttgaaacac ttcagcagct tgtacaacat tactcagaga aagctgacgg tttgtgtttt   720
   aacttaactg tgattgcatc gagttgtacc ccacaaactt ctggattggc taaagatgct   780
   tgggaagttg cacgtcgttc gttgtgtctg gagaagaagc tgggtcaggg gtgtttcgct   840
   gaagtgtggc ttggtacctg gaatggaaac acaaaagtag ccataaagac tcttaaacca   900
   ggcacaatgt cccccgaatc attccttgag gaagcgcaga tcatgaagaa gctgaagcac   960
   gacaagctgg tccagctcta tgcagtggtg tctgaggagc ccatctacat cgtcaccgag  1020
   tatatgaaca aaggaagttt actggatttc ttaaaagatg gagaaggaag agctctgaaa  1080
   ttaccaaatc ttgtggacat ggcagcacag gtggctgcag gaatggctta catcgagcgc  1140
   atgaattata tccatagaga tctgcgatca gcaaacattc tagtggggaa tggactcata  1200
   tgcaagattg ctgacttcgg attggcccga ttgatagaag acaatgagta cacagcaaga  1260
   caaggtgcaa agttccccat caagtggacg gcccccgagc gagccctgta cgggaggttc  1320
   acaatcaagt ctgacgtgtg gtcttttgga atcttactca cagagctggt caccaaagga  1380
   agagtgccat acccaggcat gaacaaccgg gaggtgctgg agcaggtgga gcgaggctac  1440
   aggatgccct gcccgcagga ctgccccatc tctctgcatg agctcatgat ccactgctgg  1500
   aaaaaggacc ctgaagaacg ccccactttt gagtacttgc agagcttcct ggaagactac  1560
   tttaccgcga cagagcccca gtaccaacct ggtgaaaacc tgtaa                  1605
<210> 81
   <211> 1717
   <212> DNA
   <213> human
<400> 81
   ccggggacgg ctgctggagc ggcgcccgcc gcggctcagc gcattcccgc tctccgcttc 60
   cctctccgct gcgtccccgc gcgaagatgg caaccgaggg gctgcacgag aacgagacgc 120
   tggcgtcgct gaagagcgag gccgagagcc tcaagggcaa gctggaggag gagcgagcca 180
   agctgcacga tgtggagctg caccaggtgg cggagcgggt ggaggccctg gggcagtttg 240
   tcatgaagac cagaaggacc ctcaaaggcc acgggaacaa agtcctgtgc atggactggt 300
   gcaaagataa gaggaggatc gtgagctcgt cacaggatgg gaaggtgatc gtgtgggatt 360
   ccttcaccac aaacaaggag cacgcggtca ccatgccctg cacgtgggtg atggcatgtg 420
   cttatgcccc atcgggatgt gccattgctt gtggtggttt ggataataag tgttctgtgt 480
   accccttgac gtttgacaaa aatgaaaaca tggctgccaa aaagaagtct gttgctatgc 540
   acaccaacta cctgtcggcc tgcagcttca ccaactctga catgcagatc ctgacagcga 600
   gcggcgatgg cacatgtgcc ctgtgggacg tggagagcgg gcagctgctg cagagcttcc 660
   acggacatgg ggctgacgtc ctctgcttgg acctggcccc ctcagaaact ggaaacacct 720
   tcgtgtctgg gggatgtgac aagaaagcca tggtgtggga catgcgctcc ggccagtgcg 780
   tgcaggcctt tgaaacacat gaatctgaca tcaacagtgt ccggtactac cccagtggag 840
   atgcctttgc ttcagggtca gatgacgcta cgtgtcgcct ctatgacctg cgggcagata 900
   gggaggttgc catctattcc aaagaaagca tcatatttgg agcatccagc gtggacttct 960
   ccctcagtgg tcgcctgctg tttgctggat acaatgatta cactatcaac gtctgggatg 1020
   ttctcaaagg gtcccgggtc tccatcctgt ttggacatga aaaccgcgtt agcactctac 1080
   gagtttcccc cgatgggact gctttctgct ctggatcatg ggatcatacc ctcagagtct 1140
   gggcctaatc atcttctgac agtgcactca tgtatacctg agaatttgaa atcttcacat 1200
   gtaaatagat attacttcta gaggagctta gagtttattg cagtgtagct taggggagca 1260
   acccatggct cacaggtcac taagcgtctc caatatgact attaaaactg tcacctctgg 1320
   aaatacacta gtgtgagcct tcagcactgc gagaatacct tcaagtacag tatttttctt 1380
   ttggaacact ttttaaaatg tatctgtttt taaggttatt ctaaattata gtagcctcaa 1440
   ctcattctgt caccagtaga attcagcagt taatatattc catattattt ctttgaatca 1500
   attcattttc agagcacttt aaagtctgat atttctcgat gtgcactgtg atgcctggaa 1560
   ccttcctctg gaagtgctga ttttatggac tgaggactgg tgactggtct gtgatagaag 1620
   caaattccaa ttccaaatgt aattagacaa aaatcatttt tttagaatgt gtttttattg 1680
   taaaagtatc tttttcagca aaaaaaaaaa aaaaaaa 1717
<210> 82
   <211> 691
   <212> DNA
   <213> human
<220>
   <221> misc_feature
   <222> (281)..(281)
   <223> N EQUALS ANY KIND BASE
<220>
   <221> misc_feature
   <222> (345) .. (345)
   <223> N EQUALS ANY KIND BASE
<220>
   <221> misc_feature
   <222> (358)..(358)
   <223> N EQUALS ANY KIND BASE
<220>
   <221> misc_feature
   <222> (443)..(443)
   <223> N EQUALS ANY KIND BASE
<220>
   <221> misc_feature
   <222> (478)..(478)
   <223> N EQUALS ANY KIND BASE
<400> 82
   tttttttttt tttttagaac cttagcactt taatagaatt agagactttg gaatttcagg     60
   tccttagaac caaagactca cagcatcttt gaaacctaga acctttgaat ctagactctt    120
   taaaccttgg actctagagt cttggaatgt taacacctgg gagggcttca gatattgcaa    180
   tccaacccct tccttttaca gatggtgatg ctactgcttt caaggtgatg ctactgcaca    240
   gagaagggga gggacctgtc tgggatggag gtgggatagg ntagagacag ggctgcaagt    300
   ggggataagg cgtggtggga aagtgggaag ggggagtttc cccantggca gtgcttanct    360
   tggatcctga gagggagtac caggtggagg gttgtctcag gcaccatcct cctgccctgg    420
   ggctgctggg gagcccctat cancaggctg agcggggcta ggggttgtgg aagggcanag    480
   gacatagcgt tcagcaggat ggacctcaac cgcagtgagg cagctacagg aatccttagg    540
   gtctggctgg gtttgggggg tcagctcctt cttgagcttc cagggggtca aggtaacctc     600
   caccttattc atggtgacat agagggattc gtcggcttct gggcagggaa gcagggcttt    660
   agtggtgtct tcaaaacttc cccgagctct g                                   691
<210> 83
   <211> 1284
   <212> DNA
   <213> human
<400> 83
   ggcctgtaca ttttcaagga attcttgaga ggttcttgga gagattctgg gagccaaaca     60
   ctccattggg atcctagctg ttttagagaa caacttgtaa tggagccttc atctcttgag    120
   ctgccggctg acacagtgca gcgcattgcg gctgaactca aatgccaccc aacggatgag    180
   agggtggctc tccacctaga tgaggaagat aagctgaggc acttcaggga gtgcttttat    240
   attcccaaaa tacaggatct gcctccagtt gatttatcat tagtgaataa agatgaaaat    300
   gccatctatt tcttgggaaa ttctcttggc cttcaaccaa aaatggttaa aacatatctt    360
   gaagaagaac tagataagtg ggccaaaata gcagcctatg gtcatgaagt ggggaagcgt    420
   ccttggatta caggagatga gagtattgta ggccttatga aggacattgt aggagccaat    480
   gagaaagaaa tagccctaat gaatgctttg actgtaaatt tacatcttct aatgttatca    540
   tttttaagcc ctacgccaaa acgatataaa attcttctag aagccaaagc cttcccttct    600
   gatcattatg ctattgagtc acaactacaa cttcacggac ttaacattga agaaagtatg    660
   cggatgataa agccaagaga gggggaagaa accttaagaa tagaggatat ccttgaagta    720
   attgagaagg aaggagactc aattgcagtg atcctgttca gtggggtgca tttttacact    780
   ggacagcact ttaatattcc tgccatcaca aaagctggac aagcgaaggg ttgttatgtt    840
   ggctttgatc tagcacatgc agttggaaat gttgaactct acttacatga ctggggagtt    900
   gattttgcct gctggtgttc ctacaagtat ttaaatgcag gagcaggagg aattgctggt    960
   gccttcattc atgaaaagca tgcccatacg attaaacctg cgagatcgga gttctttaat   1020
   taggaatgga atgcaacaga tttggacaag tcaaggacaa gagctttaga gagaccaaag   1080
   agtttttcac tgttaaagtg tccagtatgt agccgagaac catatggaga acatcaaata   1140
   cagtggaaca aatgtaactg ctattgatgt cacactttgt gaagtagtct ttgttgctta   1200
   aaaagggtga catctagtgg ctaaacatgt tatttcaaat aaataatatc gaaataaaaa   1260
   aaaaaaaaaa aaaaaaaaaa aaaa                                          1284
<210> 84
   <211> 566
   <212> DNA
   <213> human
<400> 84
   ttttgggatg cttcactttc tttattgccc atccagggga cagccaagcc agctccatct     60
   gcattctggc tgcagcgtgt acattagggg actcaggggc cacagtgtgg gaccgtgcac    120
   actggcaagg cactggcgga tgctggcagg ccagtggaca tggatagatg agaatgacaa    180
   ctcacagatg tcctagcttc cgctggccca gctgccagcc actggccatc acccttttgc    240
   ccagcatgtg tgcattgtca cccaaaacat cttgaaactt gccattagtg aggcattcaa    300
   caaagaagta agctaagtga gtaggaaaca gtgtttcctg gaatataccg cactctgcct    360
   gaaataggaa aactatgttt gccgggaagc agcagcagca ggaaagaagt tataccaaaa    420
   acgacttgta caccacagac attataaccc tttcctcaaa gaaacagtca tgttctgttg    480
   ggtattatgg acaggtctct ggaaatttat ctaataaaga ccaacaaact tccccagcag    540
   tgcctctgag taccgtgtga attctg                                         566
<210> 85
   <211> 813
   <212> DNA
   <213> human
<220>
   <221> misc_feature
   <222> (688)..(688)
   <223> N EQUALS ANY KIND BASE
<400> 85
   tttttttttt tttttttttt tttttttttt ttaaacaaac aaaaaagaag tttactaaat     60
   ttaaacactg acatcctggg aagatgccag tctttacagg cgtttgtaaa agtaaactgg    120
   ggggagtatg ttacactaat acaaagtttt acaaatgaat acaagtgaaa tatttaaatt    180
   acaatgaaat agaggaagat tggggctttg tcctgggttg gtttttttag cagtcatatt    240
   gctgttgggg agagcagcaa aagccacata tgcctccaag cactccattt attacttgaa    300
   taagacacaa gatgaattca attccaccaa gagccaagag gatagaaaac agagatacat    360
   tccattccac aatgtgcttg ggttcagtgc actcggacca tgtggaggta tccaaaaggg    420
   tcttgccctt ggggcttgga aaaggggtat ttccactggc cgagggaatc aagacatagt    480
   ggtccttctg ctaagccaag ggctgccaca atgacacagt agccagatcc tgcaattcca    540
   atgagagcag ccaatacaga agaaagcatc gcacatcgtt tgccacagtt ttcatggcca    600
   cagcagccac agcagtcatc ctgttccagc ccaatgaaga caaatgctgg caggagcatc    660
   agcaggccac cttctacgat gccagaanaa gaacacacga aagcgggttg aggggttttg    720
   ggaggcattc tttgtttccc ccttggggaa ataaaagcaa attttaaccc gggatgcccc    780
   aggaggcggg ccccaaccaa aaaaatgtcg gat                                 813
<210> 86
   <211> 2328
   <212> DNA
   <213> human
<400> 86
   gccagccgag cggccagcca gtgcggggct ggccatgtaa ggcccacagg cggtcctgcc 60
   cgcccggtgc cctgcggaga gcctcgtgca gccctgggca ccgcccctgc cctgccctga 120
   ccccttggcc ttgaaatgct gtcatcggag gagccgtccc gctcgggaca aggccagcat 180
   ggacaaagct agagctgggg caagcaagga gccttcctgt cctcgaggcc gtgggaagag 240
   aagcacgccc agggggccac tcctgagagc ctctctgtcc accaggcctc tgcagagggg 300
   tcaccatggc tctggcccga ggcagccggc agctgggggc cctggtgtgg ggcgcctgcc 360
   tgtgcgtgct ggtgcacggg cagcaggcgc agcccgggca gggctcggac cccgcccgct 420
   ggcggcagct gatccagtgg gagaacaacg ggcaggtgta cagcttgctc aactcgggct 480
   cagagtacgt gccggccgga cctcagcgct ccgagagtag ctcccgggtg ctgctggccg 540
   gcgcgcccca ggcccagcag cggcgcagcc acgggagccc ccggcgtcgg caggcgccgt 600
   ccctgcccct gccggggcgc gtgggctcgg acaccgtgcg cggccaggcg cggcacccat 660
   tcggctttgg ccaggtgccc gacaactggc gcgaggtggc cgtcggggac agcacgggca 720
   tggccctggc ccgcacctcc gtctcccagc aacggcacgg gggctccgcc tcctcggtct 780
   cggcttcggc cttcgccagc acctaccgcc agcagccctc ctacccgcag cagttcccct 840
   acccgcaggc gcccttcgtc agccagtacg agaactacga ccccgcgtcg cggacctacg 900
   accagggttt cgtgtactac cggcccgcgg gcggcggcgt gggcgcgggg gcggcggccg 960
   tggcctcggc gggggtcatc tacccctacc agccccgggc gcgctacgag gagtacggcg 1020
   gcggcgaaga gctgcccgag tacccgcctc agggcttcta cccggccccc gagaggccct 1080
   acgtgccgcc gccgccgccg ccccccgacg gcctggaccg ccgctactcg cacagtctgt 1140
   acagcgaggg cacccccggc ttcgagcagg cctaccctga ccccggtccc gaggcggcgc 1200
   aggcccatgg cggagaccca cgcctgggct ggtacccgcc ctacgccaac ccgccgcccg 1260
   aggcgtacgg gccgccgcgc gcgctggagc cgccctacct gccggtgcgc agctccgaca 1320
   cgcccccgcc gggtggggag cggaacggcg cgcagcaggg ccgcctcagc gtaggcagcg 1380
   tgtaccggcc caaccagaac ggccgcggtc tccctgactt ggtcccagac cccaactatg 1440
   tgcaagcatc cacttatgtg cagagagccc acctgtactc cctgcgctgt gctgcggagg 1500
   agaagtgtct ggccagcaca gcctatgccc ctgaggccac cgactacgat gtgcgggtgc 1560
   tactgcgctt cccccagcgc gtgaagaacc agggcacagc agacttcctc cccaaccggc 1620
   cacggcacac ctgggagtgg cacagctgcc accagcatta ccacagcatg gacgagttca 1680
   gccactacga cctactggat gcagccacag gcaagaaggt ggccgagggc cacaaggcca 1740
   gtttctgcct ggaggacagc acctgtgact tcggcaacct caagcgctat gcatgcacct 1800
   ctcataccca gggcctgagc ccaggctgct atgacaccta caatgcggac atcgactgcc 1860
   agtggatcga cataaccgac gtgcagcctg ggaactacat cctcaaggtg cacgtgaacc 1920
   caaagtatat tgttttggag tctgacttca ccaacaacgt ggtgagatgc aacattcact 1980
   acacaggtcg ctacgtttct gcaacaaact gcaaaattgt ccaatcctga tctccgggag 2040
   ggacagatgg ccaatctctc cccttccaaa gcaggccctg ctccccgggc agcctcccgc 2100
   cgaggggccc agcccccaac ccacaggcag ggaggggcat ccctccctgc cggcctcagg 2160
   gagcgaacgt ggatgaaaac cacagggatt ccggatgcca gaccccattt tatacttcac 2220
   ttttctctac agtgttgttt tgttgttgtt ggtttttatt ttttatactt tggccatacc 2280
   acagagctag attgcccagg tctgggctga ataaaacaag gtttttct 2328
<210> 87
   <211> 544
   <212> DNA
   <213> human
<400> 87
   aggcttttag aaaatttatt atgaattccg agaagtctgc tcatcatata cctcccccag 60
   ccccaaataa aacaaacaac atgtttgtac ataaagcctg gatttacttg gtacaaaatt 120
   tgagtctttg aaaaaaatag ttaatggaaa atctcaataa aaattcattt tgaaagtaac 180
   cagtactgtt cagaaataag gaagtcatgt tacttgagaa gtcacacagt tttattacag 240
   aactatgtgt atatattttg ggtttaaaac ttgccaatag ctgtttgaaa ggatagctca 300
   taatttattc aaatagatat tttattaatc aaatgttttt ggtttatcaa cataaccaaa 360
   tgtataaaaa atgtttttaa atacaagaca taactataaa gtcatgaggc tgattgacct 420
   tttaaactaa cataataaaa tctatatggt caaaatgagt ggtgatgctt taaggtaatg 480
   attatgcgtc ccatctaagg atgctgcaat ggcctagggc agttttgaaa tgtctctttg 540
   caac 544
<210> 88
   <211> 5189
   <212> DNA
   <213> human
<400> 88
<210> 89
   <211> 1061
   <212> DNA
   <213> human
<400> 89
   ctctgttttc tcaaagctga agtcggctag gtttgcaaag ctgtgggctg agcactcagg 60
   caatcacact ctcagaaact gcggcggctc tggactgcag cctcccaagg ctccatgcca 120
   gacaaagcat gcgtgtcaca cttgctacaa tagcctggat ggtttctttt gtctccaatt 180
   attcacacac agcaaatatt ttgccagata tcgaaaatga agatttcatc aaagactgcg 240
   ttcgaatcca taacaagttc cgatcagagg tgaaaccaac agccagtgat atgctataca    300
   tgacttggga cccagcacta gcccaaattg caaaagcatg ggccagcaat tgccagtttt    360
   cacataatac acggctgaag ccaccccaca agctgcaccc aaacttcact tcactgggag    420
   agaacatctg gactgggtct gtgcccattt tttctgtgtc ttccgccatc acaaactggt    480
   atgacgaaat ccaggactat gacttcaaga ctcggatatg caaaaaagtc tgtggccact    540
   acactcaggt tgtttgggca gatagttaca aagttggctg cgcagttcaa ttttgcccta    600
   aagtttctgg ctttgacgct ctttccaatg gagcacattt tatatgcaac tacggaccag    660
   gagggaatta cccaacttgg ccatataaga gaggagccac ctgcagtgcc tgccccaata    720
   atgacaagtg tttggacaat ctctgtgtta accgacagcg agaccaagtg aaacgttact    780
   actctgttgt atatccaggc tggcccatat atccacgtaa cagatacact tctctctttc    840
   tcattgttaa ttcagtaatt ctaatactgt ctgttataat taccattttg gtacagctca    900
   agtaccctaa tttagttctt ttggactaat acaattcagg aaagaaaaaa cccaaaaacc    960
   aacctcattc acatatggct tttttttaac caataacaat taggtgtact tctattttaa   1020
   aacatttcag aaaaaaatat atgttatagc aatactctta c                      1061
<210> 90
   <211> 1453
   <212> DNA
   <213> human
<400> 90
   agcgcgagtg ccagagccca gccggcgcgg agcgggagcg gtgcaggctg aggtctccga 60
   gcggctcgcc atggctggcc cgcagcagca gcccccttac ctgcacctgg ccgagctgac 120
   ggcgtcccag ttcctggaaa tatggaagca ctttgacgca gacggaaatg ggtatattga 180
   aggtaaagag ctagaaaact ttttccaaga gctggagaag gcaaggaaag gctctggcat 240
   gatgtcaaag agtgacaact ttggagaaaa gatgaaggag ttcatgcaga agtatgataa 300
   aaactcagat gggaaaatcg agatggcaga gctggcgcag atcctgccaa ccgaagagaa 360
   cttccttctg tgcttcaggc agcacgtggg ctccagcgcc gagtttatgg aggcttggcg 420
   gaagtacgac acagacagga gtggctacat cgaagccaat gagctcaagg gattcctgtc 480
   agacctgctg aagaaggcga accggccgta cgatgagccc aagctccagg aatacaccca 540
   aaccatacta cggatgtttg acttgaacgg ggatggcaaa ttgggcctct cagagatgtc 600
   ccgactcctg cctgtccagg aaaacttcct gcttaaattt cagggcatga agctgacctc 660
   agaggagttt aacgcgatct tcacatttta cgacaaggat agaagcggct acattgacga 720
   gcatgagctg gatgcccttt tgaaggatct gtacgagaaa aacaaaaagg aaatcaatat 780
   tcaacagctc accaactaca gaaagagcgt catgtccttg gcagaggcag ggaagctcta 840
   ccgcaaggac ctggagattg tgctctgcag cgagcccccc atgtaaagtg gggacggggg 900
   ctgcttctcc acctccccca aaccctgctt ctgctgccct gatgcgtcta cccagactca 960
   gagaccgtga gcgccccgcc cccaccccta cagcctgcac acacctgcct gcagagcagg 1020
   aaacgagaga tagaggatgg gcagctgggg ggctgtcctg agccccctgc acccacccct 1080
   gcccaggcag tctttgctca gtggatcaca cacatggaag gtgatggggg catgggtgga 1140
   gggtccctaa ttctcttcgc tgtgatgcat gagctccctc gctgtatgat ttaggcttct 1200
   atgtccaaca gagtggactc ttccctctcg ctcccctctg ccggtccccc atgccaccac 1260
   ccaccccaaa cttccaggtt ccatccacca ccttgccaat ggtgtagctg tcctctcaga 1320
   actcctgtgt gtggaaggca cccgcccttt ccttgccttc tttactcggc gtgctccttt 1380
   tctctttggg tttcttgttt accaaagaag agtttacaga caataaaatg gaaaggtcct 1440
   gctgtggaaa ctt 1453
<210> 91
   <211> 2223
   <212> DNA
   <213> human
<400> 91
   tcagtgtgtg cggaacgcaa gcagccgaga gcggagaggc gccgctgtag ttaactcctc      60
   cctgcccgcc gcgccgaccc tccccaggaa cccccaggga gccagcatga agcgagctca     120
   ccccgagtac agctcctcgg acagcgagct ggacgagacc atcgaggtgg agaaggagag     180
   tgcggacgag aatggaaact tgagttcggc tctaggttcc atgtccccaa ctacatcttc     240
   ccagattttg gccagaaaaa gacggagagg aataattgag aagcgccgac gagaccggat     300
   caataacagt ttgtctgagc tgagaaggct ggtacccagt gcttttgaga agcagggatc     360
   tgctaagcta gaaaaagccg agatcctgca gatgaccgtg gatcacctga aaatgctgca     420
   tacggcagga gggaaaggtt actttgacgc gcacgccctt gctatggact atcggagttt     480
   gggatttcgg gaatgcctgg cagaagttgc gcgttatctg agcatcattg aaggactaga     540
   tgcctctgac ccgcttcgag ttcgactggt ttcgcatctc aacaactacg cttcccagcg     600
   ggaagccgcg agcggcgccc acgcgggcct cggacacatt ccctggggga ccgtcttcgg     660
   acatcacccg cacatcgcgc acccgctgtt gctgccccag aacggccacg ggaacgcggg     720
   caccacggcc tcacccacgg aaccgcacca ccagggcagg ctgggctcgg cacatccgga     780
   ggcgcctgct ttgcgagcgc cccctagcgg cagcttcgga ccggtgctcc ctgtggtcac     840
   ctccgcctcc aaactgtcgc tgcctctgct ctcctcagtg gcctccctgt cggccttccc     900
   cttctctttc ggctccttcc acttactgtc tcccaatgca ctgagccctt cagcacccac 960
   gcaggctgca aaccttggca agccctatag accttggggg acggagatcg gagcttttta 1020
   aagaactgat gtagaatgag ggaggggaaa gtttaaaatc ccagctgggc tggactgttg 1080
   ccaacatcac cttaaagtcg tcagtaaaag taaaaaggaa aaaggtacac tttcagataa 1140
   tttttttttt aaagactaaa ggtttgttgg tttactttta tcttttttaa tgtttttttc 1200
   atcatgtcat gtattagcag tttttaaaaa ctagttgtta aattttgttc aagacattaa 1260
   attgaaatag tgagtataag ccaacacttt gtgataggtt tgtactgtgc ctaatttact 1320
   ttgtaaacca gaatgattcc gtttttgcct caaaatttgg ggaatcttaa catttaggta 1380
   tttttggtct gtttttctcc ttgtatagtt atggtctgtt tttagaatta attttccaaa 1440
   ccactatgct taatgttaac atgattctgt ttgttaatat tttgacagat taaggtgttg 1500
   tataaataat attcttttgg ggggagggga actatattga attttatatt tctgagcaaa 1560
   gcgttgacaa atcagatgat cagctttatc caagaaagaa gactagtaaa ttgtctgcct 1620
   cctatagcag aaaggtgaat gtacaaactg ttggtggcct gaatccatct gaccagctgc 1680
   tggtatctgc caggactggc agttctgatt tagttaggag gaccgctgat aggttaggtc 1740
   tcatttggag tgttggtgga aaggaaactg aaggtaattg aatagaatac gcctgcattt 1800
   accagcccca gcaacacaaa gaatttttaa tcacacggat ctcaaattca caaatgttaa 1860
   catggataag tgatcatggt gtgcgagtgg tcaattgagt agtacagtgg aaactgttaa 1920
   atgcataacc taattttcct gggactgcca tattttcttt taactggaaa tttttatgtg 1980
   agttttcctt ttggtgcatg gaactgtggt tgccaaggta tttaaaaggg ctttcctgcc 2040
   tccttctctt tgatttattt aatttgattt gggctataaa atatcatttt tcaggtttat 2100
   tcttttagca ggtgtagtta aacgacctcc actgaactgg gtttgacctc tgttgtactg 2160
   atgtgttgtg actaaataaa aaagaaagaa caaagtaaaa aaaaaaaaaa aaaaaaaaaa 2220
   aaa 2223
<210> 92
   <211> 4712
   <212> DNA
   <213> human
<400> 92
   cccggcggtg gcggcgtctc tggccggcct tggtgcggcg agccgagcga ggcagctctg 60
   agccgcgcgg aaatctggca ttttttaaag tttgcgcccc acaaagagga aatattccaa 120
   aggtactcag gatgtaaaag gggagatctt cacagatgcc tccgtggatg gcatggcaat 180
   ccatccatca atgagaagac catgatttct tttaattttc tgtgtgtttc cacattcccc 240
   agtgagaatt cttccacctt tttttgtgcc atgggaaaaa cctgaagggc aggcagagct 300
   gctcccgaac ttgtgacctt ctctgaggtt gcagcggctc ttgtagaaca tgactctggg 360
   acatcacttc cttttgtttt ctttcggagc tgaaccaaag aatgtgcacc ctctttctct 420
   agtgctgtgg tgtctgctta tttttgtatt tgtgctttcc atccatcttc tgtgatcaca 480
   aggcattctt aaggttttct agcacgactt gcggacatcc agactcgtgg ggggcccacc 540
   catggctcgg taagccagca gcccagggca ctggcactac catgaggcac tgcattaatt 600
   gctgcataca gctgttaccc gacggcgcac acaagcagca ggtcaactgc caagggggcc 660
   cccatcacgg tcaccaggcg tgccccacgt gcaaaggaga aaacaaaatt ctgtttcgtg 720
   tggacagtaa gcagatgaac ttgcttgctg ttctcgaagt gaggactgaa gggaacgaaa 780
   actggggtgg gtttttgcgc ttcaaaaagg ggaagcgatg tagcctcgtt tttggactga 840
   taataatgac cttggtaatg gcttcttaca tcctttctgg ggcccaccaa gagcttctga 900
   tctcatcacc tttccattac ggaggcttcc ccagcaaccc cagcttgatg gacagcgaaa 960
   acccaagtga cacaaaggag catcaccacc aatcctctgt aaataatatt tcatacatga 1020
   aggactatcc aagcattaaa ttaattatca acagcatcac aactaggatt gagttcacga 1080
   ccagacagct cccagactta gaagacctta agaagcagga gttgcatatg ttttcagtca 1140
   tccccaacaa attccttcca aacagtaaga gcccctgttg gtacgaggag ttctcggggc 1200
   agaacaccac cgacccctac ctcaccaact cctacgtgct ctactccaag cgcttccgct 1260
   ccaccttcga cgccctgcgc aaggccttct ggggccacct ggcgcacgcg cacgggaagc 1320
   acttccgcct gcgctgcctg ccgcacttct acatcatagg gcagcccaag tgcgggacca 1380
   cagacctcta tgaccgcctg cggctgcacc ctgaggtcaa gttctccgcc atcaaggagc 1440
   cacactggtg gacccggaag cgctttggaa tcgtccgcct aagagatggg ctgcgagacc 1500
   gctatcccgt ggaagattat ctggacctct ttgacctggc cgcacaccag atccatcaag 1560
   gactgcaggc cagctctgca aaggagcaga gcaagatgaa tacaatcatt atcggggagg 1620
   ccagtgcctc cacgatgtgg gataataatg cctggacgtt cttctacgac aacagcacgg 1680
   atggcgagcc accgtttctg acgcaggact tcatccacgc ctttcagcca aatgccagac 1740
   tgattgtcat gctcagggac cctgtggaga ggttgtactc agactatctc tactttgcaa 1800
   gttcgaataa atccgcggac gacttccatg agaaagtgac agaagcactg cagctgtttg 1860
   aaaattgcat gcttgattat tcactgcgcg cctgcgtcta caacaacacc ctcaacaacg 1920
   ccatgcctgt gaggctccag gttgggctct atgctgtgta ccttctggac tggctcagcg 1980
   tttttgacaa gcaacagttt ctcattcttc gcctggaaga tcatgcatcc aacgtcaagt 2040
   acaccatgca caaggtcttc cagtttctga acctagggcc cttaagtgag aagcaggagg 2100
   ctttgatgac caagagcccc gcatccaatg cacggcgtcc cgaggaccgg aacctggggc 2160
   ccatgtggcc catcacacag aagattctgc gggatttcta caggcccttc aacgctaggc 2220
   tggcgcaggt cctcgcggat gaggcgtttg cgtggaagac gacgtgagag ctgaattgtt 2280
   gctgcacgtg ctgggcccgc caatgccgtc atcatcagga ttttacaaat ctctttgcgg 2340
   ggaactgttt cactcatggt atggaaaacc ccaggactct gccactctag gcacacatga 2400
   attataacca ttttggaatt tccttcgtga tgttcgagag ctcagcaatg gacccctcac 2460
   agagctcctc tatccgaggc cattggagac cccagtttct caagaattca gctctgctct 2520
   gagcgtcctg gagcttgggg atgcagccag ctggcctgca ctgggtgtgg agagaacacc 2580
   tagggaaggc agcctggccc tgcccgcctc cgccttctgg agagcctctg ggttctgagt 2640
   cagcaagcca gaggtcatgc cacaggcctg gctggaactt acacttcacg ttcccttttt 2700
   ttccccctag agatggggtc tcgccgtgtt gcacagactg tctgtattca atggctatct 2760
   tcacaggtgt gatcatacca cattcacttc tgaaacactc ttgttgcgat cgctaacctc 2820
   actgggacag agaaccgcag tctttcgaga atggaggctc ttcatttttt ttttctcctt 2880
   tactccaaac tcagccctcc agtttcttca gatgtaaacc ctgttaacgt cactgtttcc 2940
   aaaaggaaaa aaataagtca gtttttggca gcaccttcat ctttctgacc tcctcctatt 3000
   ctgtccttgt ggacttatgt ttaacataga aaatgaatgc gtttaaaaca aaaccacttt 3060
   ctgcatttaa ccagtcctgg ctctctctct gctgcctctt catacgtttt ctcaagaact 3120
   tcagtttata attggaagag aaatttttgc tgttaatgcc agaatgagca acctcaagga 3180
   attgaacact tcttggaaaa tctaggtaat tcaagccctc atcaggttta caagatcatc 3240
   agagaaacag aggattttaa tttttagttc tggccggcta caggctccat ttctctgcct 3300
   tcccattgga aatagtttat ttccacattc tccactgcgt gtggtcaaag ttcctcaccc 3360
   agcaagggac tatagatact cgtgtcccaa ttccaaaaca caatgcacaa gctgaacttg 3420
   ggctgaacgt ggcgtgttga gatttggaat gaggtttcta agagccgtgt tcttcatgga 3480
   attttccagg ccacttggca gcttggttta ccgatggatg ggctagagat cttgtcgttt 3540
   cttggaagtc acagggaaga ttgaagagaa cgcttgagca tccttggcaa cagcccaggt 3600
   gggacctgga tgaagctttg cactcaagta ttgtcaaggg aagcttcctg tgaaccaaag 3660
   ttctcaggcc aaggtctcgc ccaccaaagc cagaaagtgc aagcacccgt ctacccagct 3720
   ctaacttgta tgtgtgagac agaccaggct tcgggggtag gaggatctgc agttgttcag 3780
   ccgtctttct gctggtgttg tctttctgcc atcagagaag ggacacacag cccgttcgaa 3840
   ggtgtgcaga gggctctgag cgccaggatg gccagggctg tttttgctac tgaaggagcg 3900
   tgtgtcctga actcccactt gcagggacag tccccacctt ctctatagcc ggcactggga   3960
   gcagccgcca gcagggaaat ctggcctgag cacaaggatg ctttagggag agatcacttc   4020
   agtgtgtgtg tatatttatt tgcagtacag tgcgcgcgtg tgtgtgtgtg tacgcgcacg   4080
   tgtgggtgag tgcgtcttct gagtgggttc tgttcagttg ctaatgaggc tcctccgctc   4140
   tggacacaac ccttttatag attaatttct ctgccaatta acttgtcatt ttcagtacat   4200
   attttactat tccacaccaa ccataattac aacaagggat ttttcttatg cactcctatg   4260
   catgtgaata acatgtggtg taattctgct tcttacagaa gtattactga aggtattatt   4320
   tccaatatta tttggtttat tatgcggatc ttttttatat atgcagtccc atcccttctg   4380
   tgccactcaa tgccatccag acatggtttt tccctccagg ggcctttctc tccagagggc   4440
   acttcggctg cctctgcttc ctctcattcg aggcccggct cttgctgaca gaataggttc   4500
   cgttctgggc ggtggttctc gagcctgcca ttcaaaacca aagcaaattg gagcatttct   4560
   cacaacatgg tattgaagtt cctttttgtt ctcaaaagtt gtgaccgtgt taaattgtac   4620
   tcccttagtc ctgtaaggta tgttaagtga atcgcagtta cgctgtactt ttattaatat   4680
   ttaacataat taaagatgga cccataagag tg                                 4712
<210> 93
   <211> 1398
   <212> DNA
   <213> human
<400> 93
   gtgtgaaatc ttcagagaag aatttctctt tagttctttg caagaaggta gagataaaga 60
   cactttttca aaaatggcaa tggtatcaga attcctcaag caggcctggt ttattgaaaa 120
   tgaagagcag gaatatgttc aaactgtgaa gtcatccaaa ggtggtcccg gatcagcggt 180
   gagcccctat cctaccttca atccatcctc ggatgtcgct gccttgcata aggccataat 240
   ggttaaaggt gtggatgaag caaccatcat tgacattcta actaagcgaa acaatgcaca 300
   gcgtcaacag atcaaagcag catatctcca ggaaacagga aagcccctgg atgaaacact 360
   taagaaagcc cttacaggtc accttgagga ggttgtttta gctctgctaa aaactccagc 420
   gcaatttgat gctgatgaac ttcgtgctgc catgaagggc cttggaactg atgaagatac 480
   tctaattgag attttggcat caagaactaa caaagaaatc agagacatta acagggtcta 540
   cagagaggaa ctgaagagag atctggccaa agacataacc tcagacacat ctggagattt 600
   tcggaacgct ttgctttctc ttgctaaggg tgaccgatct gaggactttg gtgtgaatga 660
   agacttggct gattcagatg ccagggcctt gtatgaagca ggagaaagga gaaaggggac 720
   agacgtaaac gtgttcaata ccatccttac caccagaagc tatccacaac ttcgcagagt 780
   gtttcagaaa tacaccaagt acagtaagca tgacatgaac aaagttctgg acctggagtt 840
   gaaaggtgac attgagaaat gcctcacagc tatcgtgaag tgcgccacaa gcaaaccagc 900
   tttctttgca gagaagcttc atcaagccat gaaaggtgtt ggaactcgcc ataaggcatt 960
   gatcaggatt atggtttccc gttctgaaat tgacatgaat gatatcaaag cattctatca 1020
   gaagatgtat ggtatctccc tttgccaagc catcctggat gaaaccaaag gagattatga 1080
   gaaaatcctg gtggctcttt gtggaggaaa ctaaacattc ccttgatggt ctcaagctat 1140
   gatcagaaga ctttaattat atattttcat cctataagct taaataggaa agtttcttca 1200
   acaggattac agtgtagcta cctacatgct gaaaaatata gcctttaaat catttttata 1260
   ttataactct gtataataga gataagtcca ttttttaaaa atgttttccc caaaccataa 1320
   aaccctatac aagttgttct agtaacaata catgagaaag atgtctatgt agctgaaaat 1380
   aaaatgacgt cacaagac 1398
<210> 94
   <211> 2972
   <212> DNA
   <213> human
<400> 94
   gcgcgcggct ccgatgggaa gcatgacccg ggtggcggga caagacttgc ttcccggcca     60
   cgcgcgctcg gccggccgtg gggcggggca taggcgtgac gtggtgtcgc gtatcgagtc    120
   tccgccccct tcccgcctcc ccgtatataa gacttcgccg agcactctca ctcgcacaag   180
   tggaccgggg tgttgggtgc tagtcggcac cagaggcaag ggtgcgagga ccacggccgg    240
   ctcggacgtg tgaccgcgcc tagggggtgg cagcgggcag tgcggggcgg caaggcgacc   300
   atggarcttt tgcggactat cacctaccag ccagccgcca gcaccaaaat gtgcgagcag    360
   gcgctgggca agggttgcgg aggggactcg aagaagaagc ggccgccgca gccccccgag    420
   gaatcgcagc cacctcagtc ccaggcgcaa gtgcccccgg cggcccctca ccaccatcac    480
   caccattcgc actcggggcc ggagatctcg cggattatcg tcgaccccac gactgggaag    540
   cgctactgcc ggggcaaagt gctgggaaag ggtggctttg caaaatgtta cgagatgaca    600
   gatttgacaa ataacaaagt ctacgccgca aaaattattc ctcacagcag agtagctaaa    660
   cctcatcaaa gggaaaagat tgacaaagaa atagagcttc acagaattct tcatcataag    720
   catgtagtgc agttttacca ctacttcgag gacaaagaaa acatttacat tctcttggaa    780
   tactgcagta gaaggtcaat ggctcatatt ttgaaagcaa gaaaggtgtt gacagagcca    840
   gaagttcgat actacctcag gcagattgtg tctggactga aataccttca tgaacaagaa    900
   atcttgcaca gagatctcaa actagggaac ttttttatta atgaagccat ggaactaaaa   960
   gttggggact tcggtctggc agccaggcta gaacccytgg aacacagaag gagaacgata 1020
   tgtggtaccc caaattatct ctctcctgaa gtcctcaaca aacaaggaca tggctgtgaa 1080
   tcagacattt gggccctggg ctgtgtaatg tatacaatgt tactagggag gcccccattt 1140
   gaaactacaa atctcaaaga aacttatagg tgcataaggg aagcaaggta tacaatgccg 1200
   tcctcattgc tggctcctgc caagcactta attgctagta tgttgtccaa aaacccagag 1260
   gatcgtccca gtttggatga catcattcga catgactttt ttttgcaggg cttcactccg 1320
   gacagactgt cttctagctg ttgtcataca gttccagatt tccacttatc aagcccagct 1380
   aagaatttct ttaagaaagc agctgctgct ctttttggtg gcaaaaaaga caaagcaaga 1440
   tatattgaca cacataatag agtgtctaaa gaagatgaag acatctacaa gcttaggcat 1500
   gatttgaaaa agacttcaat aactcagcaa cccagcaaac acaggacaga tgaggagctc 1560
   cagccaccta ccaccacagt tgccaggtct ggaacacccg cagtagaaaa caagcagcag 1620
   attggggatg ctattcggat gatagtcaga gggactcttg gcagctgtag cagcagcagt 1680
   gaatgccttg aagacagtac catgggaagt gttgcagaca cagtggcaag ggttcttcgg 1740
   ggatgtctgg aaaacatgcc ggaagctgat tgcattccca aagagcagct gagcacatca 1800
   tttcagtggg tcaccaaatg ggttgattac tctaacaaat atggctttgg gtaccagctc 1860
   tcagaccaca ccgtcggtgt ccttttcaac aatggtgctc acatgagcct ccttccagac 1920
   aaaaaaacag ttcactatta cgcagagctt ggccaatgct cagttttccc agcaacagat 1980
   gctcctgagc aatttattag tcaagtgacg gtgctgaaat acttttctca ttacatggag 2040
   gagaacctca tggatggtgg agatctgcct agtgttactg atattcgaag acctcggctc 2100
   tacctccttc agtggctaaa atctgataag gccctaatga tgctctttaa tgatggcacc 2160
   tttcaggtga atttctacca tgatcataca aaaatcatca tctgtagcca aaatgaagaa 2220
   taccttctca cctacatcaa tgaggatagg atatctacaa ctttcaggct gacaactctg 2280
   ctgatgtctg gctgttcatc agaattaaaa aatcgaatgg aatatgccct gaacatgctc 2340
   ttacaaagat gtaactgaaa gacttttcga atggacccta tgggactcct cttttccact 2400
   gtgagatcta cagggaagcc aaaagaatga tctagagtat gttgaagaag atggacatgt 2460
   ggtggtacga aaacaattcc cctgtggcct gctggactgg gtggaaccca gaaccaggct 2520
   aaggcataca gttcttgact ttggacaatc ccaagagtga accagaatgc agttttcctt 2580
   gagatacctg ttttaaaagg tttttcagac aattttgcag aaaggtgcat tgattcttaa 2640
   attctctctg ttgagagcat ttcagccaga ggactttgga actgtgaata tacttcctga 2700
   aggggaggga gaagggagga agctcccatg ttgtttaaag gctgtaattg gagcagcttt 2760
   tggctgcgta actgtgaact atggccatat ataatttttt ttcattaatt tttgaagata 2820
   cttgtggctg gaaaagtgca ttccttgtta ataaactttt tatttattac agcccaaaga 2880
   gcagtattta ttatcaaaat gtcttttttt ttatgttgac cattttaaac cgttggcaat 2940
   aaagagtatg aaaacgcaaa aaaaaaaaaa aa 2972